# EUROPEAN PATENT APPLICATION

(11) **EP 3 722 418 A1**
(43) Date of publication of application: **14.10.2020**
(21) Application number: 19167787.1
(22) Date of filing: 08.04.2019
(51) Int. Cl.: C12N 9/16, C12N 9/96, D21C 3/00, D21C 5/00

(54) **SOLUTION STABLE ENZYME COMPOSITION**

(71) Applicant: AB Enzymes Oy, 05200 Rajamäki (FI)
(72) Inventor: PALMUNEN, Katja, 05200 Rajamäki (FI); BESENMATTER, Werner, 64293 Darmstadt (DE); PERKKALAINEN, Mirkka, 05200 Rajamäki (FI); LEHTIKARI, Leena, 05200 Rajamäki (FI)
(74) Representative: Espatent Oy

(57) **Abstract**

A solution stable enzyme composition comprising an enzyme component, a stabilizer component, an optional antimicrobial preservative and water is disclosed, as well as its use in manufacturing of pulp and paper.

SEQUENCE LISTING

<110>  AB Enzymes

<120>  SOLUTION STABLE ENZYME COMPOSITION

<130>  31786FI-1f_

<160>  32

<170>  PatentIn version 3.5

<210>  1
<211>  558
<212>  PRT
<213>  Melanocarpus albomyces

<400>  1

Ala Pro Pro Glu Pro Pro Thr Asn Val Val Glu Lys Arg Ala Ala Pro
1               5                   10                  15

Thr Val Glu Ile Ser Thr Gly Thr Ile Val Gly Thr Thr Arg Leu Ala
            20                  25                  30

Thr Glu Ala Phe Asn Gly Ile Pro Phe Ala Leu Pro Pro Val Gly Gln
        35                  40                  45

Leu Arg Leu Lys Pro Pro Val Arg Leu Asn Ser Ser Leu Gly Val Phe
    50                  55                  60

Asp Ala Ser Gly Ile Ala Pro Ala Cys Pro Gln Phe Leu Ala Asp Ser
65                  70                  75                  80

Asp Ser Asn Glu Phe Leu Ala Gln Val Ile Asn Thr Val Thr Gly Leu
                85                  90                  95

Pro Phe Phe Gln Lys Ala Leu Lys Ile Ser Glu Asp Cys Leu Thr Ile
            100                 105                 110

Asn Val Ile Arg Pro Lys Gly Thr Lys Ala Gly Asp Lys Leu Pro Val
        115                 120                 125

Leu Phe Trp Ile Tyr Gly Gly Gly Phe Glu Leu Gly Trp Ser Ser Met
    130                 135                 140

Tyr Asp Gly Gly Pro Leu Val Thr Asn Ala Ile Gly Phe Gly Lys Pro
145                 150                 155                 160

Phe Ile Phe Val Ala Val Asn Tyr Arg Val Ala Gly Phe Gly Phe Met
                165                 170                 175

Pro Gly Lys Glu Ile Leu Ala Asp Gly Ala Ala Asn Leu Gly His Leu
        180                 185                 190

Asp Gln Arg Met Gly Leu Glu Trp Val Ala Asp Asn Ile Ala Ala Phe
    195                 200                 205

Gly Gly Asp Pro Asp Lys Val Thr Ile Trp Gly Glu Ser Ala Gly Ala
    210                 215                 220

Ile Ser Val Leu Asn Gln Met Ala Leu Phe Asp Gly Asp His Thr Tyr
225                 230                 235                 240

Lys Gly Lys Pro Leu Phe Arg Gly Ala Ile Met Asn Ser Gly Ser Ile
                245                 250                 255

Val Pro Ala Asp Pro Val Asp Cys Pro Lys Gly Gln Glu Ile Tyr Asp
            260                 265                 270

Gln Val Val Ala Lys Ala Gly Cys Ala Gly Ala Ser Asp Thr Leu Ala
        275                 280                 285

Cys Leu Arg Glu Leu Pro Tyr Glu Lys Phe Leu Asp Ala Ala Asn Ser
    290                 295                 300

Val Pro Ala Ile Leu Ser Tyr Asn Ser Val Ala Leu Ser Tyr Leu Pro
305                 310                 315                 320

Arg Pro Asp Gly Lys Val Leu Thr Lys Ser Pro Asp Val Leu Ile Lys
                325                 330                 335

Glu Gly Lys Tyr Ala Ala Val Pro Met Ile Ile Gly Asp Gln Glu Asp
            340                 345                 350

Glu Gly Thr Leu Phe Ser Leu Phe Gln Pro Asn Ile Thr Asn Ser Glu
        355                 360                 365

Lys Leu Val Thr Tyr Leu Lys Asp Leu Phe Phe His Gly Ala Ser Arg
    370                 375                 380

Glu Gln Leu Glu Gly Leu Val Asp Arg Tyr Pro Ala Arg Ile Ser Ala
385                 390                 395                 400

Gly Ser Pro Phe Arg Thr Gly Leu Leu Asn Glu Ile Tyr Pro Gly Phe
                405                 410                 415

Lys Arg Leu Ala Ala Ile Leu Gly Asp Leu Val Phe Thr Leu Thr Arg
            420                 425                 430

Arg Val Phe Leu Glu Asp Ala Thr Arg Val Asn Pro Thr Val Pro Ala
        435                 440                 445

Trp Ser Tyr Leu Ala Ser Tyr Asp Tyr Gly Thr Pro Ile Leu Gly Thr
    450                 455                 460

Phe His Gly Ser Asp Leu Leu Gln Val Phe Phe Gly Ile Leu Pro Asn
465                 470                 475                 480

Tyr Ala Ser Arg Ser Ile Gln Ser Tyr Tyr Ala Asn Phe Val Tyr Asn
                485                 490                 495

Leu Asp Pro Asn Asp Ala Ser Gly Gly Thr Ser Gly Lys Ser Lys Val
            500                 505                 510

Ala Glu Glu Trp Pro Arg Trp Thr Ala Asp Asp Arg Thr Leu Ile Gln
        515                 520                 525

Phe Phe Ala Asn Arg Asn Gly Tyr Leu Lys Asp Asp Phe Arg Ser Glu
    530                 535                 540

Ala Ala Glu Tyr Ile Gly Ala His Val Asp Tyr Leu His Ile
545                 550                 555

<210>  2
<211>  558
<212>  PRT
<213>  Chaetomium thermophilum

<400>  2

Ala Pro Pro Glu Pro Pro Thr Lys Val Leu Ala Lys Arg Ala Ala Pro
1               5                   10                  15

Thr Val Thr Ile Ser Thr Gly Thr Val Ile Gly Thr Asp Arg Ile Val
            20                  25                  30

Thr Glu Ala Phe Asn Gly Ile Pro Phe Ala Lys Ala Pro Thr Gly Asn
        35                  40                  45

Leu Arg Leu Arg Pro Pro Val Arg Ile Asn Glu Ser Leu Gly Val Ile
    50                  55                  60

Asp Ala Thr Gly Ile Ala Pro Ala Cys Pro Gln Phe Leu Ala Asp Ala
65                  70                  75                  80

Ser Ser Asn Glu Phe Leu Ala Gln Val Leu Asp Lys Val Thr Asn Leu
                85                  90                  95

Pro Phe Phe Gln Lys Ala Leu Lys Ile Thr Glu Asp Cys Leu Thr Val
        100                 105                 110

Ser Val Ile Arg Pro Lys Gly Val Lys Ala Gly Asp Lys Leu Pro Val
        115                 120                 125

Leu Phe Trp Ile Tyr Gly Gly Gly Phe Glu Leu Gly Trp Ser Ser Met
    130                 135                 140

Tyr Asp Gly Gly Pro Leu Val Ala Ser Ala Thr Ser Leu Gly Lys Pro
145                 150                 155                 160

Phe Ile Phe Val Ala Val Asn Tyr Arg Val Ala Gly Phe Gly Phe Met
                165                 170                 175

Pro Gly Lys Glu Ile Leu Ala Asp Gly Ala Ala Asn Leu Gly Leu Leu
            180                 185                 190

Asp Gln Arg Met Gly Leu Glu Trp Val Ala Asp Asn Ile Glu Ala Phe
        195                 200                 205

Gly Gly Asp Pro Asn Lys Val Thr Ile Trp Gly Glu Ser Ala Gly Ser
    210                 215                 220

Ile Ser Val Tyr Asn Gln Met Ala Leu Tyr Asp Gly Asp Asn Thr Tyr
225                 230                 235                 240

Lys Gly Lys Pro Leu Phe Arg Ala Ala Ile Met Asn Ser Gly Ser Met
                245                 250                 255

Ile Pro Ala Asp Pro Val Asp Cys Pro Lys Gly Gln Gln Val Tyr Asn
            260                 265                 270

Thr Val Val Ala Lys Ala Gly Cys Ser Gly Ala Ala Asp Thr Leu Ala
        275                 280                 285

Cys Leu Arg Ser Leu Pro Tyr Glu Lys Phe Leu Arg Ala Ala Asn Ser
    290                 295                 300

Val Pro Ala Ile Leu Ser Tyr Asn Ser Val Ala Leu Ser Tyr Leu Pro
305                 310                 315                 320

Arg Pro Asp Gly Lys Val Leu Thr Lys Ser Pro Asp Leu Leu Ala Lys
                325                 330                 335

Glu Gly Lys Phe Ala Ala Val Pro Met Ile Ile Gly Asp Gln Glu Asp
            340                 345                 350

Glu Gly Thr Leu Phe Ser Leu Phe Gln Thr Asn Val Thr Thr Thr Asp
        355                 360                 365

Arg Leu Val Thr Tyr Leu Lys Asp Ile Phe Phe His Asn Ala Ser Lys
    370                 375                 380

Lys Gln Leu Glu Asp Leu Val Asn Thr Tyr Glu Pro Trp Val Trp Ala
385                 390                 395                 400

Gly Ser Pro Phe Arg Thr Gly Leu Leu Asn Glu Val Tyr Pro Gly Phe
                405                 410                 415

Lys Arg Leu Ala Ala Ile Leu Gly Asp Leu Val Phe Thr Leu Thr Arg
            420                 425                 430

Arg Ile Phe Leu Glu Tyr Val Thr Ala Ala Lys Pro Asn Val Pro Val
        435                 440                 445

Trp Ser Tyr Leu Ala Ser Tyr Asp Tyr Gly Thr Pro Ile Leu Gly Thr
    450                 455                 460

Phe His Gly Ser Asp Leu Leu Gln Val Phe Tyr Gly Val Leu Pro Asn
465                 470                 475                 480

Tyr Ala Ser Lys Ser Ile His Ser Tyr Tyr Ala Asn Phe Ile Tyr Asn
                485                 490                 495

Leu Asn Pro Asn Asp Ala Ser Gly Gly Thr Ser Ser Lys Ser Lys Val
            500                 505                 510

Ser Glu Thr Trp Pro Gln Trp Thr Ser Ser Thr Arg Lys Leu Ile Asn
        515                 520                 525

Phe Phe Ala Asn Arg Asn Glu Phe Ile Lys Asp Asp Phe Arg Ser Asp
    530                 535                 540

Ser Ala Ala Phe Ile Arg Asn Asn Val Asn Ile Leu His Ile
545                 550                 555

<210>  3
<211>  558
<212>  PRT
<213>  Artificial Sequence

<220>
<223>  synthetic sequence

<400>  3

Ala Pro Pro Glu Pro Pro Thr Lys Val Leu Glu Lys Arg Ala Ala Pro
1               5                   10                  15

Thr Val Thr Ile Ser Thr Gly Thr Val Ile Gly Thr Asp Arg Ile Val
            20                  25                  30

Thr Glu Ala Phe Asn Gly Ile Pro Phe Ala Lys Ala Pro Thr Gly Asn
        35                  40                  45

Leu Arg Leu Lys Pro Pro Val Arg Leu Asn Glu Ser Leu Gly Val Ile
    50                  55                  60

Asp Ala Thr Gly Ile Ala Pro Ala Cys Pro Gln Phe Leu Ala Asp Ala
65                  70                  75                  80

Ser Ser Asn Glu Phe Leu Ala Gln Val Leu Asp Lys Val Thr Asn Leu
                85                  90                  95

Pro Phe Phe Gln Lys Ala Leu Lys Ile Ser Glu Asp Cys Leu Thr Val
            100                 105                 110

Ser Val Ile Arg Pro Lys Gly Val Lys Ala Gly Asp Lys Leu Pro Val
        115                 120                 125

Leu Phe Trp Ile Tyr Gly Gly Gly Phe Glu Leu Gly Trp Ser Ser Met
    130                 135                 140

Tyr Asp Gly Gly Pro Leu Val Ala Ser Ala Thr Ser Leu Gly Lys Pro
145                 150                 155                 160

Phe Ile Phe Val Ala Val Asn Tyr Arg Val Ala Gly Phe Gly Phe Met
                165                 170                 175

Pro Gly Lys Glu Ile Leu Ala Asp Gly Ala Ala Asn Leu Gly Leu Leu
            180                 185                 190

Asp Gln Arg Met Gly Leu Glu Trp Val Ala Asp Asn Ile Glu Ala Phe
        195                 200                 205

Gly Gly Asp Pro Asn Lys Val Thr Ile Trp Gly Glu Ser Ala Gly Ser
    210                 215                 220

Ile Ser Val Tyr Asn Gln Met Ala Leu Tyr Asp Gly Asp Asn Thr Tyr
225                 230                 235                 240

Lys Gly Lys Pro Leu Phe Arg Gly Ala Ile Met Asn Ser Gly Ser Met
                245                 250                 255

Ile Pro Ala Asp Pro Val Asp Cys Pro Lys Gly Gln Gln Val Tyr Asp
        260                 265                 270

Thr Val Val Ala Lys Ala Gly Cys Ser Gly Ala Ala Asp Thr Leu Ala
    275                 280                 285

Cys Leu Arg Ser Leu Pro Tyr Glu Lys Phe Leu Arg Ala Ala Asn Ser
    290                 295                 300

Val Pro Ala Ile Leu Ser Tyr Asn Ser Val Ala Leu Ser Tyr Leu Pro
305                 310                 315                 320

Arg Pro Asp Gly Lys Val Leu Thr Lys Ser Pro Asp Leu Leu Ala Lys
                325                 330                 335

Glu Gly Lys Tyr Ala Ala Val Pro Met Ile Ile Gly Asp Gln Glu Asp
            340                 345                 350

Glu Gly Thr Leu Phe Ser Leu Phe Gln Thr Asn Val Thr Thr Thr Asp
        355                 360                 365

Arg Leu Val Thr Tyr Leu Lys Asp Ile Phe Phe His Asn Ala Ser Lys
    370                 375                 380

Lys Gln Leu Glu Asp Leu Val Asn Thr Tyr Glu Pro Trp Val Trp Ala
385                 390                 395                 400

Gly Ser Pro Phe Arg Thr Gly Leu Leu Asn Glu Val Tyr Pro Gly Phe
                405                 410                 415

Lys Arg Leu Ala Ala Ile Leu Gly Asp Leu Val Phe Thr Leu Thr Arg
            420                 425                 430

Arg Ile Phe Leu Glu Tyr Ala Thr Ala Ala Asn Pro Asn Val Pro Ala
        435                 440                 445

Trp Ser Tyr Leu Ala Ser Tyr Asp Tyr Gly Thr Pro Ile Leu Gly Thr
    450                 455                 460

Phe His Gly Ser Asp Leu Leu Gln Val Phe Tyr Gly Val Leu Pro Asn
465                 470                 475                 480

Tyr Ala Ser Lys Ser Ile His Ser Tyr Tyr Ala Asn Phe Val Tyr Asn
                485                 490                 495

Leu Asp Pro Asn Asp Ala Ser Gly Gly Thr Ser Ser Lys Ser Lys Val

500                 505                 510

Ser Glu Thr Trp Pro Gln Trp Thr Ser Ser Thr Arg Lys Leu Ile Asn
        515                 520                 525

Phe Phe Ala Asn Arg Asn Glu Phe Ile Lys Asp Asp Phe Arg Ser Asp
    530                 535                 540

Ser Ala Ala Phe Ile Arg Asn Asn Val Asn Ile Leu His Ile
545                 550                 555

<210>  4
<211>  558
<212>  PRT
<213>  Myceliophthora thermophila

<400>  4

Ala Pro Pro Glu Pro Pro Thr Lys Ile Val Glu Lys Arg Ala Ala Pro
1               5                   10                  15

Thr Val Thr Ile Ala Ser Gly Ser Ile Val Gly Val Ser Arg Gln Ala
            20                  25                  30

Thr Glu Ala Phe Asn Gly Ile Pro Phe Ala Glu Pro Pro Val Gly Pro
        35                  40                  45

Leu Arg Leu Lys Pro Pro Val Arg Leu Asn Ser Ser Leu Gly Val Phe
    50                  55                  60

Asp Ala Thr Gly Ile Ala Pro Ala Cys Pro Gln Phe Phe Ala Asp Ser
65                  70                  75                  80

Asp Ser Asn Asp Phe Leu Ser Arg Val Ile Asp Thr Val Thr Ser Leu
                85                  90                  95

Pro Phe Phe Gln Lys Ala Leu Lys Val Ser Glu Asp Cys Leu Thr Ile
            100                 105                 110

Asn Val Ile Arg Pro Lys Gly Thr Lys Ala Gly Asp Asn Leu Pro Val
        115                 120                 125

Leu Phe Trp Ile Phe Gly Gly Gly Phe Glu Leu Gly Trp Ser Ser Met
    130                 135                 140

Tyr Asp Gly Gly Pro Leu Val Ser Asn Ala Ile Asp Met Gly Lys Pro
145                 150                 155                 160

Tyr Val Phe Val Ala Val Asn Tyr Arg Val Gly Gly Phe Gly Phe Met

                165                 170                 175

Pro Gly Lys Glu Ile Leu Ala Asp Gly Ser Ala Asn Leu Gly His Leu
            180                 185                 190

Asp Gln Arg Met Gly Leu Glu Trp Val Ala Asp Asn Ile Ala Ala Phe
        195                 200                 205

Gly Gly Asp Pro Asn Lys Val Thr Ile Trp Gly Glu Ser Ala Gly Ala
    210                 215                 220

Ile Ser Val Phe Asn Gln Met Ala Leu Tyr Asn Gly Asp Asn Thr Tyr
225                 230                 235                 240

Lys Gly Lys Pro Leu Phe Arg Gly Ala Ile Met Asn Ser Gly Ser Ile
                245                 250                 255

Val Pro Ala Asp Pro Val Asp Cys Pro Lys Gly Gln Ala Val Tyr Asp
            260                 265                 270

Gln Val Val Ala Lys Ala Gly Cys Ala Gly Ala Ser Asp Thr Leu Asp
        275                 280                 285

Cys Leu Arg Gln Ala Pro Tyr Asp Lys Phe Leu Ala Ala Ala Asn Ser
    290                 295                 300

Val Pro Ala Ile Leu Ser Tyr Asn Ser Val Ala Leu Ser Tyr Leu Pro
305                 310                 315                 320

Arg Pro Asp Gly Lys Val Ile Thr Lys Ser Pro Asp Val Leu Val Lys
                325                 330                 335

Glu Gly Lys Tyr Ala Ala Val Pro Met Ile Ile Gly Asp Gln Glu Asp
            340                 345                 350

Glu Gly Thr Leu Phe Ala Leu Phe Gln Ser Asn Val Thr Ser Thr Asp
        355                 360                 365

Lys Leu Val Glu Tyr Leu Gly Gly Leu Phe Phe His Gly Ala Thr Thr
    370                 375                 380

Glu Gln Ile Glu Gly Leu Val Asn Thr Tyr Ser Thr Ser Ile Leu Ala
385                 390                 395                 400

Gly Ser Pro Phe Arg Ser Gly Ile Leu Asn Glu Ile Tyr Pro Gly Phe
                405                 410                 415

Lys Arg Leu Ala Ala Ile Leu Gly Asp Leu Val Phe Thr Leu Thr Arg
            420                 425                 430

Arg Val Phe Leu Glu Ala Ala Thr Thr Val Asn Pro Asn Val Pro Ala
        435                 440                 445

Trp Ser Tyr Leu Ala Ser Tyr Asp Glu Gly Thr Pro Ile Leu Gly Thr
    450                 455                 460

Phe His Gly Ser Asp Leu Leu Gln Val Phe Tyr Gly Ile Leu Pro Asn
465                 470                 475                 480

Tyr Ala Ser Lys Ser Ile Gln Ser Tyr Tyr Ala Asn Phe Val Tyr Asn
                485                 490                 495

Leu Asp Pro Asn Asp Ala Ser Gly Gly Thr Ser Ala Lys Ser Lys Val
            500                 505                 510

Ala Glu Ser Trp Pro Arg Trp Thr Gly Thr Glu Arg Lys Leu Ile Asn
        515                 520                 525

Phe Phe Ser Asn Arg Ser Gly Tyr Leu Asn Asp Asp Phe Arg Ser Glu
    530                 535                 540

Ser Ala Asn Tyr Ile Ser Ser Asn Val Gly Ala Leu Tyr Ile
545                 550                 555

<210> 5
<211> 558
<212> PRT
<213> Corynascus thermophilus

<400> 5

Ala Pro Pro Glu Pro Pro Thr Asn Val Val Glu Lys Arg Ala Ala Pro
1               5                   10                  15

Thr Val Thr Ile Ala Ser Gly Ser Ile Val Gly Val Asn Arg Leu Ser
            20                  25                  30

Thr Glu Ala Phe Asn Gly Ile Pro Phe Ala Glu Pro Pro Val Gly Pro
        35                  40                  45

Leu Arg Leu Lys Pro Pro Val Arg Leu Asn Ser Ser Leu Gly Val Phe
    50                  55                  60

Asp Ala Ser Gly Ile Ala Pro Ala Cys Pro Gln Phe Phe Ala Asp Ser
65                  70                  75                  80

Asp Ser Asn Glu Phe Leu Pro Lys Val Ile Asp Phe Val Thr Asp Leu
                85                  90                  95

Pro Phe Phe Gln Arg Ala Leu Lys Ile Ser Glu Asp Cys Leu Thr Val
            100                 105                 110

Asn Val Ile Arg Pro Lys Gly Thr Lys Ala Gly Asp Asn Leu Pro Val
        115                 120                 125

Leu Phe Trp Ile Tyr Gly Gly Gly Phe Glu Leu Gly Trp Ser Ser Met
    130                 135                 140

Tyr Asp Gly Gly Pro Leu Val Met Asn Ala Ile Asn Val Gly Lys Pro
145                 150                 155                 160

Phe Val Phe Val Ala Val Asn Tyr Arg Val Gly Gly Phe Gly Phe Met
                165                 170                 175

Pro Gly Lys Glu Ile Leu Ala Asp Gly Ser Ala Asn Leu Gly His Leu
            180                 185                 190

Asp Gln Arg Met Ala Leu Glu Trp Val Ala Asp Asn Ile Ala Ala Phe
        195                 200                 205

Gly Gly Asp Pro Asn Lys Val Thr Ile Trp Gly Glu Ser Ala Gly Ala
    210                 215                 220

Ile Ser Val Phe Asn Gln Met Ala Leu Tyr Asp Gly Asp Asn Thr Tyr
225                 230                 235                 240

Lys Gly Lys Pro Leu Phe Arg Gly Ala Ile Met Asn Ser Gly Ser Ile
                245                 250                 255

Ile Pro Ala Asp Pro Val Asp Cys Pro Lys Gly Gln Ala Val Tyr Asp
            260                 265                 270

Gln Val Val Ala Lys Ala Gly Cys Ala Gly Ala Ser Asp Thr Leu Ala
        275                 280                 285

Cys Leu Arg Glu Leu Pro Tyr Asp Lys Phe Leu Ser Ala Ala Asn Ser
    290                 295                 300

Val Pro Ala Ile Leu Ser Tyr Asn Ser Val Ala Leu Ser Tyr Leu Pro
305                 310                 315                 320

Arg Pro Asp Gly Lys Leu Ile Thr Lys Ser Pro Asp Val Leu Val Lys
                325                 330                 335

Glu Arg Lys Tyr Ala Ala Val Pro Met Ile Ile Gly Asp Gln Glu Asp
            340                 345                 350

Glu Gly Thr Leu Phe Ala Leu Phe Gln Ser Asn Val Thr Asn Thr Asp
        355                 360                 365

Lys Leu Val Asn Tyr Leu Gly Gly Leu Phe Phe His Gly Ala Thr Thr
    370                 375                 380

Glu Gln Ile Glu Gly Leu Val Asn Thr Tyr Pro Thr Ser Ile Leu Ala
385                 390                 395                 400

Gly Ser Pro Phe Arg Ser Gly Ile Phe Asn Glu Ile Tyr Pro Gly Phe
                405                 410                 415

Lys Arg Leu Ala Ala Ile Leu Gly Asp Leu Val Phe Thr Leu Thr Arg
            420                 425                 430

Arg Val Phe Leu Glu Thr Ala Thr Ala Val Asn Pro Asp Val Pro Ala
        435                 440                 445

Trp Ser Tyr Leu Ala Ser Tyr Asp Glu Gly Thr Pro Ile Leu Gly Thr
    450                 455                 460

Phe His Gly Ser Asp Leu Leu Gln Val Phe Tyr Gly Ile Leu Pro Asn
465                 470                 475                 480

Tyr Ala Ser Arg Ser Ile Gln Ser Tyr Tyr Ala Asn Phe Val Tyr Asn
                485                 490                 495

Leu Asp Pro Asn Asp Ala Ser Gly Gly Thr Ser Ala Lys Ser Lys Val
            500                 505                 510

Ala Glu Thr Trp Pro Arg Trp Thr Pro Asn Glu Arg Lys Leu Ile Asn
        515                 520                 525

Phe Phe Ser Asn Arg Ser Gly Phe Leu Asn Asp Asp Phe Arg Ser Glu
    530                 535                 540

Ser Ala Asn Tyr Ile Gly Ala Asn Val Gly Val Phe His Val
545                 550                 555

<210> 6
<211> 558
<212> PRT
<213> Myriococcum thermophilum

<400> 6

Ala Pro Pro Glu Pro Pro Ser Arg Thr Val Glu Lys Arg Ala Ala Pro
1               5                   10                  15

Thr Val Thr Ile Ala Ser Gly Ser Ile Val Gly Val Ser Arg Leu Ala
            20                  25                  30

Thr Glu Ala Phe Asn Gly Ile Pro Phe Ala Glu Pro Pro Val Gly Pro
        35                  40                  45

Leu Arg Leu Lys Pro Pro Val Arg Leu Asn Ser Ser Leu Gly Val Phe
    50                  55                  60

Asp Ala Thr Gly Ile Ala Pro Ala Cys Pro Gln Phe Phe Ala Asp Thr
65                  70                  75                  80

Asp Ser Asn Glu Phe Leu Ala Arg Val Ile Asp Phe Val Thr Asp Leu
                85                  90                  95

Pro Phe Phe Gln Lys Ala Leu Lys Val Ser Glu Asp Cys Leu Thr Val
            100                 105                 110

Asn Val Ile Arg Pro Lys Gly Thr Lys Ala Gly Asp Asn Leu Pro Val
        115                 120                 125

Leu Phe Trp Ile Tyr Gly Gly Gly Phe Glu Leu Gly Trp Ser Ser Met
    130                 135                 140

Tyr Asp Gly Gly Pro Leu Val Thr Asn Ala Ile Asp Met Gly Lys Pro
145                 150                 155                 160

Phe Ile Phe Val Ala Val Asn Tyr Arg Val Gly Gly Phe Gly Phe Met
                165                 170                 175

Pro Gly Lys Glu Ile Leu Ala Asp Gly Ser Ala Asn Leu Gly His Leu
            180                 185                 190

Asp Gln Arg Met Gly Leu Glu Trp Val Ala Asp Asn Ile Ala Ala Phe
        195                 200                 205

Gly Gly Asp Pro Asn Lys Val Thr Ile Trp Gly Glu Ser Ala Gly Ala
    210                 215                 220

Ile Ser Val Phe Asn Gln Met Ala Leu Tyr Asp Gly Asp Asn Thr Tyr
225                 230                 235                 240

Asn Gly Lys Pro Leu Phe Arg Gly Ala Ile Met Asn Ser Gly Ser Ile
                245                 250                 255

Val Pro Ala Ala Pro Val Asp Cys Pro Lys Gly Gln Ala Val Tyr Asp
            260                 265                 270

Gln Val Val Ala Lys Ala Gly Cys Ala Gly Ala Ser Asp Thr Leu Ala
        275                 280                 285

Cys Leu Arg Glu Leu Pro Tyr Asp Lys Phe Leu Glu Ala Ala Asn Ser
    290                 295                 300

Val Pro Ala Ile Leu Ser Tyr His Ser Val Ala Leu Ser Tyr Leu Pro
305                 310                 315                 320

Arg Pro Asp Gly Lys Val Ile Thr Gln Ser Pro Asp Ile Leu Val Lys
                325                 330                 335

Glu Arg Lys Tyr Ala Ala Val Pro Met Ile Ile Gly Asp Gln Glu Asp
            340                 345                 350

Glu Gly Thr Leu Phe Ala Leu Phe Gln Pro Asn Ile Thr Asn Thr Asn
        355                 360                 365

Lys Leu Val Asp Tyr Leu Gly Ser Leu Phe Phe His Glu Ala Thr Thr
    370                 375                 380

Asn Gln Ile Glu Gly Leu Val Asn Thr Tyr Ser Thr Ser Ile Leu Asp
385                 390                 395                 400

Gly Ser Pro Phe Arg Ser Gly Ile Leu Asn Glu Ile Tyr Pro Gly Phe
                405                 410                 415

Lys Arg Leu Ala Ala Ile Leu Gly Asp Leu Val Phe Thr Leu Thr Arg
            420                 425                 430

Arg Val Phe Leu Glu Thr Ala Thr Ala Val Asn Pro Asp Val Pro Ala
        435                 440                 445

Trp Ser Tyr Leu Ala Ser Tyr Asp Glu Gly Thr Pro Ile Leu Gly Thr
    450                 455                 460

Phe His Gly Ser Asp Ile Leu Gln Val Phe Tyr Gly Ile Leu Pro Asn
465                 470                 475                 480

Tyr Ala Ser Arg Ser Ile Gln Ser Tyr Tyr Ala Asn Phe Val Tyr Asn
                485                 490                 495

Leu Asp Pro Asn Asp Ala Ser Gly Gly Thr Ser Ala Lys Ser Lys Val

500                 505                 510

Arg Glu Asp Trp Pro Arg Trp Thr Ala Thr Glu Arg Lys Leu Ile Asn
        515                 520                 525

Phe Phe Ser Asn Arg Ser Gly Tyr Leu Lys Asp Asp Phe Arg Ser Lys
    530                 535                 540

Ser Ala Gly Tyr Ile Ser Glu Asn Val Gly Ala Leu Tyr Phe
545                 550                 555

<210>  7
<211>  559
<212>  PRT
<213>  Thielavia australiensis

<400>  7

Ala Pro Pro Glu Pro Pro Ser Lys Val Glu Glu Lys Arg Ala Ala Pro
1               5                   10                  15

Thr Val Ser Ile Ser Ser Gly Thr Phe Val Gly Val Ser Gly Thr Thr
            20                  25                  30

Thr Glu Ala Phe Asn Gly Ile Pro Tyr Ala Leu Pro Pro Val Gly Pro
        35                  40                  45

Leu Arg Leu Lys Pro Pro Val Arg Leu Asn Leu Ser Leu Gly Thr Phe
    50                  55                  60

Asp Ala Ser Gly Ile Ala Pro Ala Cys Pro Gln Phe Phe Ala Asp Ser
65                  70                  75                  80

Asp Ser Asn Asp Leu Leu Ala Gln Val Ile Asp Thr Val Thr Asp Leu
                85                  90                  95

Pro Phe Phe Gln Lys Ala Leu Lys Ile Ser Glu Asp Cys Leu Ser Val
            100                 105                 110

Asn Val Ile Arg Pro Lys Gly Val Lys Ala Gly Asp Lys Leu Pro Val
        115                 120                 125

Leu Phe Trp Ile Phe Gly Gly Gly Phe Glu Leu Gly Trp Thr Ser Met
    130                 135                 140

Tyr Asp Ala Gly Pro Leu Val Thr Ser Ala Val Gln Met Gly Lys Pro
145                 150                 155                 160

Tyr Val Phe Val Ala Val Asn Tyr Arg Val Gly Gly Phe Gly Phe Met

165                 170                 175

Pro Gly Lys Glu Ile Leu Ala Asp Gly Ala Ala Asn Leu Gly His Leu
            180                 185                 190

Asp Gln Arg Met Gly Leu Glu Trp Val Ala Asp Asn Ile Ala Ala Phe
        195                 200                 205

Gly Gly Asp Pro Asp Lys Val Thr Ile Trp Gly Glu Ser Ala Gly Ala
    210                 215                 220

Ile Ser Val Leu Asn Gln Met Ala Leu Tyr Asp Gly Asp Asn Thr Tyr
225                 230                 235                 240

Asn Gly Lys Pro Leu Phe Arg Gly Ala Ile Met Asp Ser Gly Ser Val
                245                 250                 255

Val Pro Ala Asp Pro Val Asp Cys Pro Lys Gly Gln Ala Val Tyr Asp
            260                 265                 270

Ala Val Val Ala Lys Ala Gly Cys Ser Ala Ala Lys Asp Thr Leu Ala
        275                 280                 285

Cys Leu Arg Ala Leu Pro Tyr Asp Glu Phe Leu Asp Ala Ala Asn Ser
    290                 295                 300

Val Pro Ala Ile Leu Ser Tyr Asn Ser Leu Ala Leu Ser Tyr Met Pro
305                 310                 315                 320

Arg Pro Asp Gly Lys Val Leu Thr Gln Ser Ser Asp Tyr Leu Ile Lys
                325                 330                 335

Glu Gly Arg Tyr Ala Ala Val Pro Met Ile Ile Gly Asp Gln Glu Asp
            340                 345                 350

Glu Gly Thr Leu Phe Ala Leu Phe Gln Pro Asn Val Thr Thr Thr Asp
        355                 360                 365

Asp Leu Val Gly Tyr Leu Ala Ser Leu Phe Phe His Gly Ala Thr Arg
    370                 375                 380

Gln Gln Ile Glu Gly Leu Val Asp Thr Tyr Ser Pro Trp Val Trp Asp
385                 390                 395                 400

Gly Ser Pro Phe Gly Thr Gly Leu Leu Asn Glu Val Tyr Pro Gly Phe
                405                 410                 415

Lys Arg Thr Ala Ala Leu Leu Gly Asp Ile Val Phe Thr Leu Thr Arg
            420                 425                 430

Arg Val Phe Leu Glu Gln Ala Val Gln Val Asn Pro Thr Val Pro Ala
        435                 440                 445

Trp Ser Tyr Leu Ala Ser Tyr Asp Phe Gly Thr Pro Val Leu Gly Thr
    450                 455                 460

Phe His Gly Ser Asp Leu Leu Gln Val Phe Tyr Gly Leu Leu Pro Asn
465                 470                 475                 480

Cys Ala Ser Lys Ser Ile Gln Ser Tyr Tyr Ala Asn Phe Val Tyr Asn
                485                 490                 495

Leu Asp Pro Asn Asp Ala Ser Gly Gly Thr Ser Gly Arg Gly Ser Lys
            500                 505                 510

Val Ala Glu Asn Trp Pro Arg Trp Thr Gly Glu Gln Arg Thr Leu Ile
        515                 520                 525

Arg Phe Tyr Asp Asp Arg Asn Glu Tyr Leu Glu Asp Asp Phe Arg Ala
    530                 535                 540

Glu Ala Ala Lys Tyr Leu Ala Asp Asn Ala Asp Phe Leu His Val
545                 550                 555

<210> 8
<211> 558
<212> PRT
<213> Thielavia terestris

<400> 8

Ala Pro Pro Ala Pro Pro Ser Arg Val Ile Glu Lys Arg Ser Ala Pro
1               5                   10                  15

Thr Val Val Ile Ser Ser Gly Thr Ile Val Gly Val Ser Arg Glu Asp
            20                  25                  30

Thr Glu Ala Phe Thr Gly Ile Pro Phe Ala Gln Ala Pro Val Gly Pro
        35                  40                  45

Leu Arg Leu Lys Pro Pro Val Arg Leu Asn Ser Ser Leu Gly Thr Phe
    50                  55                  60

Asp Ala Ser Gln Thr Ala Pro Ala Cys Pro Gln Phe Leu Ala Asn Thr
65                  70                  75                  80

Ala Ser Asp Asp Leu Leu Ser Glu Val Leu Thr Thr Val Ser Asn Leu
                85                  90                  95

Pro Phe Phe Gln Lys Ala Leu Lys Ile Ser Glu Asp Cys Leu Asn Ile
            100                 105                 110

Asp Val Tyr Arg Pro Val Gly Thr Lys Ala Gly Asp Asn Leu Pro Val
        115                 120                 125

Leu Phe Trp Ile Phe Gly Gly Gly Phe Glu Leu Gly Trp Ser Ser Met
    130                 135                 140

Tyr Asp Gly Gly Pro Leu Val Ser Gly Ala Val Ser Leu Gly Lys Pro
145                 150                 155                 160

Tyr Ile Leu Val Ala Val Asn Tyr Arg Val Gly Gly Phe Gly Phe Met
                165                 170                 175

Pro Gly Lys Glu Ile Leu Ala Asp Gly Ala Ala Asn Leu Gly Leu Leu
            180                 185                 190

Asp Gln Arg Met Gly Leu Glu Trp Val Ala Asp Asn Ile Ala Ala Phe
        195                 200                 205

Gly Gly Asp Pro Asp Arg Val Thr Ile Trp Gly Glu Ser Ala Gly Ala
    210                 215                 220

Ile Ser Val Phe Asp Gln Met Ala Leu Tyr Asp Gly Asp Asn Thr Tyr
225                 230                 235                 240

Lys Gly Lys Pro Leu Phe Arg Gly Ala Ile Met Asn Ser Gly Ser Ile
                245                 250                 255

Val Pro Ala Asp Pro Val Asp Cys Pro Lys Gly Gln Val Val Tyr Asp
            260                 265                 270

Thr Val Val Lys Gln Ala Gly Cys Ala Ser Ser Gln Asp Thr Leu Ala
        275                 280                 285

Cys Leu Arg Ala Leu Pro Tyr Asp Lys Phe Leu Ala Ala Ala Asn Ser
    290                 295                 300

Val Pro Ala Ile Leu Ser Tyr Ser Ser Val Ala Leu Ser Tyr Leu Pro
305                 310                 315                 320

Arg Pro Asp Gly Lys Ala Leu Thr Glu Ser Pro Asp Val Leu Val Lys
                325                 330                 335

Glu Gly Lys Tyr Ala Ala Val Pro Met Ile Ile Gly Asp Gln Glu Asp
        340                 345                 350

Glu Gly Thr Leu Phe Ala Leu Phe Gln Ser Asn Ile Thr Thr Ser Asp
        355                 360                 365

Lys Leu Ala Asn Tyr Leu Gly Thr Leu Phe Phe Asn Asp Ala Ser Gln
    370                 375                 380

Ala Gln Val Gln Ala Leu Val Gly Gln Tyr Ser Pro Phe Val Trp Asp
385                 390                 395                 400

Gly Ser Pro Phe Gly Thr Gly Leu Leu Asn Glu Val Tyr Pro Gly Phe
                405                 410                 415

Lys Arg Leu Ala Ala Met Leu Gly Asp Leu Val Phe Thr Leu Thr Arg
        420                 425                 430

Arg Val Phe Leu Gln Val Ala Thr Ser Val Asn Pro Ser Val Pro Ala
        435                 440                 445

Trp Ser Tyr Leu Ala Ser Tyr Asp Ala Gly Thr Pro Ile Leu Gly Thr
    450                 455                 460

Phe His Gly Ser Asp Ile Leu Gln Val Phe Phe Gly Ile Leu Pro Asn
465                 470                 475                 480

Tyr Ala Ser Arg Ser Ile Gln Ser Tyr Tyr Ala Asn Phe Val Tyr Asn
                485                 490                 495

Leu Asp Pro Asn Asp Ala Ser Gly Gly Thr Ser Ala Gln Ser Lys Val
        500                 505                 510

Ala Glu Asp Trp Pro Gln Trp Thr Gly Ala Asp Arg Asn Met Ile Gln
        515                 520                 525

Phe Phe Ala Asp His Ser Gly Ser Leu Val Asp Asp Phe Arg Ser Gly
    530                 535                 540

Ala Ala Asp Phe Ile Ala Ala Asn Val Asp Ile Leu His Ile
545                 550                 555

<210>  9
<211>  558
<212>  PRT
<213>  Scytalidium thermophilum

<400>  9

Ala Pro Pro Glu Pro Pro Thr Arg Val Ile Glu Lys Arg Ala Asn Pro
1               5                   10                  15

Thr Val Thr Ala Pro Ala Gly Lys Ile Ile Gly Val Glu Arg Val Gly
            20                  25                  30

Thr Glu Ala Phe Asn Gly Ile Pro Phe Ala Leu Pro Pro Thr Gly Ser
        35                  40                  45

Leu Arg Leu Lys Pro Pro Val Arg Leu Asn Thr Ser Leu Ser Asn Phe
    50                  55                  60

Asp Ala Thr Gly Ile Ala Pro Ala Cys Pro Gln Phe Phe Ala Asp Ser
65                  70                  75                  80

Asp Asp Lys Gly Leu Leu Ser Met Val Leu Asp Asn Ile Thr Gly Leu
                85                  90                  95

Pro Phe Phe Gln Lys Ala Leu Lys Val Ser Glu Asp Cys Leu Thr Val
            100                 105                 110

Asn Val Ile Arg Pro Lys Gly Thr Lys Ala Gly Asp Lys Leu Pro Val
        115                 120                 125

Leu Phe Trp Ile Tyr Gly Gly Gly Phe Glu Leu Gly Trp Ser Ser Met
    130                 135                 140

Tyr Asp Gly Gly Pro Ile Val Thr Ser Ala Ala Ser Leu Gly Lys Pro
145                 150                 155                 160

Phe Val Phe Val Ala Val Asn Tyr Arg Val Gly Gly Phe Gly Phe Met
                165                 170                 175

Pro Gly Lys Glu Ile Leu Ala Asp Gly Ser Ala Asn Leu Gly Leu Leu
            180                 185                 190

Asp Gln Arg Met Gly Leu Glu Trp Val Ala Asp Asn Ile Glu Ala Phe
        195                 200                 205

Gly Gly Asp Pro Thr Lys Val Thr Ile Trp Gly Glu Ser Ala Gly Ala
    210                 215                 220

Ile Ser Val Trp Asn Gln Met Ala Leu Tyr Asp Gly Asn Asn Lys Tyr
225                 230                 235                 240

Lys Gly Lys Pro Leu Phe Arg Gly Ala Ile Met Asn Ser Gly Ser Leu
                245                 250                 255

Val Pro Thr Asn Pro Val Asp Ser Pro Lys Ala Gln Gly Ile Tyr Asp
        260                 265                 270

Ala Val Val Lys Lys Ala Gly Cys Ser Ser Ala Lys Asp Thr Leu Asn
    275                 280                 285

Cys Leu Arg Gln Leu Ser Tyr Ser Lys Phe Leu Asp Ala Ala Asn Ser
    290                 295                 300

Val Pro His Val Leu Ser Tyr Asn Ser Val Ala Leu Ser Tyr Leu Pro
305                 310                 315                 320

Arg Pro Asp Gly Lys Thr Leu Thr Lys Ser Pro Glu Leu Leu Ala Lys
                325                 330                 335

Glu Gly Lys Tyr Ala Ala Val Pro Met Ile Ile Gly Asp Gln Glu Asp
            340                 345                 350

Glu Gly Thr Leu Phe Ala Leu Phe Gln Pro Asn Leu Thr Ser Thr Ser
        355                 360                 365

Arg Leu Val Asp Tyr Phe Lys Asn Ile Tyr Phe His Asp Ala Pro Lys
    370                 375                 380

Ser Lys Ile Glu Gly Leu Val Asp Gln Tyr Ser Ser Trp Val Trp Asp
385                 390                 395                 400

Gly Ser Pro Phe Gly Ser Gly Leu Leu Tyr Asp Ile Tyr Pro Gly Phe
                405                 410                 415

Lys Arg Leu Ala Ala Ile Leu Gly Asp Ile Val Phe Thr Leu Thr Arg
            420                 425                 430

Arg Thr Phe Leu Glu Tyr Ala Thr Ala Ala Asn Pro Asn Val Pro Ala
        435                 440                 445

Trp Ser Tyr Ile Ala Ser Tyr Tyr Arg Gly Thr Pro Ile Leu Gly Thr
    450                 455                 460

Phe His Ala Ser Asp Ile Leu Gln Val Phe Tyr Gly Val Leu Pro Asn
465                 470                 475                 480

Tyr Ala Ser Arg Ser Ile His Ser Phe Tyr Ala Asn Phe Val Tyr Asn
                485                 490                 495

Leu Asp Pro Asn Asn Ala Ser Gly Gly Thr Ser Ser Lys Ser Lys Val

500                 505                 510

Ser Glu Ser Trp Pro Arg Trp Thr Ala Lys Asp Arg Lys Leu Ile Asn
        515                 520                 525

Phe Phe Trp Asp Lys Ser Glu Phe Ile Lys Asp Asp Phe Arg Glu Gly
    530                 535                 540

Ala Gly Lys Tyr Ile Ser Ser Asn Ile Gln Tyr Leu Arg Val
545                 550                 555

<210>  10
<211>  544
<212>  PRT
<213>  Malbranchea cinnamomea

<400>  10

Ala Pro Glu Lys Arg Ala Ala Ser Pro Val Val Thr Ile Ala His Pro
1               5                   10                  15

Glu Ala Thr Ile Ile Gly Ser Ser Ala Leu Ser Val Glu Thr Phe Asn
            20                  25                  30

Asp Ile Pro Phe Ala Ala Pro Pro Thr Gly Pro Leu Arg Leu Lys Pro
        35                  40                  45

Pro Lys Pro Leu Asp Gly Ala Leu Gly Thr Val Asp Ala Thr Thr Leu
    50                  55                  60

Ile Pro Lys Ser Cys Pro Gln Phe Tyr Phe Ser Ile Asp Gln Gly Ala
65                  70                  75                  80

Ile Pro Gly Asp Ile Leu Gly Asp Leu Met Asn His Pro Leu Leu Gln
                85                  90                  95

Lys Val Thr Asn Ala Gly Glu Asp Cys Leu Tyr Leu Asn Val Gln Arg
            100                 105                 110

Pro Arg Gly Thr Lys Ala Gly Asp Lys Leu Pro Val Leu Phe Trp Ile
        115                 120                 125

Tyr Gly Gly Gly Phe Gln Leu Gly Ser Thr Gln Leu Tyr Asn Gly Ala
    130                 135                 140

Ser Leu Val Gln Glu Ser Met Arg Gln Gly Lys Pro Ile Ile Phe Val
145                 150                 155                 160

Ala Val Asn Tyr Arg Val Gly Gly Phe Gly Phe Leu Pro Gly Ala Glu

                165                 170                 175

Val Leu Ala Asp Gly Ser Ala Asn Leu Gly Leu Leu Asp Gln Arg Leu
            180                 185                 190

Gly Leu Gln Trp Val Ala Asp Asn Ile Glu Ala Phe Gly Gly Asp Pro
        195                 200                 205

Glu Lys Val Thr Ile Trp Gly Glu Ser Ala Gly Ala Ile Ser Val Ala
    210                 215                 220

Ser His Met Thr Met Tyr Asp Gly Asp His Thr Tyr Lys Gly Lys Pro
225                 230                 235                 240

Leu Phe Arg Gly Ala Ile Met Asn Ser Gly Ser Gly Ile Pro Thr Asp
                245                 250                 255

Pro Val Asp Cys Pro Lys Ala Gln Ala Val Tyr Asp Ser Val Val Lys
            260                 265                 270

Tyr Ala Gly Cys Asp Thr Ala Ser Asp Thr Leu Glu Cys Leu Arg Gly
        275                 280                 285

Leu Asp Tyr Glu Glu Phe Leu Asp Ala Ala Asn Ala Val Pro Gly Ile
    290                 295                 300

Leu Ser Tyr Ser Ser Val Ala Leu Ser Tyr Leu Pro Arg Pro Asp Gly
305                 310                 315                 320

Thr Ala Phe Thr Glu Ser Pro Asp Leu Leu Ile Glu Lys Gly Lys Tyr
                325                 330                 335

Ala Lys Val Pro Phe Ile Ile Gly Asp Gln Glu Asp Glu Gly Thr Leu
            340                 345                 350

Phe Ala Leu Phe Gln Pro Asn Ile Thr Thr Arg Gly Gln Ile Val Asp
        355                 360                 365

Tyr Leu Ser Glu Arg Phe Phe His His Ala Asp Lys Ala Thr Ile Lys
    370                 375                 380

Gly Leu Val Asp Thr Tyr Gln Thr Ile Ser Ile Asp Gly Ser Pro Phe
385                 390                 395                 400

Arg Thr Gly Leu Leu Asn Asn Trp Tyr Pro Gln Phe Lys Arg Leu Ala
                405                 410                 415

Ala Ile Leu Gly Asp Leu Thr Phe Thr Ile Thr Arg Arg Val Val Leu
            420                 425                 430

Asp Ile Ile Asn Arg Val Ser Pro Glu Val Pro Ser Trp Ser Tyr Leu
        435                 440                 445

Ala Thr Tyr Asp Tyr Gly Thr Pro Ile Met Gly Thr Phe His Gly Ser
    450                 455                 460

Asp Ile Leu Gln Val Phe His Gly Ile Trp Pro Asn Tyr Ala Ala Arg
465                 470                 475                 480

Thr Ile Arg Gly Tyr Tyr Phe Asn Phe Val Tyr Asn Leu Asp Pro Asn
                485                 490                 495

Asp Gly Lys Leu Pro His Trp Pro Arg Trp Ser Glu Lys Arg Gln Leu
            500                 505                 510

Ala Glu Phe Gln Ala His Arg Val Arg Leu Leu Ala Asp Asn Phe Arg
        515                 520                 525

Ser Asp Thr Tyr Asp Tyr Leu Val Lys Asn Ile Lys Lys Phe Tyr Tyr
    530                 535                 540

<210>  11
<211>  554
<212>  PRT
<213>  Thermomyces stellatus

<400>  11

Ala Pro Thr Ala Thr Leu Asp Asp Arg Ala Ala Lys Thr Ala Thr Val
1               5                   10                  15

Glu Ile Pro Ser Pro Glu Ala Thr Val Leu Gly Asn Val Lys Asn Lys
            20                  25                  30

Val Glu Ser Phe Gly Gly Ile Pro Tyr Ala His Pro Pro Thr Gly Asp
        35                  40                  45

Leu Arg Met Arg Pro Pro Lys Arg Leu Thr Glu Pro Met Gly Thr Phe
    50                  55                  60

Asp Gly Thr Gly Pro Ala Gly Ala Cys Pro Gln Phe Val Ala Ser Asn
65                  70                  75                  80

Asp Ala Lys His Phe Leu Leu Asp Thr Leu Gly Thr Ile Ala Asn Leu
                85                  90                  95

Pro Phe Val Gln Asn Val Thr Gly Gln Ser Glu Asp Cys Leu Ser Ile
            100                 105                 110

Thr Val Ala Arg Pro Glu Gly Thr Lys Ala Asp Ala Lys Leu Pro Val
        115                 120                 125

Leu Tyr Trp Ile Phe Gly Gly Gly Phe Glu Leu Gly Trp Ser Ser Met
    130                 135                 140

Tyr Asp Gly Thr Asp Leu Ile Asn Tyr Gly Val Glu Ile Gly Lys Pro
145                 150                 155                 160

Phe Ile Phe Val Ala Val Asn Tyr Arg Val Gly Gly Tyr Gly Phe Met
                165                 170                 175

Pro Gly Lys Glu Leu Gln Asp Glu Gly Ser Ser Asn Ala Gly Leu Leu
            180                 185                 190

Asp Gln Arg Met Gly Leu Glu Trp Val Ala Asp Asn Ile Ala Ala Phe
        195                 200                 205

Gly Gly Asp Pro Asp Lys Val Thr Ile Trp Gly Glu Ser Ala Gly Ala
    210                 215                 220

Ile Ser Val Phe Ala Gln Met Ala Leu Phe Asp Gly Asp Asn Thr Tyr
225                 230                 235                 240

Lys Gly Lys Pro Leu Phe Arg Gly Gly Ile Met Ser Ser Gly Ser Ile
                245                 250                 255

Val Pro Thr Lys Pro Met Asp Ser Asp Lys Ala Gln Ala Val Phe Asp
            260                 265                 270

Lys Val Val Glu Thr Gly Gly Cys Ser Asp Ala Asp Asp Lys Leu Lys
        275                 280                 285

Cys Leu Arg Asp Leu Ser Tyr Asp Asp Phe Leu Asn Ala Ala Thr Ser
    290                 295                 300

Val Pro His Leu Leu Ser Phe Asn Ser Leu Ala Leu Ser Tyr Leu Pro
305                 310                 315                 320

Arg Pro Asp Gly Arg Thr Leu Thr Asp Ser Pro Asp Gln Leu Val Leu
                325                 330                 335

Asn Glu Lys Tyr Ala Ala Val Pro Met Ile Ile Gly Asp Gln Glu Asp
            340                 345                 350

Glu Gly Thr Leu Phe Ala Leu Phe Gln Ser Glu Leu Thr Thr Lys Asp
        355                 360                 365

Lys Leu Val Thr Tyr Leu Lys Asp Tyr Tyr Phe Asp Asp Ala Thr Glu
    370                 375                 380

Glu Gln Leu Ser Asp Leu Val Asp Thr Tyr Gly Ser Gly Leu Ser Ala
385                 390                 395                 400

Ile Arg Glu Gly Ser Pro His Arg Thr Gly Leu Leu Asn Glu Ile Phe
                405                 410                 415

Pro Gly Phe Lys Arg Arg Ala Ala Ile Leu Gly Asp Leu Val Phe Thr
            420                 425                 430

Leu Ser Arg Arg Leu Phe Leu Leu Ala Thr Ser Thr Leu Asn Lys Asp
        435                 440                 445

Val Pro Ser Trp Ser Tyr Leu Met Ser Gln Asn Glu Gly Phe Pro Ile
    450                 455                 460

Leu Gly Thr Phe His Gly Ala Asp Ile Phe Gln Thr Phe Phe Gly Ile
465                 470                 475                 480

Lys Asp Asn Tyr Ala Ala Lys Thr Val Arg Gly Tyr Phe Ile Asn Phe
                485                 490                 495

Val Tyr Ser Gln Asp Pro Asn Gly Asp Gly Asp Thr Ser Phe Pro Glu
            500                 505                 510

Trp Pro Gln Trp Ser Asp Gly Asn Lys Leu Ala His Val Phe Ala Asp
        515                 520                 525

Arg Ser Glu Leu Leu Asp Asp Asp Phe Arg Glu Asp Ser Phe Asn Trp
    530                 535                 540

Leu Ser Glu Asn Ile Asp Val Leu Arg Phe
545                 550

<210> 12
<211> 558
<212> PRT
<213> Chaetomium globosum

<400> 12

Ala Pro Pro Glu Pro Pro Ala Asn Val Ile Glu Lys Arg Ala Ala Pro
1               5                   10                  15

Thr Val Thr Val Ser Ala Gly Ser Ile Val Gly Thr Thr Arg Leu Ser
            20                  25                  30

Thr Glu Ala Phe Asn Gly Ile Pro Phe Ala Lys Pro Pro Val Gly Pro
        35                  40                  45

Leu Arg Leu Lys Pro Pro Val Arg His Asp Ser Pro Leu Gly Val Phe
    50                  55                  60

Asp Ala Ser Gly Ile Ala Pro Ala Cys Pro Gln Phe Leu Ala Asp Ser
65                  70                  75                  80

Asp Ser Asn Glu Phe Leu Ala Lys Val Ile Gly Thr Leu Thr Asn Leu
                85                  90                  95

Pro Phe Phe Gln Lys Ala Leu Lys Ile Ser Glu Asp Cys Leu Thr Val
            100                 105                 110

Asn Val Phe Arg Pro Lys Gly Thr Lys Ala Gly Asp Lys Leu Pro Val
        115                 120                 125

Leu Phe Trp Ile Phe Gly Gly Gly Phe Glu Leu Gly Trp Ser Ser Met
    130                 135                 140

Tyr Asp Gly Gly Pro Leu Val Thr Asn Ala Met Asn Met Gly Lys Pro
145                 150                 155                 160

Tyr Val Phe Val Ala Ile Asn Tyr Arg Val Gly Gly Phe Gly Phe Met
                165                 170                 175

Pro Gly Lys Glu Ile Leu Ala Asp Gly Ser Gly Asn Leu Gly His Leu
            180                 185                 190

Asp Gln Arg Met Gly Leu Glu Trp Val Ala Asp Asn Ile Ala Ala Phe
        195                 200                 205

Gly Gly Asp Pro Asp Lys Val Thr Ile Trp Gly Glu Ser Ala Gly Ala
    210                 215                 220

Ile Ser Val Phe Asn Gln Met Ala Leu Tyr Asp Gly Asp Asn Thr Tyr
225                 230                 235                 240

Lys Gly Lys Pro Leu Phe His Gly Ala Ile Met Asn Ser Gly Ser Ile
                245                 250                 255

Val Pro Ala Asp Pro Val Asp Cys Pro Lys Gly Gln Ala Ile Tyr Asp
            260                 265                 270

Gln Val Val Glu Lys Ala Gly Cys Ala Ser Ala Lys Asp Thr Leu Ala
        275                 280                 285

Cys Leu Arg Glu Ala Pro Tyr Asp Lys Phe Leu Ala Ala Ala Asn Ser
    290                 295                 300

Val Pro Ala Ile Leu Ser Tyr Asn Ser Val Ala Leu Ser Tyr Leu Pro
305                 310                 315                 320

Arg Pro Asp Gly Lys Ala Leu Thr Lys Ser Thr Asp Val Leu Val Gln
                325                 330                 335

Glu Gly Lys Tyr Ala Ala Val Pro Met Ile Ile Gly Asp Gln Glu Asp
            340                 345                 350

Glu Gly Thr Leu Phe Ala Leu Phe Gln Pro Asn Ile Thr Thr Ser Ala
        355                 360                 365

Arg Leu Val Thr Tyr Leu Lys Asp Leu Tyr Phe His Arg Ala Thr Asn
    370                 375                 380

Gln Gln Ile Glu Gly Leu Val Asp Thr Tyr Ser Pro Trp Ile Trp Asp
385                 390                 395                 400

Gly Ser Pro Phe Gly Ser Gly Ile Leu Asn Glu Ile Tyr Pro Gly Phe
                405                 410                 415

Lys Arg Leu Ala Ala Leu Leu Gly Asp Ile Val Phe Thr Leu Thr Arg
            420                 425                 430

Arg Ile Phe Leu Glu Thr Ala Thr Ala Val Asn Pro Asp Val Pro Ala
        435                 440                 445

Trp Ser Tyr Ile Ala Ser Tyr Asn Gln Gly Thr Pro Ile Met Gly Thr
    450                 455                 460

Phe His Gly Ser Asp Ile Leu Gln Val Phe Tyr Gly Ile Leu Pro Asn
465                 470                 475                 480

Tyr Ala Ser Lys Ser Ile Gln Ser Tyr Tyr Ala Asn Phe Val Tyr Asn
                485                 490                 495

Gln Asp Pro Asn Asn Ala Ala Gly Gly Thr Ser Ser Lys Ser Lys Ile
            500                 505                 510

Ser Glu Asp Trp Pro Arg Trp Thr Gly Lys Asp Arg Lys Leu Ile Asn

515                 520                 525

Phe Phe Ala Asn Arg Asn Gly Tyr Leu Lys Asp Asp Phe Arg Glu Glu
    530                 535                 540

Ala Ala Ala Tyr Leu Gly Ser His Ile Gly Ala Leu Tyr Phe
545                 550                 555

<210>  13
<211>  559
<212>  PRT
<213>  Madurella mycetomatis

<400>  13

Ala Pro Pro Gln Pro Pro Ala Lys Val Val Glu Gln Arg Ala Ala Pro
1               5                   10                  15

Thr Val Thr Ile Ser Thr Gly Thr Ile Ile Gly Ser Ser Arg Leu Ala
            20                  25                  30

Thr Glu Ala Phe Asn Gly Ile Pro Phe Ala Leu Pro Pro Val Gly Pro
        35                  40                  45

Leu Arg Leu Lys Pro Pro Val Arg Leu Asn Ser Thr Ile Gly Val Phe
    50                  55                  60

Asp Gly Ser Gly Ile Ala Pro Ala Cys Pro Gln Phe Leu Ala Asp Thr
65                  70                  75                  80

Asp Ser Asn Glu Leu Leu Ala Ser Ile Ile Asp Leu Val Thr Asn Leu
                85                  90                  95

Pro Phe Phe Gln Lys Ala Leu Lys Ile Ser Glu Asp Cys Leu Thr Ile
            100                 105                 110

Asn Val Ile Arg Pro Lys Gly Thr Lys Ala Gly Asp Lys Leu Pro Val
        115                 120                 125

Leu Phe Trp Ile Phe Gly Gly Gly Phe Glu Leu Gly Trp Thr Ser Met
    130                 135                 140

Tyr Asp Gly Gly Pro Leu Val Thr Asn Ala Met Gln Met Gly Lys Pro
145                 150                 155                 160

Tyr Ile Phe Val Ala Val Asn Tyr Arg Val Gly Gly Phe Gly Phe Met
                165                 170                 175

Pro Gly Lys Glu Ile Leu Lys Asp Gly Ala Ala Asn Leu Gly His Leu

180                 185                 190

Asp Gln Arg Met Gly Leu Glu Trp Val Ala Asp Asn Ile Ala Ala Phe
        195                 200                 205

Gly Gly Asp Pro Asp Lys Val Thr Ile Trp Gly Glu Ser Ala Gly Ala
    210                 215                 220

Ile Ser Val Phe Asn Gln Met Ala Leu Tyr Asp Gly Asn Asn Asn Tyr
225                 230                 235                 240

Asn Gly Arg Pro Leu Phe Arg Ala Ala Ile Met Asn Ser Gly Ser Ile
                245                 250                 255

Ile Pro Ala Asp Pro Val Asp Cys Pro Lys Gly Gln Glu Ile Tyr Asn
            260                 265                 270

Ala Val Val Ala Lys Ala Gly Cys Ala Ser Ala Met Asp Thr Leu Ala
        275                 280                 285

Cys Leu Arg Ala Leu Pro Tyr Glu Lys Phe Leu Ser Ala Ala Asn Ser
    290                 295                 300

Val Pro Ser Val Leu Ser Tyr Asn Ser Val Ala Leu Ser Tyr Met Pro
305                 310                 315                 320

Arg Pro Asp Gly Lys Val Leu Thr Lys Ser Thr Asp Ala Leu Val Phe
                325                 330                 335

Glu Gly Lys Tyr Ala Ala Val Pro Met Ile Ile Gly Ser Gln Glu Asp
            340                 345                 350

Glu Gly Thr Leu Phe Ser Leu Phe Gln Pro Asn Ile Thr Thr Thr Asn
        355                 360                 365

Arg Leu Val Ser Tyr Leu Glu Asp Leu Phe Phe His Gly Ala Ser Arg
    370                 375                 380

Gln Gln Ile Ala Gly Leu Val Ala Lys Tyr Pro Ala Gln Leu Trp Glu
385                 390                 395                 400

Gly Ser Pro Phe Arg Thr Gly Phe Leu Asn Glu Ile Tyr Pro Gly Phe
                405                 410                 415

Lys Arg Met Ala Ala Ile Leu Gly Asp Leu Val Phe Thr Leu Thr Arg
            420                 425                 430

Arg Ile Phe Leu Glu His Ala Val Lys Ala Asn Pro Ala Val Pro Ala
        435                 440                 445

Trp Ser Tyr Ile Ala Ser Tyr Asn Tyr Gly Thr Pro Ile Leu Gly Thr
    450                 455                 460

Phe His Gly Ser Asp Leu Leu Gln Val Phe Tyr Gly Ile Leu Pro Asn
465                 470                 475                 480

Tyr Ala Ser Lys Ser Ile Gln Ser Phe Tyr Ala Asn Phe Val Tyr Asn
                485                 490                 495

Met Asp Pro Asn Asp Ser Ser Gly Gly Thr Asn Ser Gln Ser Ser Lys
            500                 505                 510

Val Lys Glu Lys Trp Pro Gln Trp Thr Gly Glu Gln Arg Thr Leu Ile
        515                 520                 525

Gln Phe Tyr Ala Asp Arg Asn Glu Tyr Leu Glu Asp Asp Phe Arg Ser
    530                 535                 540

Glu Ala Ala Gln Tyr Ile Ala Glu His Val Asn Glu Leu His Val
545                 550                 555

<210>  14
<211>  557
<212>  PRT
<213>  Sordaria macrospora

<400>  14

Ala Pro Ala Glu Pro Pro Thr Gln Val Leu Asp Lys Arg Ala Ala Pro
1               5                   10                  15

Thr Val Thr Ile Ser Thr Gly Thr Ile Val Gly Ala Asn Gly Ile Leu
            20                  25                  30

Thr Glu Gly Phe Asn Gly Ile Pro Phe Ala Leu Pro Pro Thr Gly Asn
        35                  40                  45

Leu Arg Leu Lys Pro Pro Val Arg Leu Lys Ser Ser Leu Gly Val Phe
    50                  55                  60

Asp Ala Ser Gly Ile Ala Pro Ala Cys Pro Gln Phe Leu Ala Asp Thr
65                  70                  75                  80

Ser Ser Asn Glu Phe Leu Pro Lys Val Ile Asp Lys Leu Val Asn Thr
                85                  90                  95

Gln Leu Phe Asn Thr Ala Leu Asn Val Lys Glu Asp Cys Leu Thr Ile
        100                 105                 110

Ser Val Thr Arg Pro Lys Gly Thr Lys Ala Gly Asp Lys Leu Pro Val
    115                 120                 125

Leu Phe Trp Ile Phe Gly Gly Gly Phe Glu Leu Gly Trp Ala Ser Met
    130                 135                 140

Tyr Asp Gly Ala Pro Leu Val Thr Asn Ala Ile Asn Met Gly Lys Pro
145                 150                 155                 160

Tyr Val Tyr Val Ala Val Asn Tyr Arg Val Gly Ala Phe Gly Phe Met
                165                 170                 175

Pro Gly Lys Glu Ile Leu Lys Asp Gly Ala Ser Asn Leu Gly His Leu
            180                 185                 190

Asp Gln Arg Met Gly Leu Gln Trp Val Ala Asp Asn Ile Ala Ala Phe
        195                 200                 205

Gly Gly Asp Pro Asp Lys Val Thr Ile Trp Gly Glu Ser Ala Gly Ala
    210                 215                 220

Ile Ser Val Phe Asn Gln Met Ser Leu Tyr Asn Gly Asp Asn Lys Tyr
225                 230                 235                 240

Asn Gly Lys Pro Leu Phe Arg Gly Ala Ile Met Asn Ser Gly Ser Ile
                245                 250                 255

Val Pro Ala Asp Pro Val Asp Cys Pro Lys Gly Gln Lys Val Tyr Asp
            260                 265                 270

Thr Val Val Arg Asn Ala Gly Cys Ser Gly Ala Ala Asp Thr Leu Ala
        275                 280                 285

Cys Leu Arg Ala Leu Pro Tyr Asp Thr Phe Leu Lys Ala Ala Asn Ser
    290                 295                 300

Val Pro Ala Ile Leu Ser Tyr Thr Ser Val Ala Leu Ser Tyr Leu Pro
305                 310                 315                 320

Arg Pro Asp Gly Lys Val Leu Thr Asp Ser Ala Asp Lys Leu Val Val
                325                 330                 335

Asn Lys Lys Tyr Ala Ala Val Pro Met Ile Ile Gly Asp Gln Glu Asp
            340                 345                 350

Glu Gly Thr Leu Phe Ser Leu Phe Gln Ser Asn Ile Thr Thr Thr Ser
        355                 360                 365

Lys Leu Val Ser Tyr Leu Asn Asp Val Phe Phe His Asp Ala Thr Glu
    370                 375                 380

Ser Gln Ile Lys Ser Leu Val Ser Thr Tyr Ser Pro Leu Leu Ser Ala
385                 390                 395                 400

Gly Ser Pro Phe Gly Thr Gly Ile Phe Asn Glu Ile Tyr Pro Gly Phe
                405                 410                 415

Lys Arg Leu Ala Ala Ile Leu Gly Asp Leu Val Phe Thr Leu Thr Arg
            420                 425                 430

Arg Val Phe Leu Asp Ala Ala Ala Ser Val Asn Pro Ser Val Pro Ala
        435                 440                 445

Trp Ser Tyr Leu Ala Ser Tyr Asp Phe Gly Thr Pro Ile Leu Gly Thr
    450                 455                 460

Phe His Gly Ser Asp Leu Leu Gln Val Phe Tyr Gly Ile Leu Pro Asn
465                 470                 475                 480

Tyr Ala Ser Lys Ser Ile Gln Ser Tyr Tyr Ala Asn Phe Val Tyr Asn
                485                 490                 495

Leu Asp Pro Asn Asp Ala Ser Gly Gly Thr Ser Ser Lys Ser Lys Val
            500                 505                 510

Ala His Asn Trp Pro Gln Trp Gln Lys Glu Arg Lys Leu Val Gln Phe
        515                 520                 525

Phe Ala Asn Tyr Ala Gly Tyr Leu Lys Asp Asp Phe Arg Ser Asp Ser
    530                 535                 540

Tyr Asn Trp Ile Lys Ser Asn Val Asp Ala Leu Tyr Ile
545                 550                 555

<210>  15
<211>  558
<212>  PRT
<213>  Podospora anserina

<400>  15

Ala Pro Pro Glu Pro Pro Thr Gln Val Leu Glu Lys Arg Ala Arg Pro
1               5                   10                  15

Thr Val Ser Ile Ala Gln Gly Asn Val Val Gly Val Ser Arg Ile Asn
            20                  25                  30

Thr Glu Ala Phe Asn Gly Ile Pro Phe Ala Gln Ala Pro Val Gly Pro
        35                  40                  45

Leu Arg Leu Lys Pro Pro Val Arg Thr Thr Ala Ser Phe Gly Thr Tyr
    50                  55                  60

Asp Ala Gly Gly Ile Ala Ala Ala Cys Pro Gln Phe Leu Ala Asp Thr
65                  70                  75                  80

Asp Ser Glu Asp Leu Leu Ala Lys Ile Ile Asp Thr Val Val Asn Thr
                85                  90                  95

Ala Leu Phe Gln Lys Ala Leu Lys Ile Ser Glu Asp Cys Leu Asn Ile
            100                 105                 110

Asn Val Ile Arg Pro Ala Gly Thr Lys Ala Gly Asp Lys Leu Pro Val
        115                 120                 125

Leu Phe Trp Ile Tyr Gly Gly Gly Phe Glu Leu Gly Trp Ser Ser Met
    130                 135                 140

Tyr Asp Gly Gly Pro Leu Val Ser Asn Ala Met Ala Ala Gly Lys Pro
145                 150                 155                 160

Tyr Val Phe Val Ala Val Asn Tyr Arg Val Gly Ala Phe Gly Phe Met
                165                 170                 175

Pro Gly Lys Glu Val Leu Gln Asp Gly Ser Ala Asn Leu Gly His Leu
            180                 185                 190

Asp Gln Arg Met Gly Leu Glu Trp Val Ala Asp Asn Ile Ala Ala Phe
        195                 200                 205

Gly Gly Asp Pro Asp Lys Val Thr Ile Trp Gly Glu Ser Ala Gly Ala
    210                 215                 220

Ile Ser Val Trp Asn Gln Met Ser Leu Tyr Asp Gly Asn Ile Asn Tyr
225                 230                 235                 240

Lys Gly Lys Pro Leu Phe Arg Gly Ala Ile Met Asn Ser Gly Ser Ile
                245                 250                 255

Val Pro Ala Asp Pro Val Asp Cys Pro Lys Gly Gln Glu Ile Tyr Asp
            260                 265                 270

Thr Val Val Ala Arg Ser Gly Cys Gly Gly Ala Ala Asp Thr Leu Asn
        275                 280                 285

Cys Leu Arg Asn Leu Pro Tyr Glu Thr Phe Leu Glu Ala Ala Asn Ser
    290                 295                 300

Val Pro Ala Ile Leu Ser Tyr Ser Ser Val Ala Leu Ser Tyr Leu Pro
305                 310                 315                 320

Arg Pro Asp Gly Val Ala Leu Thr Asp Ser Ala Asp Arg Leu Val Lys
                325                 330                 335

Ala Gly Lys Tyr Ala Ala Val Pro Met Ile Ile Gly Asp Gln Glu Asp
            340                 345                 350

Glu Gly Thr Leu Phe Ser Leu Phe Gln Lys Asn Val Thr Thr Thr Ser
        355                 360                 365

Arg Leu Glu Asp Tyr Leu Arg Gly Tyr Phe Phe His Gly Ala Thr Ala
    370                 375                 380

Gln Gln Val Lys Gly Leu Val Ser Gln Tyr Ser Pro Leu Ile Ser Ala
385                 390                 395                 400

Gly Ser Pro Phe Gly Ser Gly Leu Phe Asn Glu Ile Tyr Pro Gly Phe
                405                 410                 415

Lys Arg Leu Ala Ala Leu Leu Gly Asp Ile Val Phe Thr Leu Thr Arg
            420                 425                 430

Arg Val Phe Ile Glu Tyr Ala Ile Ala Ala Asn Pro Asn Val Pro Val
        435                 440                 445

Trp Ser Tyr Leu Ala Ser Tyr Asn Glu Gly Thr Pro Ile Leu Gly Thr
    450                 455                 460

Phe His Gly Ser Asp Leu Leu Gln Val Phe Tyr Gly Ile Leu Pro Asn
465                 470                 475                 480

Tyr Ala Ser Lys Ser Ile Gln Asn Phe Tyr Ala Asn Phe Val Tyr Asn
                485                 490                 495

Leu Asn Pro Asn Asp Ala Ser Gly Gly Thr Ser Ala Lys Ser Lys Val
            500                 505                 510

Lys Glu Asn Trp Pro Thr Trp Asn Thr Lys Asp Lys Arg Leu Ile Gln

515                 520                 525

Phe Phe Asn Asn Arg Asn Gly Tyr Leu Lys Asp Asp Phe Arg Glu Gly
    530                 535                 540

Ala Lys Gln Tyr Ile Ala Ala Asn Ile Asp Ala Leu His Ile
545                 550                 555

<210>  16
<211>  557
<212>  PRT
<213>  Neurospora tetrasperma

<400>  16

Ala Pro Ala Glu Pro Pro Thr Gln Val Leu Asp Lys Arg Ala Ala Pro
1               5                   10                  15

Thr Val Thr Ile Ser Thr Gly Met Ile Val Gly Ala Asn Gly Ile Leu
            20                  25                  30

Thr Glu Ala Phe Asn Gly Ile Pro Tyr Ala Leu Pro Pro Thr Gly Asn
        35                  40                  45

Leu Arg Leu Lys Pro Pro Val Arg Leu Lys Ser Ser Leu Gly Val Phe
    50                  55                  60

Asp Ala Ser Gly Ile Gly Pro Ala Cys Pro Gln Phe Leu Ala Asp Thr
65                  70                  75                  80

Ser Ser Asn Glu Phe Leu Pro Gln Val Ile Asp Lys Ile Val Asn Thr
                85                  90                  95

Gln Leu Phe Lys Thr Ile Leu Asn Val Lys Glu Asp Cys Leu Thr Ile
            100                 105                 110

Ser Ile Thr Arg Pro Lys Gly Thr Lys Ala Gly Asp Lys Leu Pro Val
        115                 120                 125

Leu Phe Trp Ile Phe Gly Gly Gly Phe Glu Leu Gly Ser Ala Ser Met
    130                 135                 140

Tyr Asp Gly Ala Pro Leu Val Thr Asn Ala Ile Asn Met Gly Lys Pro
145                 150                 155                 160

Tyr Val Tyr Val Ala Val Asn Tyr Arg Val Gly Gly Phe Gly Phe Met
                165                 170                 175

Pro Gly Lys Glu Ile Leu Lys Asp Gly Ser Ser Asn Leu Gly His Leu

180                 185                 190

Asp Gln Arg Met Gly Leu Gln Trp Val Ala Asp Asn Ile Ala Ala Phe
        195                 200                 205

Gly Gly Asp Pro Asp Lys Val Thr Ile Trp Gly Glu Ser Ala Gly Ala
    210                 215                 220

Met Ser Val Phe Asn Gln Met Ser Leu Tyr Asp Gly Asp Asn Thr Tyr
225                 230                 235                 240

Asn Gly Lys Pro Leu Phe Arg Gly Ala Ile Met Asn Ser Gly Ser Ile
                245                 250                 255

Val Pro Ala Gly Pro Val Asp Cys Pro Lys Gly Gln Lys Val Tyr Asp
            260                 265                 270

Thr Val Val Lys Asn Ala Gly Cys Ser Gly Ala Ala Asp Thr Leu Ala
        275                 280                 285

Cys Leu Arg Ala Leu Pro Tyr Glu Thr Phe Leu Lys Ala Ala Asn Ser
    290                 295                 300

Val Pro Gly Ile Leu Ser Tyr Asn Ser Val Ala Leu Ser Tyr Leu Pro
305                 310                 315                 320

Arg Pro Asp Gly Lys Val Leu Thr Gln Ser Ser Asp Lys Leu Met Leu
                325                 330                 335

Ala Lys Lys Tyr Ala Ala Val Pro Met Ile Ile Gly Asp Gln Glu Asp
            340                 345                 350

Glu Gly Thr Leu Phe Ser Leu Phe Gln Ser Asn Ile Thr Thr Thr Ser
        355                 360                 365

Lys Leu Val Ser Tyr Leu Asn Asp Ile Phe Phe Asn Asp Ala Thr Glu
    370                 375                 380

Ser Gln Ile Lys Ser Leu Val Ser Thr Tyr Ser Thr Leu Ile Ser Ala
385                 390                 395                 400

Gly Ser Pro Phe Gly Thr Gly Leu Phe Asn Glu Ile Tyr Pro Gly Phe
                405                 410                 415

Lys Arg Leu Ala Ala Ile Leu Gly Asp Leu Ile Phe Thr Leu Ser Arg
            420                 425                 430

Arg Ile Phe Leu Asp Ala Ala Thr Thr Leu Asn Pro Ser Val Pro Ala
        435                 440                 445

Trp Ser Tyr Leu Ala Ser Tyr Asn Phe Gly Thr Pro Ile Leu Gly Thr
    450                 455                 460

Phe His Ala Ser Asp Ile Leu Gln Val Phe Tyr Gly Ile Leu Pro Asn
465                 470                 475                 480

Tyr Ala Ser Lys Ser Ile Gln Ser Tyr Tyr Ala Asn Phe Val Tyr Asn
                485                 490                 495

Leu Asp Pro Asn Asp Ala Ser Gly Gly Thr Ser Ser Lys Ser Lys Val
            500                 505                 510

Gly Gln Asp Trp Pro Gln Trp Gln Lys Glu Arg Lys Leu Val Gln Phe
        515                 520                 525

Phe Ala Asp Tyr Ala Gly Tyr Leu Thr Asp Asp Phe Arg Ser Glu Ser
    530                 535                 540

Tyr Asn Trp Ile Lys Ala Asn Ile Asp Ala Leu His Ile
545                 550                 555

<210>  17
<211>  557
<212>  PRT
<213>  Neurospora crassa

<400>  17

Ala Pro Ala Glu Pro Pro Thr Gln Val Leu His Lys Arg Ala Ala Pro
1               5                   10                  15

Thr Val Thr Ile Ser Thr Gly Thr Ile Val Gly Ala Asn Gly Ile Leu
            20                  25                  30

Thr Glu Ala Phe Asn Gly Ile Pro Tyr Ala Leu Pro Pro Thr Gly Asn
        35                  40                  45

Leu Arg Leu Lys Pro Pro Val Arg Leu Lys Ser Ser Leu Gly Val Phe
    50                  55                  60

Asp Ala Ser Gly Ile Gly Pro Ala Cys Pro Gln Phe Leu Ala Asp Thr
65                  70                  75                  80

Ser Ser Asn Glu Phe Leu Pro Gln Val Ile Asp Lys Ile Val Asn Thr
                85                  90                  95

Gln Leu Phe Lys Thr Ile Leu Asn Val Lys Glu Asp Cys Leu Thr Ile
            100                 105                 110

Ser Val Thr Arg Pro Lys Gly Thr Lys Ala Gly Asp Lys Leu Pro Val
        115                 120                 125

Leu Phe Trp Ile Phe Gly Gly Gly Phe Glu Leu Gly Ser Ala Ser Met
    130                 135                 140

Tyr Asp Gly Ala Pro Leu Val Thr Asn Ala Ile Asn Met Gly Lys Pro
145                 150                 155                 160

Tyr Val Tyr Val Ala Val Asn Tyr Arg Val Gly Gly Phe Gly Phe Met
                165                 170                 175

Pro Gly Lys Glu Ile Leu Lys Asp Gly Ser Ser Asn Leu Gly His Leu
            180                 185                 190

Asp Gln Arg Met Gly Leu Gln Trp Val Ala Asp Asn Ile Ala Ala Phe
        195                 200                 205

Gly Gly Asp Pro Asp Lys Val Thr Ile Trp Gly Glu Ser Ala Gly Ala
    210                 215                 220

Met Ser Val Phe Asn Gln Met Ser Leu Tyr Asp Gly Asp Asn Thr Tyr
225                 230                 235                 240

Asn Gly Lys Pro Leu Phe Arg Gly Ala Ile Met Asn Ser Gly Ser Ile
                245                 250                 255

Val Pro Ala Gly Pro Val Asp Cys Pro Lys Gly Gln Lys Val Tyr Asp
            260                 265                 270

Thr Val Val Lys Asn Ala Gly Cys Ser Gly Ala Ala Asp Thr Leu Ala
        275                 280                 285

Cys Leu Arg Ala Leu Pro Tyr Glu Thr Phe Leu Lys Ala Ala Asn Ser
    290                 295                 300

Val Pro Gly Ile Leu Ser Tyr Asn Ser Val Ala Leu Ser Tyr Leu Pro
305                 310                 315                 320

Arg Pro Asp Gly Lys Ala Leu Thr Gln Ser Ala Asp Lys Leu Met Leu
                325                 330                 335

Ala Lys Lys Tyr Ala Ala Val Pro Met Ile Ile Gly Asp Gln Glu Asp
            340                 345                 350

Glu Gly Thr Leu Phe Ser Leu Phe Gln Ser Asn Ile Thr Thr Thr Ser
        355                 360                 365

Lys Leu Val Ser Tyr Leu Asn Asp Ile Phe Phe Asn Asp Ala Thr Glu
    370                 375                 380

Ser Gln Ile Lys Ser Leu Val Ser Thr Tyr Ser Thr Leu Ile Ser Ala
385                 390                 395                 400

Gly Ser Pro Phe Gly Thr Gly Leu Phe Asn Glu Ile Tyr Pro Gly Phe
                405                 410                 415

Lys Arg Leu Ala Ala Ile Leu Gly Asp Leu Ile Phe Thr Leu Ser Arg
            420                 425                 430

Arg Ile Phe Leu Asp Ala Ala Thr Thr Leu Asn Pro Ser Val Pro Ala
        435                 440                 445

Trp Ser Tyr Leu Ala Ser Tyr Asn Phe Gly Thr Pro Ile Leu Gly Thr
    450                 455                 460

Phe His Ala Ser Asp Ile Leu Gln Val Phe Tyr Gly Ile Leu Pro Asn
465                 470                 475                 480

Tyr Ala Ser Lys Ser Ile Gln Ser Tyr Tyr Ala Asn Phe Val Tyr Asn
                485                 490                 495

Leu Asp Pro Asn Asp Ala Ser Gly Gly Thr Ser Ser Lys Ser Lys Val
            500                 505                 510

Ser Gln Asp Trp Pro Gln Trp Gln Lys Glu Arg Lys Leu Val Gln Phe
        515                 520                 525

Phe Ser Asp Tyr Ala Gly Tyr Leu Ala Asp Asp Phe Arg Ser Asp Ser
    530                 535                 540

Tyr Asn Trp Ile Lys Ala Asn Leu Asp Ala Leu His Ile
545                 550                 555

<210>  18
<211>  557
<212>  PRT
<213>  Coniochaeta ligniaria

<400>  18

Ala Pro Thr Pro Asn Pro Val Ile Glu Asp Arg Ala Ala Lys Val Ser
1               5                   10                  15

Val Ala Ile Ala Pro Ser Ser Thr Val Val Gly Ala Ser Thr Leu Gly
            20                  25                  30

Val Glu Lys Phe Ser Gly Ile Pro Tyr Ala Val Pro Pro Val Gly Pro
        35                  40                  45

Leu Arg Leu Lys Pro Pro Val Arg Leu Thr Ala Ser Leu Gly Thr Phe
    50                  55                  60

Asp Ala Thr Gly Ile Glu Ala Ala Cys Pro Gln Phe Leu Leu Ser Thr
65                  70                  75                  80

Gly Gly Asn Ser Leu Ile Thr Gln Val Leu Gly Asn Val Leu Asp Leu
                85                  90                  95

Pro Phe Phe Gln Thr Val Thr Lys Gln Ser Glu Asp Cys Leu Thr Ile
            100                 105                 110

Asn Val Ile Arg Pro Ala Gly Val Lys Ala Gly Asp Asn Leu Pro Val
        115                 120                 125

Leu Phe Trp Ile Phe Gly Gly Gly Phe Glu Leu Gly Trp Thr Ser Met
    130                 135                 140

Tyr Asp Gly Ser Ser Leu Val Ser Asn Ala Val Asn Ala Gly Lys Pro
145                 150                 155                 160

Phe Val Phe Val Ala Val Asn Tyr Arg Val Gly Gly Phe Gly Phe Leu
                165                 170                 175

Pro Gly Lys Glu Ile Leu Gln Asp Gly Ser Ala Asn Leu Gly Leu Leu
            180                 185                 190

Asp Gln Arg Met Gly Leu Glu Trp Ala Ala Asp Asn Ile Ala Ala Phe
        195                 200                 205

Gly Gly Asp Pro Ser Lys Val Thr Ile Trp Gly Glu Ser Ala Gly Ser
    210                 215                 220

Ile Ser Val Leu Asp Gln Met Val Leu Tyr Gly Gly Asn Asn Gln Tyr
225                 230                 235                 240

Asn Gly Lys Pro Leu Phe Arg Gly Ala Ile Met Asn Ser Gly Ser Ile
                245                 250                 255

Val Pro Ala Asp Pro Val Asp Cys Pro Lys Gly Gln Val Val Tyr Asp
            260                 265                 270

Thr Val Val Arg Ala Ala Gly Cys Ser Ser Ser Thr Asp Thr Leu Ala
        275                 280                 285

Cys Leu Arg Ser Ala Asp Tyr Thr Thr Phe Leu Asn Ala Ala Asn Ser
    290                 295                 300

Val Pro Gly Ile Leu Ser Tyr Asn Ser Val Ala Leu Ser Tyr Leu Pro
305                 310                 315                 320

Arg Pro Asp Gly Thr Ala Ile Thr Asp Ser Pro Asp Val Leu Val Leu
                325                 330                 335

Ser Gly Lys Tyr Ala Ala Val Pro Met Ile Ile Gly Asp Gln Glu Asp
            340                 345                 350

Glu Gly Thr Leu Phe Ser Leu Phe Gln Ser Asn Thr Thr Thr Thr Ser
        355                 360                 365

Ala Leu Ile Asp Tyr Leu Asn Asn Ile Phe Phe His Asp Ala Thr Arg
    370                 375                 380

Glu Gln Ile Ala Ala Leu Val Ala Thr Tyr Asp Asp Ser Ile Thr Ala
385                 390                 395                 400

Gly Ser Pro Phe Arg Thr Gly Ile Phe Asn Glu Val Tyr Pro Gly Phe
                405                 410                 415

Lys Arg Leu Ala Ala Ile Leu Gly Asp Leu Val Phe Gln Leu Thr Arg
            420                 425                 430

Arg Ala Phe Leu Thr Gly His Val Ala Ile Asn Pro Ser Val Pro Ala
        435                 440                 445

Trp Ser Tyr Met Ala Ser Tyr Asp Tyr Gly Thr Pro Ile Leu Gly Ser
    450                 455                 460

Phe His Gly Ser Asp Leu Leu Gln Val Phe Tyr Gly Ile Leu Pro Asn
465                 470                 475                 480

Ala Ala Ser Lys Ser Ile Gln Ser Tyr Tyr Ala Asn Phe Val Tyr Asn
                485                 490                 495

Leu Asp Pro Asn Asp Ala Ser Gly Gly Thr Gly Thr Gly Thr Lys Val
            500                 505                 510

Ala Leu Gly Trp Pro Gln Trp Lys Asp Gly Asn Gln Leu Val Gln Phe

515                 520                 525

Phe Asn Ile Gly Ser Thr Leu Ile Lys Asp Asp Phe Arg Ser Gly Thr
    530                 535                 540

Phe Asn Trp Ile Val Gly Asn Ile Pro Tyr Leu Arg Ile
545                 550                 555

<210>  19
<211>  558
<212>  PRT
<213>  Cutaneotrichosporon oleaginosum

<400>  19

Ala Pro Ala Pro Val Val Asp Asp Ser His Leu Asp Glu Arg Ala Leu
1               5                   10                  15

Thr Ile Thr Val Ser Ala Pro Ala Gly Thr Ile Val Gly Lys Arg Gly
            20                  25                  30

Leu Leu Thr Asp Asp Phe Asn Gly Ile Pro Tyr Ala Ala Pro Pro Ile
        35                  40                  45

Ser Asn Leu Arg Leu Arg Pro Pro Gln Arg Ile Ser Ser Pro Leu Asn
    50                  55                  60

Asn Phe Asp Ala Thr Lys Ala Ala Ala Ala Cys Pro Gln Phe Leu Ala
65                  70                  75                  80

Asp Ser Asp Asp Ser Gly Leu Leu Ala Gln Val Met Ser Thr Val Ser
                85                  90                  95

Arg Thr Ala Leu Phe Gln Arg Ala Leu Lys Ile Ser Glu Asp Cys Leu
            100                 105                 110

Ser Val Asn Val Ile Arg Pro Arg Gly Thr Lys Ala Gly Asp Asn Leu
        115                 120                 125

Pro Val Leu Phe Trp Ile Tyr Gly Gly Gly Phe Glu Leu Gly Trp Ser
    130                 135                 140

Ser Met Tyr Asp Gly Gly Gly Leu Val Gln Asn Ala Ala Leu Asn Gly
145                 150                 155                 160

Lys Pro Tyr Val Phe Val Ala Val Asn Tyr Arg Val Gly Ala Trp Gly
                165                 170                 175

Phe Met Pro Gly Lys Glu Ile Leu Ala Asp Gly Ala Gly Asn Leu Gly

            180                 185                 190

Leu Leu Asp Gln Arg Met Gly Leu Glu Trp Val Ala Asp Asn Ile Ala
        195                 200                 205

Ala Phe Gly Gly Asp Pro Ser Arg Val Thr Ile Trp Gly Glu Ser Ala
    210                 215                 220

Gly Ala Ile Ser Val Trp Asn Gln Met Thr Ala Tyr Gly Gly Asn Ile
225                 230                 235                 240

Glu Tyr Lys Gly Lys Pro Leu Phe Arg Gly Ala Ile Met Asn Ser Gly
                245                 250                 255

Ser Ile Ile Pro Ala Asp Pro Val Asp Cys Pro Lys Gly Gln Ala Ile
            260                 265                 270

Tyr Asp Ala Val Val Glu Lys Ala Gly Cys Ser Gly Pro Asp Ser Leu
        275                 280                 285

Ala Cys Leu Arg Gln Leu Pro Leu Asp Gln Phe Thr Glu Ala Ala Asn
    290                 295                 300

Ser Val Pro Ala Ile Leu Ser Tyr Thr Ser Val Ala Leu Ser Tyr Leu
305                 310                 315                 320

Pro Arg Pro Asp Gly Ser Phe Met Pro Glu Ser Pro Gln Gln Leu Leu
                325                 330                 335

Leu Lys Gly Arg Tyr Ala Ala Val Pro Met Ile Ile Gly Asp Gln Glu
            340                 345                 350

Asp Glu Gly Thr Leu Phe Ser Leu Phe Gln Ala Asn Thr Thr Glu Thr
        355                 360                 365

Thr Pro Met Ile Val Arg Tyr Leu Lys Asp Leu Phe Phe Asn Gly Ala
    370                 375                 380

Ser Glu Ala Thr Leu Thr Ala Leu Val Glu Thr Tyr Glu Asp Val Pro
385                 390                 395                 400

Ala Glu Gly Ser Pro Phe Arg Thr Gly Pro Leu Asn Glu Val Tyr Lys
                405                 410                 415

Gly Phe Lys Arg Leu Ala Ala Ile Leu Gly Asp Leu Val Phe Thr Ile
            420                 425                 430

Thr Arg Arg Ile Phe Leu Asp Ile Ala Lys Thr Val Asn Pro Ser Val
        435                 440                 445

Pro Ala Trp Ser Tyr Ile Ala Ser Tyr Asn Tyr Gly Thr Pro Ile Leu
    450                 455                 460

Gly Thr Phe His Gly Ser Asp Leu Leu Gln Val Phe Phe Gly Ile Leu
465                 470                 475                 480

Pro Asn Lys Ala Ser Arg Asp Ile Gln Ala Tyr Tyr Ser Asn Phe Val
                485                 490                 495

Tyr Asn Leu Asp Pro Asn Asn Tyr Ser Gly Gly Thr Asn Arg Leu Ser
            500                 505                 510

Arg Val Ile Asp Asn Trp Pro Gln Trp Glu Arg Gly Thr Lys Lys Leu
        515                 520                 525

Ile Gln Phe Tyr Ala His His Phe Gln Pro Met Ile Asp Asp Phe Arg
    530                 535                 540

Thr Asp Ser Glu Gln Ile Leu Ser Ser Asn Phe Glu Ser Phe
545                 550                 555

<210>  20
<211>  556
<212>  PRT
<213>  Sporothrix schenckii

<400>  20

Ala Pro Pro Ser Thr Pro Ala Ile Met Glu Arg Ala Ala Gln Val Thr
1               5                   10                  15

Met Val Leu Pro Leu Gly Thr Val Ile Gly Thr Ser Ser Asn Gly Val
            20                  25                  30

Glu Ser Tyr Asn Ala Ile Pro Phe Ala Gln Pro Pro Thr Gly Asn Leu
        35                  40                  45

Arg Leu Lys Pro Pro Val Lys Arg Thr Ala Ser Tyr Gly Val Leu Asp
    50                  55                  60

Gly Ala Gly Pro Ala Ala Ala Cys Pro Gln Phe Val Glu Ser Thr Ser
65                  70                  75                  80

Gly Ser Leu Leu Ser Ser Val Ile Gly Thr Leu Ala Asn Ser Pro Phe
                85                  90                  95

Ile Gln Thr Thr Thr Asp Gln Thr Glu Asp Cys Leu Thr Ile Asp Val
        100                 105                 110

Tyr Arg Pro Ala Gly Thr Lys Ala Thr Asp Lys Leu Pro Val Leu Phe
    115                 120                 125

Trp Ile Phe Gly Gly Gly Phe Glu Leu Gly Trp Thr Ser Met Tyr Asp
    130                 135                 140

Gly Ala Gly Leu Val Ala Val Ser Ile Ser Leu Gly Lys Pro Met Val
145                 150                 155                 160

Phe Val Ala Val Asn Tyr Arg Val Gly Gly Phe Gly Phe Leu Pro Gly
                165                 170                 175

Ala Glu Val Leu Ala Asp Gly Ala Ser Asn Leu Gly Leu Leu Asp Gln
            180                 185                 190

Arg Met Gly Leu Glu Trp Thr Ala Asp Asn Ile Gly Tyr Phe Gly Gly
        195                 200                 205

Asp Ser Gly Lys Val Thr Ile Trp Gly Glu Ser Ala Gly Ala Ile Ser
    210                 215                 220

Val Ala Asp Gln Met Met Leu Tyr Asn Gly Asn Asn Thr Tyr Lys Gly
225                 230                 235                 240

Lys Ala Leu Phe Arg Gly Ala Ile Met Asp Ser Gly Ser Ile Val Pro
                245                 250                 255

Ala Asp Pro Val Asp Cys Pro Lys Gly Gln Ala Val Tyr Asn Gln Val
            260                 265                 270

Val Ala Lys Ala Gly Cys Ser Ser Ser Ser Asp Thr Leu Ala Cys Leu
        275                 280                 285

Arg Ala Ala Pro Tyr Asn Thr Phe Leu Asn Ala Val Asn Ser Val Pro
    290                 295                 300

Gly Ile Leu Ser Tyr Ser Ser Val Ala Leu Ser Tyr Leu Pro Arg Pro
305                 310                 315                 320

Asp Gly Val Val Leu Val Asp Ser Pro Asp Val Leu Ile Thr Asn Asn
                325                 330                 335

Lys Tyr Ala Ala Val Pro Met Ile Ile Gly Asp Gln Glu Asp Glu Gly
            340                 345                 350

Thr Leu Phe Ser Leu Phe Gln Ser Asn Ile Thr Asn Thr Asn Gln Leu
        355                 360                 365

Val Thr Tyr Leu Ser Asp Tyr Tyr Phe His Asn Ala Thr Thr Ala Gln
    370                 375                 380

Ile Gln Ala Leu Val Ala Thr Tyr Pro Asp Asn Ala Ser Ala Gly Ser
385                 390                 395                 400

Pro Phe Asn Thr Leu Leu Leu Asn Glu Val Tyr Pro Glu Phe Lys Arg
                405                 410                 415

Leu Ala Ala Ile Leu Gly Asp Leu Val Phe Thr Leu Thr Arg Arg Ala
            420                 425                 430

Phe Leu Ala Glu Thr Asn Asn Arg Asn Pro Ser Val Pro Ser Trp Ser
        435                 440                 445

Tyr Leu Ala Ser Tyr Asp Tyr Gly Thr Pro Val Leu Gly Thr Phe His
    450                 455                 460

Gly Ser Asp Leu Leu Gln Val Phe Tyr Gly Ile Leu Pro Asn Ala Ala
465                 470                 475                 480

Ser Lys Ser Ile His Ser Tyr Tyr Phe Asn Phe Leu Tyr Asn Gln Asp
                485                 490                 495

Pro Asn Asn Ala Ser Gly Gly Thr Ser Ser Thr Ala Ser Arg Val Ser
            500                 505                 510

Leu Thr Trp Pro Gln Trp Gly Ala Gly Asn Gln Leu Val Gln Phe Trp
        515                 520                 525

Ala Ala Thr Ser Asn Leu Leu Ala Asp Asn Phe Arg Asn Ser Ser Tyr
    530                 535                 540

Thr Trp Ile Thr Ser Asn Ile Ala Ser Leu His Phe
545                 550                 555

<210>  21
<211>  549
<212>  PRT
<213>  Stachybotrys chlorohalonata

<400>  21

Leu Pro Lys Pro Asp Ala Thr Leu Glu Glu Arg Ala Asn Ser Val Thr
1               5                   10                  15

Val Ala Leu Pro Ser Gly Thr Val Ile Gly Ala Ser Leu Leu Ser Val
            20                  25                  30

Glu Ser Phe Arg Gly Ile Pro Phe Ala Asp Pro Pro Val Gly Pro Leu
        35                  40                  45

Arg Leu Lys Pro Pro Gln Arg Leu Ser Glu Pro Leu Gly Thr Phe Asp
    50                  55                  60

Ala Thr Gly Ile Ala Pro Ala Cys Pro Gln Met Phe Ile Ser Thr Gly
65                  70                  75                  80

Thr Arg Asp Leu Ile Gly Gly Val Leu Gln Glu Leu Leu Asp Leu Pro
                85                  90                  95

Phe Leu Gln Pro Val Thr Gly Gln Glu Asp Cys Leu Ser Ile Thr Val
            100                 105                 110

Gln Arg Pro Ala Gly Thr Gln Ala Gly Asp Lys Leu Pro Val Leu Phe
        115                 120                 125

Trp Ile Phe Gly Gly Gly Phe Gln Leu Gly Ser Thr Leu Met Tyr Asp
    130                 135                 140

Gly Ala Ser Leu Leu Ala Thr Ala Val Ala Gln Gly Gln Pro Phe Val
145                 150                 155                 160

Phe Val Ala Val Asn Tyr Arg Val Ala Gly Phe Gly Phe Met Pro Gly
                165                 170                 175

Ala Glu Ile Leu Ala Asp Gly Ser Ala Asn Leu Gly Leu Leu Asp Gln
            180                 185                 190

Arg Met Gly Leu Glu Trp Val Ala Asp Asn Ile Ala Ala Phe Gly Gly
        195                 200                 205

Asp Pro Asp Lys Val Thr Ile Trp Gly Glu Ser Ala Gly Ser Ile Ser
    210                 215                 220

Val Leu Asp Gln Met Leu Leu Tyr Gly Gly Asp Ala Ser Tyr Asn Gly
225                 230                 235                 240

Arg Pro Leu Phe Arg Gly Ala Ile Met Asn Ser Gly Thr Val Val Pro
                245                 250                 255

Ala Asp Pro Val Asp Cys Pro Lys Gly Gln Glu Val Tyr Asp Thr Val
            260                 265                 270

Val Arg Asn Ser Gly Cys Glu Gly Ser Gly Asp Thr Leu Ala Cys Leu
        275                 280                 285

Arg Glu Leu Pro Tyr Asp Thr Phe Leu Asp Ala Ala Asn Ser Val Pro
    290                 295                 300

Ser Leu Leu Ser Tyr Ser Ser Val Ala Leu Ser Tyr Leu Pro Arg Pro
305                 310                 315                 320

Asp Gly Thr Val Leu Pro Asp Ser Pro Gln Thr Leu Val Ser Gln Gly
                325                 330                 335

Arg Tyr His Ala Val Pro Met Ile Val Gly Asp Gln Glu Asp Glu Gly
            340                 345                 350

Thr Leu Phe Ala Leu Phe Gln Pro Asn Leu Thr Thr Thr Ala Glu Met
        355                 360                 365

Ala Ala Tyr Leu Gly Asp Leu Phe Phe His Ala Pro Ser Val Gln Gln
    370                 375                 380

Leu Thr Thr Met Val Gln Thr Trp Pro Val Ser Leu Thr Ala Gly Ser
385                 390                 395                 400

Pro Phe Arg Thr Gly Leu Phe Asn Glu Trp Tyr Pro Gly Phe Lys Arg
                405                 410                 415

Leu Ala Ala Ile Leu Gly Asp Ile Val Phe Thr Leu Thr Arg Arg Val
            420                 425                 430

Phe Leu Glu Ser Ala Ala Ala Ala Asn Pro His Val Pro Val Trp Ser
        435                 440                 445

Tyr Leu Ser Ser Tyr Asn Tyr Gly Thr Pro Val Met Gly Thr Phe His
    450                 455                 460

Gly Ser Asp Leu Leu Gln Val Phe Tyr Gly Ile Leu Pro Asn Asn Ala
465                 470                 475                 480

Met Arg Ser Cys Arg Thr Tyr Tyr Phe Asn Phe Leu Tyr Asn Leu Asp
                485                 490                 495

Pro Asn Glu Gly Val Gly Gly Tyr Leu Arg Trp Pro Glu Trp Lys Glu
            500                 505                 510

Ala Lys Thr Leu Leu Trp Phe Gln Thr Ala Ala Lys Asn Ser Ile Leu

515                 520                 525

Arg Asp Asp Phe Arg Gln Glu Ser Phe Asn Trp Ile Ser Gln Asn Val
    530                 535                 540

Asn Leu Leu His Ile
545

<210>  22
<211>  554
<212>  PRT
<213>  Colletotrichum orchidophilum

<400>  22

Val Ser Asp His Val Gln Pro Leu Glu Asp Arg Ala Val Ala Asn Ala
1               5                   10                  15

Thr Val Val Leu Gln Thr Ala Thr Val Ile Gly Asn Val Met Asn Asn
            20                  25                  30

Val Glu Ser Phe Gly Gly Ile Pro Tyr Ala Lys Pro Pro Thr Gly Gln
        35                  40                  45

Leu Arg Leu Lys Pro Pro Val Arg Leu Thr Gly Asn Ile Gly Thr Phe
    50                  55                  60

Asp Ala Thr Gly Pro Ala Ala Ala Cys Pro Gln Met Leu Ser Ser Ser
65                  70                  75                  80

Asp Ser Glu Asn Ile Leu Phe Asn Leu Leu Gly Ser Ile Ala Asn Leu
                85                  90                  95

Pro Phe Val Gln Lys Ala Thr Gly Gln Thr Glu Asp Cys Leu Thr Ile
            100                 105                 110

Thr Val Ala Arg Pro Ala Gly Thr Lys Ala Asp Ala Lys Leu Pro Val
        115                 120                 125

Leu Tyr Trp Ile Phe Gly Gly Gly Phe Glu Leu Gly Trp Ser Ser Met
    130                 135                 140

Tyr Asp Gly Thr Gly Leu Val Gln His Gly Val Asp Ile Ser Lys Pro
145                 150                 155                 160

Phe Val Phe Val Ala Val Asn Tyr Arg Val Ala Gly Phe Gly Phe Met
                165                 170                 175

Pro Gly Lys Glu Ile Leu Ala Asp Gly Ser Ala Asn Leu Gly Leu Leu

            180                 185                 190

Asp Gln Arg Met Gly Leu Glu Trp Val Ala Asp Asn Ile Ala Ala Phe
        195                 200                 205

Gly Gly Asp Pro Asp Lys Val Thr Ile Trp Gly Glu Ser Ala Gly Ala
    210                 215                 220

Ile Ser Val Phe Asp Gln Met Ala Leu Tyr Asn Gly Asp Asn Lys Tyr
225                 230                 235                 240

Asn Gly Lys Ala Leu Phe Arg Gly Ala Ile Met Asn Ser Gly Ser Ile
                245                 250                 255

Ala Pro Thr Asp Pro Val Asp Cys Pro Lys Gly Gln Ala Val Tyr Asp
            260                 265                 270

Lys Val Val Ala Glu Ala Gly Cys Ala Gly Lys Ala Asp Thr Leu Asn
        275                 280                 285

Cys Leu Arg Glu Ala Asp Tyr Asn Thr Phe Leu Asn Ala Val Thr Ser
    290                 295                 300

Val Pro Gly Ile Leu Ser Tyr Asn Ser Leu Ala Leu Ser Tyr Leu Pro
305                 310                 315                 320

Arg Pro Asp Gly Lys Thr Leu Thr Ala Ser Pro Asp Val Leu Ala Lys
                325                 330                 335

Asn Gly Gln Tyr Ala Ala Val Pro Met Ile Ile Gly Asp Gln Glu Asp
            340                 345                 350

Glu Gly Thr Leu Phe Gly Ile Phe Gln Pro Asn Leu Thr Thr Thr Ala
        355                 360                 365

Lys Leu Val Thr Tyr Leu Lys Asp Tyr Tyr Phe Ala Thr Ala Thr Glu
    370                 375                 380

Ala Gln Ile Glu Ala Tyr Val Ala Thr Tyr Asp Asp Gly Val Thr Ala
385                 390                 395                 400

Val Ile Asn Gly Ser Pro His Arg Thr Gly Leu Leu Asn Glu Ile Phe
                405                 410                 415

Pro Gly Phe Lys Arg Arg Ser Ala Ile Leu Gly Asp Leu Val Phe Thr
            420                 425                 430

Leu Thr Arg Arg Val Phe Leu Asn Leu Ala Thr Ala Val Gln Pro Gly
        435                 440                 445

Val Pro Ser Trp Ser Tyr Leu Ser Ser Tyr Asp Tyr Gly Thr Pro Ile
    450                 455                 460

Leu Gly Thr Phe His Gly Ser Asp Leu Leu Gln Val Phe Tyr Gly Ile
465                 470                 475                 480

Lys Asp Asn Tyr Ala Ala Arg Ser Ile Arg Thr Tyr Tyr Thr Asn Phe
                485                 490                 495

Val Tyr Ala Ser Asp Pro Asn Val Gly Leu Asn Gly Ala Tyr Pro Ala
            500                 505                 510

Trp Pro Gln Trp Ser Gln Gly Gln Thr Leu Leu Gln Phe Phe Ala Asp
        515                 520                 525

Lys Ala Ser Thr Leu Lys Asp Asp Phe Arg Lys Thr Ser Ser Asp Trp
    530                 535                 540

Ile Leu Asn Asn Ala Gly Ile Leu Tyr Ile
545                 550

<210>  23
<211>  554
<212>  PRT
<213>  Colletotrichum incanum

<400>  23

Val Pro Asp Asp Phe Arg Pro Leu Glu Asp Arg Ala Ala Ser Thr Ala
1               5                   10                  15

Thr Val Val Leu Asp Thr Ala Thr Val Val Gly Asn Val Met Asn Lys
            20                  25                  30

Val Glu Ser Phe Gly Gly Ile Pro Tyr Ala Lys Pro Pro Thr Gly Gln
        35                  40                  45

Leu Arg Leu Lys Pro Pro Val Arg Leu Thr Glu Asn Ile Gly Thr Phe
    50                  55                  60

Asp Ala Thr Gly Pro Ala Ala Ala Cys Pro Gln Met Ile Ser Ser Ser
65                  70                  75                  80

Glu Ser Glu Asn Ile Leu Phe Asp Leu Leu Gly Asp Ile Ala Asn Leu
                85                  90                  95

Pro Phe Val Gln Lys Ala Thr Gly Gln Thr Glu Asp Cys Leu Ser Ile
            100                 105                 110

Thr Val Ala Arg Pro Gln Gly Thr Lys Ala Asp Ala Lys Leu Pro Val
        115                 120                 125

Leu Tyr Trp Ile Phe Gly Gly Gly Phe Gln Leu Gly Trp Ser Ser Met
    130                 135                 140

Tyr Asp Gly Thr Ser Leu Val Gln His Gly Val Asp Ile Gly Lys Pro
145                 150                 155                 160

Phe Ile Phe Val Ala Val Asn Tyr Arg Val Ala Gly Phe Gly Phe Met
                165                 170                 175

Pro Gly Lys Glu Ile Leu Ala Asp Gly Ser Ala Asn Leu Gly Leu Leu
            180                 185                 190

Asp Gln Arg Met Gly Leu Glu Trp Val Ala Asp Asn Ile Ala Ala Phe
        195                 200                 205

Gly Gly Asp Pro Asp Lys Val Thr Ile Trp Gly Glu Ser Ala Gly Ala
    210                 215                 220

Ile Ser Val Phe Asp Gln Met Ala Leu Tyr Asn Gly Asp Asn Lys Tyr
225                 230                 235                 240

Lys Gly Lys Ala Leu Phe Arg Gly Ala Ile Met Asn Ser Gly Ser Ile
                245                 250                 255

Thr Pro Thr Asp Pro Val Asp Cys Pro Lys Gly Gln Ala Val Tyr Asp
            260                 265                 270

Lys Val Val Ser Arg Ala Gly Cys Ser Gly Lys Ala Asp Thr Leu Asn
        275                 280                 285

Cys Leu Arg Glu Val Asp Phe Thr Thr Phe Leu Asn Ala Val Thr Ser
    290                 295                 300

Val Pro Gly Ile Leu Ser Tyr Ser Ser Ile Ala Leu Ser Tyr Leu Pro
305                 310                 315                 320

Arg Pro Asp Gly Lys Thr Leu Thr Ala Ser Pro Asp Val Leu Ala Lys
                325                 330                 335

Asn Gly Gln Tyr Ala Ala Val Pro Met Ile Ile Gly Asp Gln Glu Asp
            340                 345                 350

Glu Gly Thr Leu Phe Gly Val Phe Gln Pro Asn Leu Thr Thr Thr Asp
        355                 360                 365

Lys Leu Val Thr Tyr Leu Lys Asp Tyr Tyr Phe Ala Thr Ala Thr Glu
    370                 375                 380

Ala Gln Ile Arg Ala Tyr Val Ala Thr Tyr Asp Asp Gly Leu Asn Ala
385                 390                 395                 400

Val Ile Asn Gly Ser Pro His Arg Thr Gly Leu Leu Asn Glu Ile Phe
                405                 410                 415

Pro Gly Phe Lys Arg Arg Ser Ala Val Leu Gly Asp Leu Val Phe Thr
            420                 425                 430

Leu Thr Arg Arg Val Phe Leu Asn Leu Ala Thr Ala Val Asn Pro Ser
        435                 440                 445

Val Pro Ser Trp Ser Tyr Leu Ala Ser Tyr Asn Tyr Gly Thr Pro Ile
    450                 455                 460

Leu Gly Thr Phe His Gly Ser Asp Leu Leu Gln Val Phe Tyr Gly Val
465                 470                 475                 480

Lys Asp Asn Tyr Ala Ala Arg Ser Ile Arg Thr Tyr Tyr Thr Asn Phe
                485                 490                 495

Val Tyr Ala Leu Asp Pro Asn Val Gly Ser Asn Gly Gln Tyr Pro Asn
            500                 505                 510

Trp Pro Lys Trp Ser Gln Gly Gln Asn Leu Ile Gln Phe Phe Ala Asp
        515                 520                 525

Lys Ala Ser Thr Leu Lys Asp Asp Phe Arg Lys Thr Ser Ser Asp Trp
    530                 535                 540

Ile Leu Asn Asn Ala Gly Ser Leu Tyr Phe
545                 550

<210>  24
<211>  554
<212>  PRT
<213>  Colletotrichum tofieldiae

<400>  24

Val Pro Asp Asp Phe Gln Pro Leu Glu Asp Arg Ala Ala Ser Thr Ala
1               5                   10                  15

Thr Val Val Leu Asp Thr Ala Thr Val Val Gly Asn Val Met Asn Lys
        20                  25                  30

Val Glu Ser Phe Gly Gly Ile Pro Tyr Ala Lys Pro Pro Thr Gly Gln
        35                  40                  45

Leu Arg Leu Lys Pro Pro Val Arg Leu Thr Glu Asn Ile Gly Thr Phe
    50                  55                  60

Asp Ala Thr Gly Pro Ala Ala Ala Cys Pro Gln Met Ile Ser Ser Ser
65                  70                  75                  80

Asp Ser Glu Asn Ile Leu Phe Asp Leu Leu Gly Asp Ile Ala Asn Leu
                85                  90                  95

Pro Phe Val Gln Lys Ala Thr Gly Gln Thr Glu Asp Cys Leu Ser Ile
            100                 105                 110

Thr Val Ala Arg Pro Gln Gly Thr Lys Ala Asp Ala Lys Leu Pro Val
        115                 120                 125

Leu Tyr Trp Ile Phe Gly Gly Gly Phe Gln Leu Gly Trp Ser Ser Met
    130                 135                 140

Tyr Asp Gly Thr Ser Leu Val Gln His Gly Val Asp Ile Ser Lys Pro
145                 150                 155                 160

Phe Val Phe Val Ala Val Asn Tyr Arg Val Ala Gly Phe Gly Phe Met
                165                 170                 175

Pro Gly Lys Glu Ile Leu Ala Asp Gly Ser Ala Asn Leu Gly Leu Leu
            180                 185                 190

Asp Gln Arg Met Gly Leu Glu Trp Val Ala Asp Asn Ile Ala Ala Phe
        195                 200                 205

Gly Gly Asp Pro Asp Lys Val Thr Ile Trp Gly Glu Ser Ala Gly Ala
    210                 215                 220

Ile Ser Val Phe Asp Gln Met Ala Leu Tyr Asn Gly Asn Asn Lys Tyr
225                 230                 235                 240

Lys Gly Lys Ala Leu Phe Arg Gly Ala Ile Met Asn Ser Gly Ser Ile
                245                 250                 255

Thr Pro Thr Asp Pro Val Asp Cys Pro Lys Gly Gln Ala Val Tyr Asp
            260                 265                 270

Lys Val Val Ser Arg Ala Gly Cys Ser Gly Lys Ala Asp Thr Leu Asn
        275                 280                 285

Cys Leu Arg Glu Val Asp Tyr Thr Thr Phe Leu Asn Ala Val Thr Ser
    290                 295                 300

Val Pro Gly Ile Leu Ser Tyr Ser Ser Ile Ala Leu Ser Tyr Leu Pro
305                 310                 315                 320

Arg Pro Asp Gly Lys Thr Leu Thr Ala Ser Pro Asp Val Leu Ala Lys
                325                 330                 335

Asn Gly Gln Tyr Ala Ala Val Pro Met Ile Ile Gly Asp Gln Glu Asp
            340                 345                 350

Glu Gly Thr Leu Phe Gly Val Phe Gln Pro Asn Leu Thr Thr Thr Asp
        355                 360                 365

Lys Leu Val Ser Tyr Leu Lys Asp Tyr Tyr Phe Ala Thr Ala Thr Glu
    370                 375                 380

Ala Gln Ile Arg Ala Tyr Val Ala Thr Tyr Asp Asp Gly Leu Asn Ala
385                 390                 395                 400

Val Ile Asn Gly Ser Pro His Arg Thr Gly Leu Leu Asn Glu Ile Phe
                405                 410                 415

Pro Gly Phe Lys Arg Arg Ser Ala Val Leu Gly Asp Leu Val Phe Thr
            420                 425                 430

Leu Thr Arg Arg Val Phe Leu Asn Leu Ala Thr Ala Val Asn Pro Ser
        435                 440                 445

Val Pro Ser Trp Ser Tyr Leu Ala Ser Tyr Asn Tyr Gly Thr Pro Ile
    450                 455                 460

Leu Gly Thr Phe His Gly Ser Asp Leu Leu Gln Val Phe Tyr Gly Val
465                 470                 475                 480

Lys Asp Asn Tyr Ala Ala Arg Ser Ile Arg Thr Tyr Tyr Thr Asn Phe
                485                 490                 495

Leu Tyr Ala Leu Asp Pro Asn Val Gly Ser Asn Gly Gln Tyr Pro Asn
            500                 505                 510

Trp Pro Lys Trp Ser Gln Gly Gln Asn Leu Ile Gln Phe Phe Ala Asp

515                 520                 525

Lys Ala Ser Thr Leu Lys Asp Asp Phe Arg Lys Thr Ser Ser Asp Trp
    530                 535                 540

Ile Leu Asn Asn Ala Gly Ser Leu Tyr Phe
545                 550

<210>  25
<211>  550
<212>  PRT
<213>  Hypoxylon sp. EC38

<400>  25

Leu Pro Ala Pro Thr Val Ile Leu Glu Lys Arg Ala Thr Thr Thr Ser
1               5                   10                  15

Val Val Ile Pro Gln Gly Thr Ile Ile Gly Ser Val Ser Asn Gly Val
            20                  25                  30

Glu Asn Tyr Gly Gly Ile Pro Phe Ala Gln Ala Pro Glu Gly Gly Leu
        35                  40                  45

Arg Leu Lys Pro Pro Gln Arg Leu Asn Thr Ser Leu Gly Thr Phe Asp
    50                  55                  60

Ala Thr Gly Ala Gly Pro Ser Cys Pro Gln Met Phe Phe Ser Thr Gly
65                  70                  75                  80

Gly Asn Asp Leu Leu Ile Ser Val Leu Gly Asn Leu Ile Asn Thr Pro
                85                  90                  95

Leu Phe Gln Thr Val Thr Asp Gln Lys Glu Asp Cys Leu Thr Met Arg
            100                 105                 110

Val Gln Arg Pro Ala Gly Thr Lys Ser Asp Ala Lys Leu Pro Val Leu
        115                 120                 125

Phe Trp Ile Phe Gly Gly Gly Phe Glu Leu Gly Ser Pro Gln Met Tyr
    130                 135                 140

Asp Ala Ser Ser Leu Ile Ser Asn Gly Val Asp Gln Gly Lys Pro Phe
145                 150                 155                 160

Ile Phe Val Ser Val Asn Tyr Arg Thr Gly Gly Phe Gly Phe Leu Gly
                165                 170                 175

Gly Lys Glu Val Leu Ala Asp Gly Ala Ser Asn Leu Gly Leu Leu Asp

180                 185                 190

Gln Arg Met Gly Leu Glu Trp Val Gln Asp Asn Ile Ala Ala Phe Gly
        195                 200                 205

Gly Asp Pro Glu Lys Val Thr Ile Trp Gly Glu Ser Ala Gly Ala Ile
    210                 215                 220

Ser Val Phe Asp Gln Met Ala Leu Tyr Gly Gly Asp Asn Thr Tyr Asn
225                 230                 235                 240

Gly Lys Lys Leu Phe Arg Ala Ala Ile Met Asn Ser Gly Ser Val Val
                245                 250                 255

Pro Ala Asn Pro Val Asp Gly Ile Gln Ala Gln Asn Ile Tyr Asn Thr
            260                 265                 270

Val Val Gln Glu Ala Gly Cys Ala Ser Ala Ala Asp Thr Leu Ala Cys
        275                 280                 285

Leu Arg Ser Ala Asp Tyr Asp Thr Phe Leu His Ala Ala Asn Ser Val
    290                 295                 300

Pro Gly Ile Leu Ser Tyr Thr Ser Val Ala Leu Ser Tyr Leu Pro Arg
305                 310                 315                 320

Pro Asp Gly Lys Val Leu Pro Asp Ser Pro Asp Val Leu Ala Arg Asn
                325                 330                 335

Gly Gln Tyr Ala Ala Val Pro Met Ile Ile Gly Asp Gln Glu Asp Glu
            340                 345                 350

Gly Thr Leu Phe Ser Leu Phe Gln Ser Asn Leu Thr Asn Thr Asp Glu
        355                 360                 365

Leu Val Asp Tyr Ile Trp Lys Val Phe Phe Pro Ala Val Asp Arg Asp
    370                 375                 380

Asp Val Val Gly Leu Val Asn Ser Tyr Asp Pro Ser Ile Thr Ala Gly
385                 390                 395                 400

Ser Pro Phe Asn Thr Gly Ile Leu Asn Asn Ile Tyr Pro Glu Phe Lys
                405                 410                 415

Arg Leu Ala Ala Ile Leu Gly Asp Met Val Phe Thr Leu Thr Arg Arg
            420                 425                 430

Ile Phe Leu Gln Thr Thr Leu Ser Ala Asn Pro Asn Val Pro Ser Trp
        435                 440                 445

Ser Tyr Leu Ala Ser Tyr Asp Tyr Gly Thr Pro Ile Leu Gly Thr Phe
    450                 455                 460

His Ala Ser Asp Ile Leu Gln Val Phe Tyr Gly Ile Leu Pro Asn Tyr
465                 470                 475                 480

Ala Ser Lys Ala Ile Gln Ser Tyr Tyr Ile Ser Phe Val Tyr Thr Met
                485                 490                 495

Asp Pro Asn Asp Gly Thr Ser Ser Asp Phe Pro Gln Trp Pro Gln Trp
            500                 505                 510

Lys Asp Asn Arg Asn Leu Met Gln Phe Trp Asn Asn Lys Ser Asn Leu
        515                 520                 525

Leu Ala Asp Asp Phe Arg Lys Thr Ala Glu Glu Tyr Ile Ser Ala His
    530                 535                 540

Ala Asp Ile Phe Leu Ile
545                 550

<210>  26
<211>  546
<212>  PRT
<213>  Aspergillus glaucus

<400>  26

Ala Pro Ala Ile Leu Gly Ala Pro Thr Val Thr Ile Pro Ser Pro Gln
1               5                   10                  15

Ala Thr Val Ala Gly Gln Ala Gly Leu Lys Val Glu Ser Phe Asn Gly
            20                  25                  30

Ile Pro Phe Ala Gln Pro Pro Thr Gly Ser Leu Arg Leu Lys Pro Pro
        35                  40                  45

Gln Pro Ile Glu Ser Ala Leu Gly Asp Val Asp Ala Thr Gly Ile Ala
    50                  55                  60

Gln Ser Cys Pro Gln Phe Phe Phe Ala Thr Asp Thr Ser Glu Phe Pro
65                  70                  75                  80

Gly Ser Val Val Gly Gln Leu Ala Asn Ile Pro Leu Phe Gln Lys Ile
                85                  90                  95

Thr Asn Ala Gly Glu Asp Cys Leu Thr Leu Asn Ile Arg Arg Pro Ala
            100                 105                 110

Gly Thr Thr Ala Glu Asp Lys Leu Pro Val Leu Val Trp Ile Phe Gly
        115                 120                 125

Gly Gly Phe Glu Leu Gly Ser Ser Thr Thr Tyr Asp Gly Ala Ser Leu
    130                 135                 140

Val Gln Asn Ser Ile Asp Leu Asp Met Pro Ile Val Phe Val Ala Met
145                 150                 155                 160

Asn Tyr Arg Val Gly Gly Phe Gly Phe Met Pro Gly Ala Glu Ile Leu
                165                 170                 175

Ala Asp Gly Ala Ala Asn Leu Gly Leu Leu Asp Gln Arg Leu Ala Leu
            180                 185                 190

Glu Trp Val Ala Asp Asn Ile Ala Ala Phe Gly Gly Asp Pro Asp Lys
        195                 200                 205

Val Thr Ile Trp Gly Glu Ser Ala Gly Ser Ile Ser Val Phe Asp Gln
    210                 215                 220

Met Met Leu Tyr Asp Gly Asp Ile Asp Tyr Lys Gly Ser Pro Leu Phe
225                 230                 235                 240

Arg Gly Ala Ile Met Asn Ser Gly Ser Val Val Pro Ala Asp Pro Val
                245                 250                 255

Asp Cys Asp Lys Gly Gln Asn Ile Tyr Asp Thr Val Val Ala Tyr Ala
            260                 265                 270

Gly Cys Asp Thr Ala Glu Asp Thr Leu Glu Cys Leu Arg Gly Leu Asp
        275                 280                 285

Tyr Thr Asp Phe Leu Asn Ala Ala Asn Ser Val Pro Gly Ile Leu Ser
    290                 295                 300

Tyr Asn Ser Val Ser Leu Ser Tyr Leu Pro Arg Pro Asp Gly Lys Val
305                 310                 315                 320

Phe Thr Gln Ser Pro Asp Phe Ala Val Gln Ser Gly Lys Tyr Ala Ser
                325                 330                 335

Val Pro Phe Ile Val Gly Asp Gln Glu Asp Glu Gly Thr Leu Phe Ala
            340                 345                 350

Leu Phe Gln Ser Asn Leu Thr Thr Thr Asp Asp Leu Ile Glu Tyr Phe
        355                 360                 365

Ser Asp Ile Phe Phe Phe His Ile Ser Arg Asp Gln Leu Gln Ala Leu
    370                 375                 380

Ile Asp Thr Tyr Glu Asp Thr Ala Glu Asn Gly Ser Pro Phe Arg Thr
385                 390                 395                 400

Gly Ile Phe Asn Asn Trp Tyr Pro Gln Phe Lys Arg Leu Gly Ala Ile
                405                 410                 415

Leu Gly Asp Leu Thr Phe Thr Leu Thr Arg Arg Leu Phe Leu Lys Tyr
            420                 425                 430

Ala Lys Glu Leu Lys Pro Asp Val Pro Ala Trp Ser Tyr Leu Ala Ser
        435                 440                 445

Tyr Asp Tyr Gly Thr Pro Ile Leu Gly Thr Phe His Gly Ser Asp Ile
    450                 455                 460

Leu Gln Val Phe Tyr Gly Ile Leu Pro Asn Tyr Ala Ser Arg Ser Ile
465                 470                 475                 480

His Ser Tyr Tyr Leu Ser Phe Val Tyr Glu Leu Asp Pro Asn Lys Arg
                485                 490                 495

Arg Gly Glu Glu Phe Pro Glu Trp Pro Gln Trp Thr Asn Glu Glu Val
            500                 505                 510

Glu Leu Met Gln Phe Phe Lys Asp Lys Gly Glu Leu Leu Lys Asp Asp
        515                 520                 525

Phe Arg Asn Glu Thr Tyr Asp Phe Leu Leu Glu Asn Thr Gln Ala Phe
    530                 535                 540

Arg Ile
545

<210> 27
<211> 549
<212> PRT
<213> Eutypa lata

<400> 27

Ala Pro Ala Pro Thr His Val Leu Glu Asp Arg Val Val Lys Thr Thr
1               5                   10                  15

Val Thr Thr Pro Gln Gly Thr Ile Val Gly Ser Ser Val Leu Gly Val
            20                  25                  30

Glu Ser Phe Ala Gly Ile Pro Phe Ala Glu Ala Pro Val Gly Ser Leu
        35                  40                  45

Arg Leu Lys Pro Pro Gln Arg Leu Asn Ser Ser Leu Gly Asp Phe Asp
    50                  55                  60

Ala Thr Gly Leu Gly Pro Ser Cys Pro Gln Met Phe Phe Ser Thr Glu
65                  70                  75                  80

Gly Asp Leu Leu Ser Ser Val Leu Gly Asn Leu Ile Asn Thr Pro Leu
                85                  90                  95

Phe Gln Thr Val Thr Gly Gln Thr Glu Asp Cys Leu Thr Met Arg Val
            100                 105                 110

Gln Arg Pro Ala Gly Thr Lys Ala Asp Ala Lys Leu Pro Val Leu Phe
        115                 120                 125

Trp Ile Phe Gly Gly Ala Phe Glu Leu Gly Ser Ala Gln Met Tyr Asp
    130                 135                 140

Gly Ser Ser Leu Ile Leu Asn Gly Val Ala Gln Asp Lys Pro Phe Ile
145                 150                 155                 160

Phe Ala Ala Val Asn Tyr Arg Ile Gly Gly Phe Gly Phe Leu Pro Gly
                165                 170                 175

Lys Glu Val Leu Ala Asp Gly Ala Ala Asn Leu Gly Leu Leu Asp Gln
            180                 185                 190

Arg Met Gly Leu Glu Trp Val Ala Asp Asn Ile Ala Ala Phe Gly Gly
        195                 200                 205

Asp Pro Glu Lys Val Thr Met Trp Gly Glu Ser Ala Gly Ala Ile Ser
    210                 215                 220

Val Phe Asp Gln Met Ala Leu Tyr Gly Gly Asp Asn Lys Tyr Asn Glu
225                 230                 235                 240

Lys Ser Leu Phe Arg Ala Ala Ile Met Asn Ser Gly Ser Val Val Pro
                245                 250                 255

Ala Asp Pro Val Asp Cys Ala Lys Cys Gln Val Val Tyr Asp Thr Val
            260                 265                 270

Val Glu Glu Ala Asn Cys Ala Ser Ala Ser Asp Thr Leu Glu Cys Leu
        275                 280                 285

Arg Glu Ala Asp Tyr Asp Thr Phe Leu Asn Ala Ala Asn Ser Val Pro
    290                 295                 300

Gly Val Leu Ser Tyr Ser Ser Val Ala Leu Ser Tyr Leu Pro Arg Pro
305                 310                 315                 320

Asp Gly Phe Ala Leu Pro Asn Ser Pro Asp Leu Leu Ala Gln Ala Gly
                325                 330                 335

Gln Tyr Ala Ala Val Pro Met Ile Ile Gly Asp Val Glu Asp Glu Gly
            340                 345                 350

Thr Leu Phe Ser Leu Phe Gln Pro Asn Ile Thr Thr His Asp Asp Ile
        355                 360                 365

Val Asp Tyr Leu Ser Glu Leu Leu Phe His Gly Ala Thr Arg Glu Gln
    370                 375                 380

Leu Asp Ala Leu Val Ser Thr Tyr Asp Pro Ser Ile Ala Ala Gly Ser
385                 390                 395                 400

Pro Phe Arg Thr Ser Phe Leu Asn Asn Val Tyr Pro Lys Phe Lys Thr
                405                 410                 415

Leu Ala Ala Ile Leu Gly Asp Leu Val Phe Thr Leu Thr Arg Arg Ile
            420                 425                 430

Phe Leu Arg Thr Thr Leu Ala Ala Asn Pro Asp Val Pro Ala Trp Ser
        435                 440                 445

Tyr Leu Ala Ser Tyr Asp Tyr Gly Thr Pro Ile Leu Gly Thr Phe His
    450                 455                 460

Gly Ser Asp Leu Leu Gln Val Phe Tyr Gly Ile Leu Pro Asn Tyr Ala
465                 470                 475                 480

Ser Ala Ala Phe Gln Ser Tyr Tyr Ile Ser Phe Leu Tyr Thr Met Asp
                485                 490                 495

Pro Asn Glu Gly Thr Ser Asp Lys Phe Leu Glu Trp Pro Gln Trp Ser
            500                 505                 510

Glu Asn Glu Asp Leu Met Trp Ile His Asn Asp Asp Gly Glu Leu Leu

515                 520                 525

Ala Asp Asp Phe Arg Ala Glu Ser Cys Thr Phe Ile Thr Asp Asn Val
    530                 535                 540

Asp Ala Leu Gln Ile
545

<210>  28
<211>  558
<212>  PRT
<213>  Fusarium oxysporum

<400>  28

Gln Asp Phe Pro Ala Pro Ala Gln Thr Ile Gln Glu Arg Ala Asp Lys
1               5                   10                  15

Thr Ala Thr Val Thr Ile Ser Ser Pro Asp Ala Thr Ile Leu Gly Lys
            20                  25                  30

Val Ala Asn Gly Val Glu Ser Phe Asn Gly Ile Pro Phe Ala Glu Ala
        35                  40                  45

Pro Thr Gly Gln Leu Arg Leu Arg Pro Pro Lys Arg Val Glu Asn Ser
    50                  55                  60

Leu Gly Glu Phe Asp Gly Thr Arg Leu Ala Arg Ala Cys Pro Gln Met
65                  70                  75                  80

Leu Leu Ser Ser Glu Asn Lys Asn Leu Leu Phe Glu Ile Ala Gly Asp
                85                  90                  95

Leu Ser Asn His Pro Phe Phe Gln Thr Val Thr Gly Gln Ser Glu Asp
            100                 105                 110

Cys Leu Ser Ile Thr Ile Thr Arg Pro Glu Gly Thr Ser Gln Asp Ala
        115                 120                 125

Lys Leu Pro Val Leu Phe Trp Ile Tyr Gly Gly Gly Phe Gln Phe Gly
    130                 135                 140

Trp Ser Ser Met Tyr Asp Gly Thr Gly Leu Ile Lys His Gly Leu Asp
145                 150                 155                 160

Met Arg Lys Pro Phe Ile Phe Val Ala Ile Asn Tyr Arg Thr Ser Gly
                165                 170                 175

Phe Gly Phe Met Pro Gly Lys Glu Val Leu Ala Asp Gly Ala Ser Asn

180                 185                 190

Leu Gly Leu Leu Asp Gln Arg Met Ala Leu Glu Trp Val Ala Asp Asn
        195                 200                 205

Ile Glu Ala Phe Gly Gly Asp Pro Ser Leu Val Thr Leu Trp Gly Glu
    210                 215                 220

Ser Ala Gly Ala Ile Ser Ile Leu Asn Gln Met Ala Leu Tyr Asp Gly
225                 230                 235                 240

Asp Asn Thr Tyr Asn Gly Lys Pro Leu Phe Arg Gly Ala Ile Met Asn
                245                 250                 255

Ser Gly Ser Ile Val Pro Ala Asp Pro Val Asp Cys Pro Lys Gly Gln
            260                 265                 270

Lys Val Tyr Asp Lys Val Val Lys Glu Ala Gly Cys Ser Gly Glu Glu
        275                 280                 285

Asp Thr Leu Asn Cys Leu Arg Glu Val Asp Tyr Ser Thr Phe Leu Lys
    290                 295                 300

Ala Val Thr Ser Val Pro Ala Phe Leu Ser Tyr Asn Ser Val Ala Leu
305                 310                 315                 320

Ser Tyr Leu Pro Arg Pro Asp Gly Lys Val Leu Thr Glu Ser Pro Glu
                325                 330                 335

Ile Leu Val Gly Ser Lys Lys Tyr Ala Ala Val Pro Met Ile Ile Gly
            340                 345                 350

Asp Gln Glu Asp Glu Gly Thr Leu Leu Ala Ile Phe Gln Pro Asn Leu
        355                 360                 365

Thr Thr Thr Glu Asp Leu Val Arg Tyr Leu Lys Asp Leu His Phe His
    370                 375                 380

Gly Ala Ser Glu Ala Gln Leu Thr Glu Leu Val Lys Thr Tyr Asp Glu
385                 390                 395                 400

Gly Ile Ser Ala Val Val Asn Gly Ser Pro Phe Arg Thr Gly Pro Leu
                405                 410                 415

Asn Glu Val Phe Pro Gly Phe Lys Arg Arg Ala Ala Leu Leu Gly Asp
            420                 425                 430

Ile Ala Phe Thr Leu Ser Arg Arg Ala Phe Leu Thr Leu Ala Asn Leu
        435                 440                 445

Ala Asn Pro Thr Thr Pro Ser Trp Ser Tyr Leu Ala Thr Tyr Asn Phe
    450                 455                 460

Gly Thr Pro Ile Phe Gly Thr Phe His Ala Ser Asp Ile Ile Gln Val
465                 470                 475                 480

Phe Phe Gly Ile Leu Pro Asn Tyr Ala Ser Arg Ser Ile Arg Thr Tyr
                485                 490                 495

Tyr Ile Asn Phe Leu Tyr Asp Leu Asp Pro Asn Ser Gly Ile Asn Gly
            500                 505                 510

Lys Tyr Pro Asn Trp Pro Gln Trp Asp Gln Gly Gln Lys Leu Met Asn
        515                 520                 525

Phe Glu Ala Asn Arg Ala Ser Leu Ile Asn Asp Asp Phe Arg Lys Asp
    530                 535                 540

Ser Tyr Glu Phe Leu Leu Gln Asn Ala Gln Ser Leu Tyr Val
545                 550                 555

<210>  29
<211>  554
<212>  PRT
<213>  Fusarium avenaceum

<400>  29

Ser Pro Asn Pro Asp Pro Ser Ile Asp Gln Arg Ala Ala Gly Thr Ala
1               5                   10                  15

Thr Val Val Leu Pro Ser Ala Thr Val Leu Gly Asn Val Met Asn Lys
            20                  25                  30

Val Glu Ser Phe Gly Gly Ile Pro Tyr Ala Glu Ala Pro Glu Gly Lys
        35                  40                  45

Leu Arg Leu Arg Pro Pro Lys Arg Leu Gln Lys Ser Leu Gly Thr Phe
    50                  55                  60

Asp Ala Thr Gly Pro Ala Gly Ala Cys Pro Gln Met Leu Ser Ser Thr
65                  70                  75                  80

Lys Ser Lys Asp Val Leu Phe Gln Leu Leu Gly Ser Ile Ala Asn Leu
                85                  90                  95

Pro Phe Val Gln Thr Val Thr Gly Gln Ser Glu Asp Cys Leu Thr Ile
            100                 105                 110

Thr Val Ala Arg Pro Glu Gly Thr Lys Ala Asn Ala Lys Leu Pro Val
        115                 120                 125

Leu Phe Trp Ile Phe Gly Gly Gly Phe Gln Leu Gly Trp Ser Ser Met
    130                 135                 140

Tyr Asp Gly Thr Gly Leu Val Asn Tyr Gly Val Gly Leu Asn Gln Pro
145                 150                 155                 160

Phe Ile Phe Val Ala Val Asn Tyr Arg Val Gly Gly Phe Gly Phe Leu
                165                 170                 175

Pro Gly Lys Glu Ile Gln Asp Glu Gly Ser Gly Asn Leu Gly Leu Leu
            180                 185                 190

Asp Gln Arg Met Gly Leu Glu Trp Val Ala Asp Asn Ile Ala Ala Phe
        195                 200                 205

Gly Gly Asp Pro Asp Lys Val Thr Ile Trp Gly Glu Ser Ala Gly Ala
    210                 215                 220

Ile Ser Val Phe Asp Gln Met Ala Leu Tyr Asp Gly Asp His Lys Tyr
225                 230                 235                 240

Asn Gly Lys Pro Leu Phe Arg Gly Ala Ile Met Asn Ser Gly Ser Val
                245                 250                 255

Val Pro Cys Glu Pro Leu Asp Gly Val Lys Gly Gln Ala Val Tyr Asp
            260                 265                 270

Gln Val Ile Glu Thr Ala Gly Cys Val Gly Lys Ser Asp Thr Leu Asp
        275                 280                 285

Cys Leu Arg Asn Leu Asp Tyr Thr Asp Phe Leu Asn Ala Val Asn Ser
    290                 295                 300

Val Pro Gly Ile Leu Ser Tyr Asn Ser Leu Ala Leu Ser Tyr Leu Pro
305                 310                 315                 320

Arg Pro Asp Gly Arg Thr Leu Thr Ala Ser Pro Asp Val Leu Ala Leu
                325                 330                 335

Asn Arg Lys Tyr Ala Ala Val Pro Met Ile Ile Gly Asn Gln Glu Asp
            340                 345                 350

Glu Gly Thr Leu Phe Ser Ile Phe Gln Ala Asn Leu Thr Ser Asp Glu
        355                 360                 365

Lys Leu Val Thr Tyr Leu Lys Asp Tyr Tyr Phe His Ala Ala Thr Ala
    370                 375                 380

Pro Gln Leu Arg Asp Leu Val Arg Thr Tyr Gly Ser Gly Leu Gly Ala
385                 390                 395                 400

Ile Thr Gln Asn Ser Pro Phe Arg Ser Gly Ile Leu Asn Glu Ile Phe
                405                 410                 415

Pro Gly Phe Lys Lys Arg Ala Ala Ile Leu Gly Asp Leu Val Phe Ile
            420                 425                 430

Leu Ala Arg Arg Leu Phe Leu Gln Ala Ala Thr Gly Val Asn Gly Asp
        435                 440                 445

Val Pro Ser Trp Ala Tyr Met Ser Ser Tyr Asp Gln Gly Thr Pro Val
    450                 455                 460

Leu Gly Thr Phe His Gly Ser Asp Leu Ile Gln Val Phe Tyr Gly Ile
465                 470                 475                 480

Lys Asp Asn Tyr Ala Ala Arg Ser Ile Arg Ser Tyr Tyr Ile Asn Phe
                485                 490                 495

Val Tyr Ser Leu Asp Pro Asn Ala Ala Ser Asn Ala Lys Tyr Pro Ser
            500                 505                 510

Trp Pro Gln Trp Lys Asn Gly His Lys Leu Met Gln Phe Phe Ala Asp
        515                 520                 525

Lys Val Thr Ile Ile Asn Asp Asp Phe Arg Ser Ala Ser Tyr Asp Trp
    530                 535                 540

Leu Val Lys Asn Ile Ala Ser Leu Lys Phe
545                 550

<210>  30
<211>  557
<212>  PRT
<213>  Diaporthe ampelina

<400>  30

Leu Pro Ala Arg Gln Gly Ser Pro Val Val Asn Gly Arg Ala Ala Ser
1               5                   10                  15

Pro Thr Val Val Leu Ser Pro Ser Asn Thr Val Val Gly Leu Ala Ser
            20                  25                  30

Gly Asn Val Glu Lys Tyr Ala Gly Ile Pro Phe Ala Asp Pro Pro Thr
        35                  40                  45

Gly Ser Leu Arg Leu Lys Pro Pro Lys Lys Leu Ser Thr Asn Leu Gly
    50                  55                  60

Asp Ala Tyr Asp Ala Ile Asn Pro Ala Ala Ala Cys Pro Gln Met Leu
65                  70                  75                  80

Val Ser Thr Gly Glu Asn Gln Ser Leu Phe Leu Gln Val Leu Gly Asp
                85                  90                  95

Leu Val Ser Leu Pro Ile Ile Gln Ala Ala Thr Asn Gln Ser Glu Asp
            100                 105                 110

Cys Leu Thr Val Ser Val Ile Arg Pro Ala Gly Val Glu Ala Gly Ala
        115                 120                 125

Asn Leu Pro Val Leu Tyr Trp Ile Phe Gly Gly Ala Phe Glu Leu Gly
    130                 135                 140

Trp Ala Ala Met Tyr Asp Gly Thr Asp Leu Leu Asp Gln Ala Ile Ser
145                 150                 155                 160

Gln Asp Gln Pro Tyr Ile Phe Val Ala Val Asn Tyr Arg Val Ala Gly
                165                 170                 175

Phe Gly Phe Met Pro Gly Lys Glu Ile Leu Ala Asp Gly Ser Ser Asn
            180                 185                 190

Leu Gly Leu Leu Asp Gln Arg Ala Gly Leu Glu Trp Val Ala Asp Asn
        195                 200                 205

Ile Glu Ala Phe Gly Gly Asp Pro Thr Lys Val Thr Leu Trp Gly Glu
    210                 215                 220

Ser Ala Gly Ala Ile Ser Val Phe Asp Gln Met Ala Leu Tyr Gly Gly
225                 230                 235                 240

Asp Asn Asn Tyr Asn Gly Lys Pro Leu Phe Arg Gly Ala Ile Met Asn
                245                 250                 255

Ser Gly Ser Ile Val Pro Ala Asp Pro Val Asp Cys Pro Lys Gly Gln
            260                 265                 270

Val Ile Tyr Asp Thr Val Val Lys Glu Gly Gly Cys Ser Gly Ala Ala
        275                 280                 285

Asp Thr Leu Glu Cys Leu Arg Ala Leu Pro Tyr Asp Lys Phe Leu Asp
    290                 295                 300

Ala Ala Asn Ser Val Pro Gly Phe Leu Gly Tyr Gln Ser Val Ala Leu
305                 310                 315                 320

Ser Tyr Leu Pro Arg Pro Asp Gly Thr Val Leu Pro Asp Ser Pro Asp
                325                 330                 335

Val Leu Ala Ala Gln Gly Arg Tyr Ala Ala Val Pro Met Ile Asn Gly
            340                 345                 350

Asp Gln Glu Asp Glu Gly Thr Ile Phe Thr Leu Phe Thr Ser Asn Val
        355                 360                 365

Thr Thr Thr Gln Asp Leu Leu Glu Tyr Leu Ala Thr Tyr Tyr Phe Asn
    370                 375                 380

Ser Ala Ser Asp Glu Gln Leu Thr Ala Leu Leu Asp Thr Tyr Pro Gln
385                 390                 395                 400

Asp Ile Thr Gln Gly Ser Pro Phe Arg Thr Gly Ile Leu Asn Glu Val
                405                 410                 415

Tyr Pro Gln Phe Lys Arg Ile Ser Ala Leu Leu Gly Asp Leu Val Phe
            420                 425                 430

Thr Leu Ser Arg Arg Gly Phe Leu Glu Asn Thr Asn Ala Ala Asn Pro
        435                 440                 445

Ser Val Pro Ser Trp Ser Tyr Leu Ala Ser Tyr Asn Phe Gly Thr Pro
    450                 455                 460

Phe Leu Gly Thr Phe His Gly Ser Asp Leu Leu Gln Val Phe Phe Gly
465                 470                 475                 480

Ile Tyr Asp Asn Tyr Ala Ala Arg Ser Ile Arg Thr Tyr Tyr Phe Asn
                485                 490                 495

Phe Leu His Asn Leu Asp Pro Asn Val Gly Tyr Gly Ser Tyr Pro Asn
            500                 505                 510

Trp Pro Gln Trp Lys Asp Thr Gly Glu Gly Asn Gln Leu Val Gln Phe

515                 520                 525

Tyr Ala Leu Ser Gln Asn Tyr Leu Ala Asp Asp Phe Arg Ser Asp Thr
    530                 535                 540

Tyr Ala Phe Ile Lys Glu Asn Ile Ala Ser Leu Arg Ile
545                 550                 555

<210>  31
<211>  549
<212>  PRT
<213>  Ophiostoma piceae

<400>  31

Met Pro Lys Arg Arg Asp Gln Ser Ile Glu Pro Arg Thr Thr Val Asn
1               5                   10                  15

Val Asn Tyr Pro Glu Gly Glu Val Val Gly Val Ser Val Leu Gly Ile
            20                  25                  30

Glu Ser Phe Arg Gly Val Pro Phe Ala Gln Pro Pro Val Gly Asn Leu
        35                  40                  45

Arg Leu Lys Pro Pro Val Arg Tyr Thr Glu Asn Ile Gly Thr Lys Asp
    50                  55                  60

Thr Thr Gly Ile Gly Pro Ser Cys Pro Gln Met Tyr Leu Ser Thr Gly
65                  70                  75                  80

Asn Gly Glu Leu Leu Phe Gln Leu Val Gly Asn Leu Ile Asn Ile Pro
                85                  90                  95

Leu Phe Gln Thr Ala Thr Leu Ser Ser Glu Asp Cys Leu Thr Leu Asn
            100                 105                 110

Ile Gln Arg Pro Ala Gly Thr Thr Ser Asn Ser Ser Leu Pro Val Leu
        115                 120                 125

Phe Trp Ile Phe Gly Gly Gly Phe Glu Leu Gly Thr Asn Gln Tyr Tyr
    130                 135                 140

Asp Gly Ile Asp Leu Leu Thr Glu Gly Ile Ser Leu Gly Glu Pro Phe
145                 150                 155                 160

Ile Phe Val Ala Ile Asn Tyr Arg Val Gly Gly Phe Gly Phe Leu Gly
                165                 170                 175

Gly Lys Glu Ile Lys Ala Asp Gly Ser Ser Asn Leu Gly Leu Leu Asp

180                 185                 190

Gln Arg Ile Ala Leu Glu Trp Val Ala Asp Asn Ile Ala Ser Phe Gly
        195                 200                 205

Gly Asp Pro Ser Lys Val Thr Ile Trp Gly Glu Ser Ala Gly Ser Ile
    210                 215                 220

Ser Val Phe Asp Gln Met Ala Leu Tyr Gly Gly Asn Asn Lys Tyr Lys
225                 230                 235                 240

Gly Lys Ala Leu Phe Arg Gly Gly Ile Met Asn Ser Gly Ser Val Val
                245                 250                 255

Pro Ala Ala Pro Val Asp Gly Val Lys Ala Gln Ala Ile Tyr Asp His
            260                 265                 270

Val Val Ser Glu Ala Gly Cys Ala Gly Thr Ser Asp Thr Leu Ala Cys
        275                 280                 285

Leu Arg Thr Val Asp Tyr Thr Lys Phe Leu Thr Ala Val Asn Ser Val
    290                 295                 300

Pro Gly Ile Val Ser Tyr Ser Ser Ile Ala Leu Ser Tyr Leu Pro Arg
305                 310                 315                 320

Pro Asp Gly Val Val Leu Ile Asp Ser Pro Glu Glu Ile Val Lys Asn
                325                 330                 335

Lys Gln Tyr Ala Ala Val Pro Met Ile Ile Gly Asp Gln Glu Asp Glu
            340                 345                 350

Gly Thr Leu Phe Ala Val Leu Pro Asn Asn Ile Thr Ser Thr Ala Lys
        355                 360                 365

Ile Val Gln Tyr Phe Gln Asp Leu Tyr Phe Tyr Asn Ala Thr Lys Glu
    370                 375                 380

Gln Leu Thr Ala Phe Val Asn Thr Tyr Pro Thr Asp Ile Thr Ala Gly
385                 390                 395                 400

Ser Pro Phe Asn Thr Gly Ile Phe Asn Glu Leu Tyr Pro Gly Phe Lys
                405                 410                 415

Arg Leu Ala Ala Ile Leu Gly Asp Met Thr Phe Thr Leu Ala Arg Arg
            420                 425                 430

Ala Phe Leu Gln Leu Cys Ser Glu Val Asn Pro Asp Val Pro Ser Trp
        435                 440                 445

Ser Tyr Leu Ala Ser Tyr Asp Tyr Gly Phe Pro Phe Leu Gly Thr Phe
    450                 455                 460

His Ala Thr Asp Ile Leu Gln Val Phe Tyr Gly Val Leu Pro Asn Tyr
465                 470                 475                 480

Ala Ser Gly Ser Ile Gln Lys Tyr Tyr Ile Asn Phe Val Thr Thr Gly
                485                 490                 495

Asp Pro Asn Lys Gly Ala Ala Val Asp Ile Gln Trp Pro Gln Trp Ser
            500                 505                 510

Ala Lys Lys Asn Ile Leu Gln Ile Tyr Ala Thr Lys Ala Val Ile Val
        515                 520                 525

Ala Asp Asn Phe Arg Ala Lys Ser Tyr Glu Tyr Leu Tyr Asn Asn Ile
    530                 535                 540

Gly Ile Phe Arg Ile
545

<210> 32
<211> 528
<212> PRT
<213> Pleurotus sapidus

<400> 32

Thr Pro Leu Pro Arg Ala Asn Ser Val Thr Leu Asp Ser Ala Thr Phe
1               5                   10                  15

Thr Gly Thr Thr Ser Gly Arg Val Thr Lys Phe Leu Gly Ile Pro Tyr
            20                  25                  30

Ala Gln Pro Pro Thr Gly Asp Arg Arg Phe Arg Leu Pro Glu Pro Ile
        35                  40                  45

Pro Pro Tyr Thr Gly Thr Val Arg Ala Thr Ala Phe Gly Pro Ala Cys
    50                  55                  60

Pro Gln Gln Ser Ala Arg Leu Pro Leu Pro Asp Gly Leu Ala Ser Asp
65                  70                  75                  80

Val Val Asp Leu Ile Val Asn Thr Ala Tyr Lys Ala Val Phe Pro Asp
                85                  90                  95

Asn Glu Asp Cys Leu Ser Ile Asn Val Val Val Pro Thr Ser Ala Thr
            100                 105                 110

Pro Thr Ser Lys Leu Pro Val Ala Val Trp Ile Phe Gly Gly Gly Phe
        115                 120                 125

Glu Leu Gly Ser Pro Ser Leu Tyr Asp Gly Gly Leu Ile Val Glu Arg
    130                 135                 140

Ser Ile Gln Leu Gly Glu Pro Val Ile Tyr Val Ser Met Asn Tyr Arg
145                 150                 155                 160

Leu Ser Ala Phe Gly Phe Leu Ala Ser Gln Glu Val Lys Asp Thr Gly
                165                 170                 175

Val Gly Asn Leu Gly Leu Gln Asp Gln Arg Glu Ala Leu Arg Trp Ile
            180                 185                 190

Gln Lys Tyr Ile Ser Ser Phe Gly Gly Asp Pro Thr Lys Val Thr Ile
        195                 200                 205

Trp Gly Glu Ser Ala Gly Ala Ile Ser Val Ala Leu His Met Val Ala
    210                 215                 220

Asn Asn Gly Asn His Glu Gly Leu Phe Arg Gly Ala Phe Met Gln Ser
225                 230                 235                 240

Gly Ser Pro Ile Pro Val Gly Asp Ile Ser His Gly Gln Thr Tyr Tyr
                245                 250                 255

Asp Ala Ile Ala Ala Glu Thr Gly Cys Ser Ser Ala Ala Asp Thr Leu
            260                 265                 270

Ala Cys Leu Arg Ser Val Pro Tyr Ala Thr Leu Lys Thr Ala Val Asp
        275                 280                 285

Arg Thr Pro Phe Ile Phe Asp Tyr Gln Ser Leu Ala Leu Ala Arg Ile
    290                 295                 300

Pro Arg Ala Asp Gly Val Phe Leu Thr Asp Asn Pro Gln Arg Leu Val
305                 310                 315                 320

Gln Ala Gly Lys Val Ala Asn Val Pro Phe Val Thr Gly Asp Cys Asp
                325                 330                 335

Asp Glu Gly Thr Leu Phe Ser Leu Ala Asn Leu Asn Val Thr Thr Thr
            340                 345                 350

Ser Gln Val Arg Thr Tyr Ile Lys Thr Phe Phe Met Pro Gln Ser Thr
        355                 360                 365

Asn Ala Glu Leu Asp Gln Met Leu Asn His Tyr Pro Leu Asp Leu Thr
    370                 375                 380

Gln Gly Ser Pro Phe Asp Thr Gly Ile Leu Asn Ala Leu Ser Pro Gln
385                 390                 395                 400

Phe Lys Arg Leu Ala Ala Phe Gln Gly Asp Ala Val Phe Gln Ala Pro
                405                 410                 415

Arg Arg Phe Phe Leu Gln Gln Arg Ser Gly Lys Gln Asn Thr Trp Ala
            420                 425                 430

Phe Leu Ser Lys Arg Phe Lys Val Ala Pro Phe Leu Gly Ser Phe His
        435                 440                 445

Ala Ser Asp Ile Leu Asn Val Tyr Phe Gly Gly Glu Leu Gly Asp Tyr
    450                 455                 460

Leu Ile Asn Phe Val Asn Asn Leu Asp Pro Asn Gly Gln Gly Arg Gly
465                 470                 475                 480

Ile Asn Trp Pro Lys Tyr Thr Thr Ser Ser Pro Asn Leu Val Thr Phe
                485                 490                 495

Asn Asp Asn Leu Leu Phe Pro Val Thr Ile Thr Gln Asp Thr Phe Arg
            500                 505                 510

Thr Asp Ala Ile Asn Phe Leu Thr Gly Val Thr Leu Ala Asn Pro Leu
        515                 520                 525

## Description

### FIELD OF THE INVENTION

The present invention relates to solution stable enzyme compositions that are suitable for use in industrial applications such as in the manufacture of pulp and paper.

### BACKGROUND OF THE INVENTION

Solution stable enzyme compositions intended for commercial use advantageously have sufficient stability during storage and satisfactory shelf life. Compositions comprising sterol esterase are useful for hydrolysis and synthesis of esters, for example esters of phytosterols, cholesterol and glycerol with carboxylic acids, particularly fatty acids. Such compositions can be applied industrially as processing aid, e.g. in the production and recycling of pulp and paper as far as the enzymes are active under conditions typically encountered in these processes.

To enhance the stability of aqueous solutions of enzymes and generally proteins, many strategies have been pursued, and reviewed by Gianfreda et al. in Molecular and Cellular Biochemistry 1991 pp 97-128. These strategies include protein modification by glycosylation, pegylation, cross-linking, site-directed mutagenesis and chemical modification of amino acids. Another strategy is the addition of excipients, for example pH buffers, salts, surfactants, amino acids, sugars, organic solvents, polymers and cyclodextrins.

For example hydroxypropyl-β-cyclodextrin, sorbitol and the surfactant polyethylene glycol sorbitan monolaurate, also known under the trade name Tween 20, have been evaluated for their stabilizing ability on the protein porcine growth hormone by Charman et al. in Pharmaceutical Research 1993 pp 954-962, who concluded that in contrast to hydroxypropyl-β-cyclodextrin and Tween 20, sorbitol was only marginally effective and offered no advantage against precipitation.

An excipient effective in stabilizing all protein and enzyme solutions is unknown. Nevertheless, particular excipients that stabilize specific enzymes have been found. For example serine protease inhibitors were found to stabilize lipases (EP2521732 B1). Lipolytic enzymes in liquid products for pulp and paper applications, for example Resinase®HT and Stickaway® from Novozymes disclosed in associated data sheets, are stabilized with propylene glycol. The pH of Stickaway® is between 6.5 and 8.5. At pH values above 8 the sterol esterase from *Melanocarpus albomyces* was clearly less stable, and more stable within the pH range between 3 and 7 in a setup by Kontkanen et al. in Applied Microbiology and Biotechnology 2006 pp 696-704.

Pleiss et al. in Journal of Molecular Catalysis B 2000 pp 491-508 corroborated that almost all lipases, many esterases and all known cutinases, just as serine proteases, have a catalytic triad composed of serine, histidine and aspartate.

Instead of aspartate, glutamate is present in sterol esterases' catalytic triad, which is composed of serine, histidine and glutamate.

A common lipase, also called triacylglycerol lipase and classified by the IUBMB Enzyme Nomenclature as EC 3.1.1.3, for example the lipases from *Candida antarctica, Thermomyces lanuginosus* and *Rhizomucor miehei,* investigated by Kontkanen et al. in Journal of Biochemistry 2004 pp 51-59, is highly active on glycerol esters, but is without measurable activity on sterol esters.

A sterol esterase, classified as EC 3.1.1.13, hydrolyzes many different esters, including the esters of sterol and glycerol with short and long chain carboxylic acids.

An acetylcholine esterase, classified as EC 3.1.1.7, hydrolyzes acetylcholine and other choline esters. The distinction of esterase and lipase due to their sequence and three-dimensional structure is reviewed by Fojan et al. in Biochimie 2000 pp 1033-1041.

Enzyme activities on plant and wood sterol ester mixtures were compared to activities on cholesterol esters by Kontkanen et al. in Journal of Biochemistry 2004 pp 51-59, who concluded that pure cholesterol esters can be used as model substrates for sterol esterases. Examples of sterols are cholesterol, phytosterols and ergosterol, which naturally occur in animals, plants and fungi, respectively. Sterol esterases from animal sources are genuine cholesterol esterases. Cholesterol esterases from microorganisms are in general terms sterol esterases.

Anyhow, sterol esterases and cholesterol esterases are one enzyme class of EC 3.1.1.13.

To maintain stability of an aqueous solution of the sterol esterase from *Melanocarpus albomyces,* Kontkanen et al. in Enzyme and Microbial Technology 2006 pp 265-273 used 1 % of the nonionic surfactant polyethylene glycol p-isooctyl-phenyl ether, also known under the trade name Triton X-100.

Thus, a stabilizing compound that is effective for all enzymes has not been reported and there is a need for providing liquid compositions where enzymes remain stable.

### SUMMARY OF THE INVENTION

Previously unanticipated, the inventors have found that certain organic polyhydroxy compounds stabilize an aqueous composition comprising an enzyme having WGESAG sequence motif and a catalytic triad composed of S, H and E/D. The stabilizing effect can be achieved even without the addition of a surfactant, if the organic polyhydroxy compound has three or more hydroxyl groups per molecule.

According to the first aspect of the invention is provided a solution stable enzyme composition comprising:
(a) an enzyme component comprising an enzyme characterized by having the amino acids sequence WGESAG and a catalytic triad composed of S, H and E/D;
(b) a stabilizer component comprising an organic polyhydroxy compound that has three or more hydroxyl groups per molecule;
(c) an optional antimicrobial preservative component preventing growth of a microorganism; and
(d) water having dissolved therein said components (a), (b) and (c).

In an embodiment the concentration of the polyhydroxy compound in the composition is from 20 to 75 % by weight, preferably from 25 to 70 % by weight, more preferably from 30 to 65 % by weight, and most preferably from 33 to 60 % by weight. In an embodiment the lower limit of the concentration of the polyhydroxy compound is 20% by weight, preferably 22%, 24%, 25% by weight, more preferably 26%, 28%, 30% by weight, most preferably 33% by weight. In an embodiment the upper limit of the concentration of the polyhydroxy compound is 75% by weight, preferably 74%, 72%, 70% by weight, more preferably 68%, 66%, 65% by weight and most preferably 60% by weight.

In an embodiment the amount of water in the composition is at least 10 % by weight, preferably at least 12%, 14%, 15 % by weight, more preferably at least 16%, 18%, 20 % by weight and most preferably at least 25% by weight.

In an embodiment the pH of the composition is between 2 and 10, preferably between 2.5 and 9, more preferably between 3 and 8 and most preferably between 3.5 and 7.5.

According to the second aspect of the invention is provided a use, and a method of using, of the solution stable enzyme composition according to the first aspect in the manufacture of pulp. In an embodiment the solution stable enzyme composition is used by adding it in such a way that it is brought into a contact with material used in the manufacture of pulp where enzymatic treatment is needed.

According to the third aspect of the invention is provided a use, and a method of using, of the solution stable enzyme composition according to the first aspect in the manufacture of paper. In an embodiment the solution stable enzyme composition is used by adding it in such a way that it is brought into a contact with material used in the manufacture of paper where enzymatic treatment is needed.

### SEQUENCE LISTINGS

The following sequences are mature protein sequences without signal peptides.

The sequences are written from amino- to carboxyl-terminus. The actual enzyme molecules present in an aqueous solution can be shortened from both endings of the sequences (amino- and/or carboxyl-terminus) without loss of enzyme function. For example, compared to SEQ ID NO: 1, Kontkanen et al. in Enzyme and Microbial Technology 2006 pp 265-273 found that the first 13 amino-terminal amino acids of the sequence SEQ ID NO: 1 are absent in their preparation of sterol esterase from *Melanocarpus albomyces,* since their sequence started with AAPXVEISTG. Thus, in an embodiment are disclosed N-terminally truncated enzymes having enzyme activity.
SEQ ID NO: 1 is the sequence of a sterol esterase from *Melanocarpus albomyces.*
SEQ ID NO: 2 is the sequence of a sterol esterase from *Chaetomium thermophilum.*
SEQ ID NO: 3 is the sequence of a synthetic enzyme derived from alignment of the sequences of the sterol esterases from *Scytalidium thermophilum, Myceliophthora thermophila, Thielavia terestris, Corynascus thermophilus, Myriococcum thermophilum, Melanocarpus albomyces* and *Chaetomium thermophilum.*
SEQ ID NO: 4 is the sequence of a sterol esterase from *Myceliophthora thermophila.*
SEQ ID NO: 5 is the sequence of a sterol esterase from *Corynascus thermophilus.*
SEQ ID NO: 6 is the sequence of a sterol esterase from *Myriococcum thermophilum.*
SEQ ID NO: 7 is the sequence of a sterol esterase from *Thielavia australiensis.*
SEQ ID NO: 8 is the sequence of a sterol esterase from *Thielavia terestris.*
SEQ ID NO: 9 is the sequence of a sterol esterase from *Scytalidium thermophilum.*
SEQ ID NO: 10 is the sequence of a sterol esterase from *Malbranchea cinnamomea.*
SEQ ID NO: 11 is the sequence of a sterol esterase from *Thermomyces stellatus.*
SEQ ID NO: 12 is the sequence of a sterol esterase from *Chaetomium globosum.*
SEQ ID NO: 13 is the sequence of a sterol esterase from *Madurella mycetomatis.*
SEQ ID NO: 14 is the sequence of a sterol esterase from *Sordaria macrospora.*
SEQ ID NO: 15 is the sequence of a sterol esterase from *Podospora anserina.*
SEQ ID NO: 16 is the sequence of a sterol esterase from *Neurospora tetrasperma.*
SEQ ID NO: 17 is the sequence of a sterol esterase from *Neurospora crassa.*
SEQ ID NO: 18 is the sequence of a sterol esterase from *Coniochaeta ligniaria.*
SEQ ID NO: 19 is the sequence of a sterol esterase from *Cutaneotrichosporon oleaginosum.*
SEQ ID NO: 20 is the sequence of a sterol esterase from *Sporothrix schenckii.*
SEQ ID NO: 21 is the sequence of a sterol esterase from *Stachybotrys chlorohalonata.*
SEQ ID NO: 22 is the sequence of a sterol esterase from *Colletotrichum orchidophilum.*
SEQ ID NO: 23 is the sequence of a sterol esterase from *Colletotrichum incanum.*
SEQ ID NO: 24 is the sequence of a sterol esterase from *Colletotrichum tofieldiae.*
SEQ ID NO: 25 is the sequence of a sterol esterase from *Hypoxylon* sp. EC38.
SEQ ID NO: 26 is the sequence of a sterol esterase from *Aspergillus glaucus.*
SEQ ID NO: 27 is the sequence of a sterol esterase from *Eutypa lata.*
SEQ ID NO: 28 is the sequence of a sterol esterase from *Fusarium oxysporum.*
SEQ ID NO: 29 is the sequence of a sterol esterase from *Fusarium avenaceum.*
SEQ ID NO: 30 is the sequence of a sterol esterase from *Diaporthe ampelina.*
SEQ ID NO: 31 is the sequence of a sterol esterase from *Ophiostoma piceae.*
SEQ ID NO: 32 is the sequence of a sterol esterase from *Pleurotus sapidus* CAH17527.

### DETAILED DESCRIPTION

Sterol esterases are enzymes that act on sterol ester bonds, particularly sterol esterases catalyze the hydrolysis, alcoholysis, acidolysis, transacylation, transesterification and/or synthesis of sterol esters. In an embodiment the sterol esterases of the invention belong to the class EC 3.1.1.13.

Enzymes are catalytic proteins.

Proteins are polypeptides.

Polypeptides are long chains of amino acids linked by amide bonds. In an embodiment peptides are molecules composed of up to 20 amino acids, and polypeptides are molecules composed of more than 20 amino acids.

The generally accepted IUPAC single letter abbreviations for amino acids and their side chains in polypeptides are utilized, particularly S for serine, H for histidine, A for alanine, G for glycine, E for glutamic acid and glutamate, D for aspartic acid and aspartate, W for tryptophan.

A fragment of an enzyme characterized by a specific amino acid sequence is a polypeptide having one or more amino acids absent from the amino- and/or carboxyl-terminus of said sequence, and/or one or more deletions and/or insertions of one or more amino acids in said sequence, for example due to alternative splicing, wherein the fragment has enzyme activity. In an embodiment a fragment of an enzyme has the same or similar enzyme activity, and optionally stability, as the non-fragmented enzyme.

Propylene glycol is 1,2-propanediol, which is an organic polyhydroxy compound with two hydroxyl groups per molecule. 1,2-propanediol exists in two enantiomers and mixtures thereof, including the racemic mixture, that are encompassed in this definition of propylene glycol.

Sugar alcohols are organic compounds that can be produced by hydrogenation of carbohydrates, particularly monosaccharides, disaccharides, trisaccharides, oligosaccharides and polysaccharides. The hydrogenation causes reduction of an aldehyde- or a ketone-group to a hydroxyl-group. Monosaccharides are aldehydes or ketones that have two or more hydroxyl-groups per molecule. Thus the sugar alcohols have three or more hydroxyl-groups per molecule.

Cyclitols are cycloalkanes that have a hydroxyl-group on three or more ring atoms. The cyclitols have three or more hydroxyl-groups per molecule.

Pulp is a wet mass of material that is originally obtained from plants. Such pulp is manufactured from e.g. wood, cotton, papyrus, straw, fruits, paper and rags.

The ester of cholesterol with linoleic acid is cholesteryl linoleate.

Thermophilic fungi are fungi that grow at 45°C, preferably at 50°C, or higher temperatures.

A thermostable enzyme is an enzyme that retains at least 50% enzyme activity after incubation in an aqueous composition or environment at 50°C, preferably 60°C, more preferably 70°C, for 5 minutes, preferably for 10 minutes, more preferably for 30 minutes, most preferably for 1 hour. Enzyme activity can be determined according to Example 1 below.

The articles "a" and "an", as well as "another", are meant to refer to one or to more than one, that is to one or at least one, including several, of the grammatical object of "a", "an" or "another".

The word "comprise" and variations thereof such as "comprises" and "comprising" are meant inclusively and include additional possible components.

In an embodiment of the enzymes according to the invention the catalytic triad is composed of serine, histidine and glutamate/aspartate, and said serine of the catalytic triad is embedded in the sequence WGESAG.

A catalytic triad is a complex of three amino acid residues involved in catalysis. Such residues of a catalytic triad function as nucleophile, base and acid during catalysis. Beside a catalytic triad, also an oxyanion hole and a hydrophobic substrate binding site is formed by active site residues of the enzyme. Residues of the catalytic triad can be identified by mutation experiments, by structure determination or by sequence alignment with homologues that have known catalytic residues. Examples of sequence alignment tools are Clustal Omega, PfamScan, Muscle and Emboss Needle, which uses the Needleman-Wunsch algorithm. These tools are available online, for example at https://www.ebi.ac.uk/services/all and at https://www.ebi.ac.uk/Tools/pfa/.

Catalytic triads can be found in hydrolase and transferase enzymes. The positions of the catalytic triad residues in SEQ ID NO: 1-9 are S221, H466 and E353. Examples for enzymes according to the invention include, but are not limited to enzymes comprising amino acid sequences disclosed in SEQ ID NO: 1-32 and fragments thereof. Further examples for enzymes according to the invention include, but are not limited to sterol esterases from *Melanocarpus albomyces, Chaetomium thermophilum, Scytalidium thermophilum, Myceliophthora thermophila, Thielavia terestris, Thielavia australiensis, Corynascus thermophilus, Myriococcum thermophilum, Malbranchea cinnamomea, Thermomyces stellatus* and other thermophilic fungi. Such thermophilic fungi include, but are not limited to thermophilic ascomycetes (e.g. *Canariomyces thermophila, Chaetomidium pingtungium, Chaetomium britannicum, Chaetomium mesopotamicum, Chaetomium senegalensis, Chaetomium thermophile, Chaetomium virginicum, Corynascus sepedonium, Corynascus thermophilus, Crassicarpon thermophilum, Coonemeria aegyptiaca, Coonemeria crustacea, Dactylomyces thermophilus, Melanocarpus albomyces, Melanocarpus thermophilus, Myriococcum thermophilum, Crassicarpon hotsonii, Talaromyces byssochlamydioides, Talaromyces duponti, Talaromyces emersonii, Talaromyces thermophilus, Thermoascus aurantiacus, Thermomyces stellatus, Thielavia australiensis, Thielavia minor, Thielavia terricola*), thermophilic zygomycetes (e.g. *Rhizomucor miehei, Rhizomucor nainitalensis, Rhizomucor pusillus, Rhizopus microspores, Rhizopus rhizopodiformis*) and thermophilic deuteromycetes (e.g. *Acremonium alabamense, Acremonium thermophilum, Arthrinium pterospermum, Chrysosporium tropicum, Malbranchea cinnamomea, Myceliophthora fergusi, Myceliophthora hinnulea, Myceliophthora thermophila, Scytalidium indonesicum, Scytalidium thermophilum, Remersonia thermophila, Thermomyces ibadanensis, Thermomyces lanuginosus).*

Genes and polypeptides derived from thermophilic microorganisms that express thermostable enzymes, are interesting for enzyme compositions stable during storage and application. Particularly genes derived from thermophilic fungi, that lead to high enzyme yields in fungal expression hosts, are particularly interesting for enzyme production and stability during storage and application.

Maheshwari et al. in Microbiology and Molecular Biology Reviews 2000 pp 461-488 reported that among the eukaryotic organisms, only a few species of fungi have the ability to thrive at temperatures between 45 and 55°C. Such fungi comprise thermophilic species, which are not as extreme as thermophilic species of bacteria and archaea. Maheshwari et al. estimated that only some 30 species out of approximately 50000 known fungal species breach this upper temperature limit of eukaryotes. Salar et al. in Journal of Agricultural Technology 2007 pp 77-107 reported 42 species of thermophilic fungi.

Further examples for enzymes according to the invention include, but are not limited to sterol esterases from fungi, particularly *Basidiomycota,* particularly *Pleurotus species.* Such examples for enzymes according to the invention also include, but are not limited to sterol esterases from *Ascomycota,* particularly *Melanocarpus, Chaetomium, Chaetomidium, Corynascus, Crassicarpon, Canariomyces, Colletotrichum, Coonemeria, Crassicarpon, Dactylomyces, Malbranchea, Myriococcum, Neurospora, Ophiostoma, Talaromyces, Thermoascus, Thermomyces, Thielavia, Fusarium* and *Aspergillus species.*

One method of demonstrating relationship among enzymes is sequence comparison.

Percentages of sequence identity were calculated with the algorithm Clustal Omega, available online at https://www.ebi.ac.uk/Tools/msa/.

**Table 1. Percent identity matrix of closely related enzymes, sterol esterases derived from thermophilic fungi SEQ ID NO:**

| | **1** | **2** | **3** | **4** | **5** | **6** | **7** | **8** | **9** | **10** |
|---|---|---|---|---|---|---|---|---|---|---|
| **1** | 100.0% | 80.7% | 82.8% | 84.6% | 85.7% | 84.6% | 81.0% | 79.2% | 75.5% | 63.0% |
| **2** | 80.7% | 100.0% | 97.9% | 78.1% | 78.7% | 77.6% | 75.8% | 74.6% | 75.8% | 60.2% |
| **3** | 82.8% | 97.9% | 100.0% | 80.3% | 80.8% | 79.8% | 78.0% | 76.7% | 78.0% | 61.7% |
| **4** | 84.6% | 78.1% | 80.3% | 100.0% | 91.8% | 91.6% | 80.8% | 79.2% | 76.5% | 61.9% |
| **5** | 85.5% | 78.7% | 80.8% | 91.8% | 100.0% | 91.2% | 79.4% | 77.8% | 75.5% | 62.0% |
| **6** | 84.6% | 77.6% | 79.8% | 91.6% | 91.2% | 100.0% | 79.9% | 78.3% | 76.9% | 63.3% |
| **7** | 81.0% | 75.8% | 78.0% | 80.8% | 79.4% | 79.9% | 100.0% | 77.2% | 74.4% | 60.6% |
| **8** | 79.2% | 74.6% | 76.7% | 79.2% | 77.8% | 78.3% | 77.2% | 100.0% | 71.9% | 61.3% |
| **9** | 75.5% | 75.8% | 78.0% | 76.5% | 75.5% | 76.9% | 74.4% | 71.9% | 100.0% | 60.4% |
| **10** | 63.0% | 60.2% | 61.7% | 61.9% | 62.0% | 63.3% | 60.6% | 61.3% | 60.4% | 100.0% |

**Table 2. Percent identity matrix of enzymes distinct from sterol esterases**

| | | A | B | C | D | E | F | G | H | I | J | K |
|---|---|---|---|---|---|---|---|---|---|---|---|---|
| A: | CAI96520 | 100% | 46% | 38% | 19% | 16% | 18% | 20% | 14% | 28% | 27% | 31% |
| B: | P32947 | 46% | 100% | 42% | 15% | 14% | 18% | 18% | 15% | 26% | 27% | 28% |
| C: | ACX69980 | 38% | 42% | 100% | 15% | 17% | 16% | 16% | 15% | 31% | 28% | 30% |
| D: | CAA83122 | 19% | 15% | 15% | 100% | 6% | 16% | 20% | 23% | 13% | 14% | 14% |
| E: | AAC08588 | 16% | 14% | 17% | 6% | 100% | 32% | 13% | 9% | 12% | 16% | 12% |
| F: | CAA00250 | 18% | 18% | 16% | 16% | 32% | 100% | 11% | 16% | 16% | 18% | 16% |
| G: | AMR67078 | 20% | 18% | 16% | 20% | 13% | 11% | 100% | 18% | 15% | 14% | 14% |
| H: | P00590 | 14% | 15% | 15% | 23% | 9% | 16% | 18% | 100% | 13% | 11% | 11% |
| I: | CAA36703 | 28% | 26% | 31% | 13% | 12% | 16% | 15% | 13% | 100% | 31% | 32% |
| J: | P04058.2 | 27% | 27% | 28% | 14% | 16% | 18% | 14% | 11% | 31% | 100% | 32% |
| K: | P37967.2 | 31% | 28% | 30% | 14% | 12% | 16% | 14% | 11% | 32% | 32% | 100% |

Caption to table 2: The used sequence accession codes stand for the following enzymes:
A: CAI96520 for the sterol esterase from *Melanocarpus albomyces*
B: P32947 for the lipase 3 from *Candida rugosa*
C: ACX69980 for the lipase from *Geotrichum candidum*
D: CAA83122 for the lipase B from *Candida antarctica*
E: AAC08588 for the lipase from *Thermomyces lanuginosus*
F: CAA00250 for the lipase from *Rhizomucor miehei*
G: AMR67078 for the lipase from *Pseudomonas alcaligenes*
H: P00590 for the cutinase from *Fusarium solani*
I: CAA36703 for the protein D2 from *Dictyostelium discoideum*
J: P04058.2 for the acetylcholin esterase from *Torpedo californica*
K: P37967.2 for the para-nitrobenzyl esterase from *Bacillus subtilis*

The serine of the catalytic triad of sterol esterases is embedded in the conserved sequence WGESAG. This sequence of WGESAG is absent in related but different enzymes with glutamate in the catalytic triad, e.g. acetylcholine esterase from *Torpedo californica,* para-nitrobenzyl esterase from *Bacillus subtilis,* lipases from *Geotrichum candidum* and *Candida rugosa,* which have instead the sequence FGESAG. Absence of the WGESAG sequence, actually of any GXSXG motif, in an unrelated family of cholesterol esterases derived from actinomycetes bacteria was revealed by Xiang et al. in Biochimica et Biophysica Acta 2007 pp 112-120. Different from common lipases, which are not active on sterol esters, the lipase from *Candida rugosa* is active on both substrates, glycerol esters and sterol esters. Beside substrate specificity, sequence features are determining properties to classify enzymes. In some cases sequence features are more accurate than specific activities as classification means. Due to the sequence-structure-function relationship, sequence features and substrate specificities are linked. Also other enzyme properties like stability and receptiveness to certain stabilizer components have their root cause in specific sequences.

Thus, in an embodiment the stabilizing effect the inventors have found for the polyhydroxy compound is characteristic for the enzymes that preferably have both the WGESAG motif and the catalytic triad composed of S, H and E/D, wherein the S residue is part of said motif. In the Examples below, this stabilizing effect is shown for various enzymes carrying said motif and the catalytic triad, and with various polyhydroxy compounds. Thus, the above parameters define a limited set of enzymes and polyhydroxy compounds for which the stabilizing effect is shown.

An enzyme according to the invention can be isolated from its original biological source, or it can be produced in a cell-free system or produced as a secreted or intracellular protein in its original host or in an expression host, such as in a heterologous expression host. Such expression hosts are, but are not limited to filamentous fungi, yeasts, bacteria, plants and algae, for example *Trichoderma reesei, Aspergillus oryzae, Pichia pastoris, Bacillus subtilis* and *Escherichia coli;* and were reviewed by Fernández et al. in Advanced Technologies for Protein Complex Production and Characterization 2016 pp 15-24, and by Yin et al. in Journal of Biotechnology 2007 335-347. Kontkanen et al. in Applied Microbiology and Biotechnology 2006 pp 696-704 described expression of the sterol esterase from *Melanocarpus albomyces* in *Trichoderma reesei.*

An enzyme according to the invention - and a gene encoding the enzyme according to the invention - can be derived from polypeptides and nucleic acids found in nature, or from a synthetic polypeptide or synthetic nucleic acid with sequence information derived from nucleic acids or polypeptides found in nature. Such sequence information can be derived from more than one sequence found in nature, for example calculation of a common ancestor sequence, calculation of a synthetic sequence from an alignment of known homologous sequences, particularly calculation of the most frequent amino acid at each position and derivation of a consensus sequence.

Furthermore an enzyme according to the invention - and a gene encoding the enzyme according to the invention - can contain one or more alterations without loss of function. Examples of such alterations are insertions, deletions and/or substitutions, preferably conservative substitutions, relative to a polypeptide or nucleic acid found in nature. The experimental exchangeability of amino acids in proteins was reviewed by Yampolsky and Stoltzfus in Genetics 2005 pp 1459-1472. Amino acid alterations are designated by their single letters separated by a slash, e.g. E/D means E or D. A particular example for such conservative substitutions are exchanges between E and D, because E and D are both acidic amino acids that differ only in one methylene spacer in their side chains. Further examples of the conservative substitutions are substitutions within the group of basic amino acids (e.g. R/K/H), acidic amino acids and their amides (e.g. E/D/N/Q), aromatic amino acids (e.g. W/F/Y), hydrophobic amino acids (e.g. F/L/I/V/A), tiny amino acids (e.g. G/A/S), medium amino acids (e.g. T/S/A/V/M/C), charged amino acids (e.g. E/D/R/K/H) and other polar amino acids (e.g. S/T/N/Q/H). Beside substitutions by the twenty canonical amino acids, also other genetically encoded amino acids, for example selenocysteine and pyrrolysine, and so-called unnatural amino acids can be incorporated in proteins. Example of such unnatural amino acids are reviewed by Wang et al. in Chemistry & Biology 2009 pp 323-336. By stepwise solid-phase peptide synthesis any available amino acid can be incorporated in peptides, which can be chemically ligated to larger peptides and polypeptides, to produce proteins and enzymes.

Furthermore an enzyme according to the invention can be a fusion polypeptide in which another polypeptide and/or oligopeptide is fused at the amino- and/or carboxyl-terminus to a polypeptide characterized by enzyme activity on sterol esters. Examples of such oligopeptides and polypeptides are, but are not limited to polyhistidine-tags, signal peptides, linkers, binding domains, antibodies, chaperones, fluorescent proteins and enzymes, for example with cholesterol oxidase activity.

The concentration of enzymes in compositions according to the invention is selected from 0.0001 to 10 % by weight and any range inbetween. Such percentage is meant as active enzyme protein weight per total weight of composition. In another embodiment the enzyme concentration is from 0.01 to 10 % by weight, preferable 0.1 to 8 % by weight and more preferable 1 to 5% by weight. Such percentages are meant on dry matter basis. In another embodiment the enzyme concentration is specified as enzyme activity per composition weight and selected from 10 to 100000 SEU/g, preferable from 100 to 50000 SEU/g, more preferable from 200 to 30000 SEU/g and most preferable from 400 to 10000 SEU/g. The enzyme activity can be determined using the method described in Example 1 below.

In an embodiment the enzyme is a sterol esterase. Sterol esterases are versatile enzymes, which have broad substrate specificity. Beside sterol and glycerol esters of carboxylic acids (for example short and long chain carboxylic acids, saturated and unsaturated fatty acids), also other natural and artificial esters, for example polyesters (e.g. polyethylene terephthalate), polymers comprising vinyl acetate monomer (e.g. polyvinyl acetate) and para-nitrophenyl esters can be substrates and/or products of enzymes according to the invention. Therefore, such enzymes are useful in many industrial applications, particularly in pulp and paper, food, feed, textile, detergent, personal care and diagnostic industries. Specific examples within such industries are, but are not limited to use in a cleaning application, particularly for laundry and/or contact lenses, use as processing aid in manufacture of polyester textiles and/or wool, use in production and/or recycling of paper and/or pulp, use in a biosensor and/or a diagnostic reagent for measurement of total cholesterol (e.g. in blood) and use in synthesis of sterol esters. Examples of use of such sterol esters, particularly esters of cholesterol and phytosterol, more particularly stigmasteryl oleate, are use in food (e.g. added to margarines), feed, cosmetics and pharmaceutical formulations, as well as use in technical applications such as in liquid crystal display, which contains cholesterol esters.

Examples of sterols according to the invention include, but are not limited to, cholesterol, ergosterol, lanosterol, phytosterols, sitosterol, stigmasterol, campesterol, sitostanol, stigmastanol, campestanol, brassicasterol, fucosterol and cycloartenol.

Examples of sterol esters according to the invention include, but are not limited to, esters of cholesterol, esters of ergosterol, esters of lanosterol, esters of phytosterols, esters of sitosterol, esters of stigmasterol, esters of campesterol, esters of sitostanol, esters of stigmastanol, esters of campestanol esters of brassicasterol, esters of fucosterol and esters of cycloartenol.

Examples of esters of cholesterol according to the invention include, but are not limited to, cholesteryl linoleate, cholesteryl linolenate, cholesteryl myristoleate, cholesteryl palmitoleate, cholesteryl oleate, cholesteryl sapienate, cholesteryl arachidonate, cholesteryl erucate, cholesteryl crotonate. cholesteryl phenylpropionate, cholesteryl phenylacetate, cholesteryl cinnamate, cholesteryl benzoate, cholesteryl nitrobenzoate, cholesteryl dichlorobenzoate, cholesteryl chloroformate, cholesteryl formate, cholesteryl acetate, cholesteryl propionate, cholesteryl butyrate, cholesteryl valerate (cholesteryl pentanoate), cholesteryl caproate, cholesteryl heptanoate, cholesteryl octanoate (cholesteryl caprylate), cholesteryl nonanoate (cholesteryl pelargonate), cholesteryl decanoate (cholesteryl caprate), cholesteryl laurate, cholesteryl myristate, cholesteryl palmitate, cholesteryl stearate, cholesteryl eicosanoate (cholesteryl arachidate), cholesteryl docosanoate (cholesteryl behenate), cholesteryl tetracosanoate (cholesteryl lignocerate), cholesteryl hexacosanoate (cholesteryl cerotate), cholesteryl acetoacetate, cholesteryl hemisuccinate and cholesteryl isobutyrate.

Enzyme activity according to the invention can be measured using a sterol ester as enzyme substrate and measuring pH change or applying a pH-stat method, which measurers the release of carboxylic acid from the corresponding ester, or the method described below in Example 1, which measures the release of cholesterol from cholesteryl linoleate. In this detailed described method cholesteryl linoleate can be replaced by other esters of sterol, particularly esters of cholesterol, more particularly esters of cholesterol as exemplified above.

In an embodiment the enzyme component is spent fermentation broth containing the enzyme. The spent fermentation broth is obtainable e.g. by recombinant production of the enzyme. The spent fermentation broth may be concentrated and/clarified after production of the enzyme. In another embodiment a mixture of spent fermentation broths from several fermentations can be used.

In an embodiment the solution stable enzyme composition remains clear after 4 weeks storage at 4°C, preferably after 8 weeks storage at 4°C, more preferably after 24 week storage at 4°C. In an embodiment the solution stable enzyme composition remains clear after 4 weeks storage at 20°C, preferably after 8 weeks storage at 20°C, more preferably after 24 week storage at 20°C. In an embodiment the solution stable enzyme composition remains clear after 4 weeks storage at 37°C, preferably after 8 weeks storage at 37°C, more preferably after 24 week storage at 37°C. Thus, a solution stable enzyme composition is a composition which does not significantly turn turbid or precipitate during storage.

In an embodiment the solution stable enzyme composition has after 4 weeks storage at 4°C, preferably after 8 weeks storage at 4°C, a remaining enzyme activity of more than one third, preferably more than half of its activity compared to the enzyme activity directly after preparing such enzyme composition. In an embodiment the solution stable enzyme composition has after 4 weeks storage at 20°C, preferably after 8 weeks storage at 20°C, a remaining enzyme activity of more than one third, preferably more than half of its activity compared to the enzyme activity directly after preparing such enzyme composition. In an embodiment the solution stable enzyme composition has after 4 weeks storage at 37°C, preferably after 8 weeks storage at 37°C, a remaining enzyme activity of more than one third, preferably more than half of its activity compared to the enzyme activity directly after preparing such enzyme composition.

A polyhydroxy compound according to the invention is an organic polyhydroxy compound that has three or more hydroxyl groups, or four or more hydroxyl groups, or five or more hydroxyl groups, per molecule. Examples of such polyhydroxy compounds include, but are not limited to, pentaerythritol, trimethylolpropane, polyvinyl alcohol, certain carbohydrates, cyclitols and sugar alcohols. Examples of sugar alcohols according to the invention include, but are not limited to, sorbitol, mannitol, xylitol, glycerol, erythritol, threitol, arabitol, ribitol, galactitol, fucitol, iditol, inositol, volemitol, isomalt, maltitol, lactitol, maltotriitol, maltotetraitol, polyglycitol and hydrogenated starch hydrolysates. Inositol is an example for a sugar alcohol and simultaneously for a cyclitol. Naturally occurring cyclitols have six ring atoms and four or more hydroxyl-groups on ring atoms. Examples of such cyclitols are inositol, bornesitol, conduritol, ononitol, pinitol, pinpollitol, quebrachitol, valienol, viscumitol, ciceritol. Polyhydroxy compounds according to the invention are also carbohydrates that are soluble in aqueous solutions and that have three or more hydroxyl groups, or four or more hydroxyl groups, or five or more hydroxyl groups, per molecule. Examples of such carbohydrates include, but are not limited to sucrose, glucose, fructose, lactose, galactose, mannose, maltose, trehalose, cellobiose, raffinose, stachyose, arabinose, xylose, xylulose, ribose, ribulose, desoxyribose, erythrose, erythrulose, threose, talose, rhamnose, fucose, fucosidolactose, lactulose, sophorose, isomaltose, maltotriose, maltooligosaccharides, maltodextrins, fructo-oligosaccharides, inulins, fructans, galactans, glucans, glycogen, pullulan, dextrans, dextrins, cyclodextrins, amylose, amylopectin, starch and pectins.

Examples of water insoluble carbohydrates are cellulose and chitin.

In an embodiment the polyhydroxy compound has in addition to hydroxyl groups also another functional group. Examples of such functional group are carbonyl group, ether group, carboxyl group, amide group, amine group, imine group, thiol group, thioether group, disulfide group, sulfonyl group and sulfinyl group.

In an embodiment the organic polyhydroxy compound consists of the elements carbon, hydrogen and oxygen. In another embodiment the organic polyhydroxy compound consists of the elements carbon, hydrogen, oxygen and another element. Examples of such element are nitrogen, sulfur, phosphorus, boron, fluorine, chlorine, bromine and iodine.

In an embodiment the organic polyhydroxy compound is a synthetic organic polyhydroxy compound which is preferably added in the composition. Thus, preferably the organic polyhydroxy compound is not naturally present in the composition in a significant amount.

In an embodiment the polyhydroxy compound is a mixture of sorbitol and glycerol.

In another embodiment the polyhydroxy compound is a mixture of maltitol and sorbitol; a mixture of maltitol and glycerol; or a mixture of sorbitol and maltitol and glycerol. In another embodiment the polyhydroxy compound is a mixture of mannitol and sorbitol; a mixture of mannitol and glycerol; or a mixture of sorbitol and mannitol and glycerol. In another embodiment the polyhydroxy compound is a mixture of xylitol and sorbitol; a mixture of xylitol and glycerol; or a mixture of sorbitol and xylitol and glycerol.

In an embodiment the polyhydroxy compound is a mixture of two or more polyhydroxy compounds selected from the group consisting of sorbitol, glycerol, maltitol, mannitol and xylitol in any combination.

In an embodiment a mixture of polyhydroxy compounds is used instead of a single polyhydroxy compound. Any appropriate mixture of the polyhydroxy compounds can be used unless the polyhydroxy compounds are incompatible with each other, with the enzyme component, or any other component present in the enzyme composition.

An upper limit for the concentration of polyhydroxy compounds in compositions according to the invention is their limit of solubility. Aqueous sorbitol solutions are commercially available at a concentration of 70 % by weight and maltitol solutions at a concentration of 75 % by weight. A lower limit for the concentration of polyhydroxy compounds in compositions according to the invention is their effectiveness as stabilizing excipient in compositions according to the invention. The effectiveness can be determined using the method described in Example 2 below.

In an embodiment the solution stable enzyme composition contains by weight at least 20%, 24%, 25%, 28%, 30%, 33%, 35%, 40% or 50% of said polyhydroxy compound. Such embodiments with high concentrations of said polyhydroxy compound are advantageous because such compositions remain liquid at temperatures below 0°C. Such compositions can be stored at temperatures below 0°C, for example stored outside in winter, without freezing. The freezing points of compositions with high concentrations of sorbitol, maltitol, lactitol, and hydrogenated corn syrup were reported by Uraji et al. in Food Science Technology International 1996 pp 38-42.

In an embodiment the solution stable enzyme composition contains a mixture of at least two different polyhydroxy compounds. Various ratios of different polyhydroxy compounds are efficient in providing storage stability, as shown in the examples below. The first polyhydroxy compound may be present for example at about 20, 25, 30, 40, or 50% by weight, whereas the second polyhydroxy compounds may be present for example at about 5, 6, 7, 8, 9, 10, 15, 20, 25 or 30 % by weight.

Antimicrobial preservation means are especially important during long storage at ambient temperatures of aqueous compositions comprising organic compounds, especially if such compositions have not been sterilized. Sterilization of liquid products is less common if such products are used for technical applications rather than for medical applications. Thus, in the present invention the solution stable enzyme composition preferably contains antimicrobial preservative to prevent microbial growth.

Antimicrobial preservatives according to the invention are preferably antibacterial chemical compounds and/or antifungal chemical compounds, more preferably chemical compounds against molds, yeasts and/or acid-tolerant bacteria. Such antimicrobial preservatives are for example fungicides, fungistatics, bactericides and/or bacteriostatics. Examples of antimicrobial preservatives according to the invention include, but are not limited to isothiazolinones, particularly 1,2-benzisothiazolin-3-one, 2-methyl-4-isothiazolin-3-one, 5-chloro-2-methyl-4-isothiazolin-3-one, 2-octylisothiazolin-3-one and 4,5-dichlor-2-octylisothiazolin-3-one, phenoxyethanol, benzoic acid, hydroxybenzoic acids, particularly 4-hydroxybenzoic acid and 2-hydroxybenzoic acid, which is also called salicylic acid, sorbic acid, propionic acid, lactic acid, hexanoic acid, octanoic acid, sulfur dioxide, furthermore salts of these acids, particularly sodium, potassium and calcium benzoate, hydroxybenzoate, salicylate, sorbate, propionate, hexanoate, octanoate, sulfite, bisulfite and metabisulfite. Further examples of antimicrobial preservatives are esters of hydroxybenzoic acids, for example methyl 4-hydroxybenzoate, ethyl 4-hydroxybenzoate, propyl 4-hydroxybenzoate, butyl 4-hydroxybenzoate, heptyl 4-hydroxybenzoate, isobutyl 4-hydroxybenzoate, isopropyl 4-hydroxybenzoate, phenyl 4-hydroxybenzoate and benzyl 4-hydroxybenzoate (which are also called methylparaben, ethylparaben, propylparaben, butylparaben, heptylparaben, isobutylparaben, isopropylparaben, phenylparaben and benzylparaben respectively) as well as corresponding salts thereof, for example sodium methyl 4-hydroxybenzoate, sodium ethyl 4-hydroxybenzoate, sodium propyl 4-hydroxybenzoate and so forth.

In a further embodiment instead of, or in addition to, the antimicrobial preservative component, a high concentration of the stabilizer component is used to prevent microbial growth. A sufficiently high concentration is a concentration, which entails an antimicrobial effect due to high osmotic pressure and/or low water activity. Thus, the stabilizer component according to the invention may function simultaneously as antimicrobial preservative according to the invention. Examples of such compounds at high concentrations are sucrose at concentrations of at least approximately 50% by weight, sorbitol at concentrations of at least approximately 45% by weight, glycerin at concentrations of at least approximately 25% by weight and mixtures of glycerin and sorbitol at various high concentrations as determined by Barr and Tice in Journal of the American Pharmaceutical Association 1957 pp 217-223.

Compounds having antimicrobial preservative effect are used according to the invention at concentrations from 0.002 to 75 % by weight, preferably from 0.005 to 10 % by weight, more preferably from 0.01 % to 5 % by weight, even more preferably from 0.02 to 2 % by weight, most preferably from 0.04 to 0.5 % by weight. A lower limit for the concentration of antimicrobial preservatives in compositions according to the invention is their effectiveness to prevent growth of a microorganism in such compositions.

An antimicrobial effect can be determined by counting viable cells (for example as colony forming units) in an aqueous composition (for example a composition according to the invention) after inoculation with a microorganism (for example the acid-tolerant bacterium *Lactobacillus buchneri,* the mold *Aspergillus oryzae* or the yeast *Pichia pastoris*) and incubation for one or more, particularly several weeks at a storage temperature (for example 8°C, 25°C or 37°C) and comparing with a similar aqueous composition without antimicrobial preservative and/or comparing with inoculated aqueous composition before incubation.

Acid-tolerant bacteria, molds and yeasts are food spoilage microorganisms.

To achieve long storage stability of compositions according to the invention, the addition of protease inhibitors, antioxidants or surfactants was not necessary.

The present invention is further described by referring to the following embodiments.

In an embodiment the solution stable enzyme composition is for industrial use. In another embodiment it is for use in manufacturing of paper and/or pulp.

In an embodiment the pH of the solution stable enzyme composition is selected in the range from strongly acidic over neutral to slightly alkaline. In an embodiment the pH of the solution stable enzyme composition is selected in the acidic range. In an embodiment the pH of the solution stable enzyme composition is selected in the citrate buffered range, which is from pH 3.0 to 6.2. In an embodiment the pH of the solution stable enzyme composition is selected in the acetate buffered range, which is from pH 3.5 to 5.8. In an embodiment the pH of the solution stable enzyme composition is selected in the phosphate buffered range, which is from pH 5.8 to 8.0. In an embodiment the pH of the solution stable enzyme composition is selected in an unbuffered neutral range. In an embodiment pH of the solution stable enzyme composition is buffered to a pH range 3.7-8.1 with a pH buffer solution, preferably citrate buffer, acetate buffer or phosphate buffer. In an embodiment the pH of the solution stable enzyme composition is selected from the range between 3 and 8. These ranges are particularly advantageous because within said ranges a good stability can be achieved for a long time with the polyhydroxy compounds of the invention, as is revealed in the examples. Furthermore within said ranges a good stability can be achieved with buffer substances that are compatible during a long storage time with all components of the composition according to the invention.

In an embodiment the enzyme is a hydrolase characterized by having enzyme activity on a sterol ester, preferably an ester of sterol with a fatty acid, more preferably an ester of cholesterol with linoleic acid.

In an embodiment the solution stable enzyme composition comprises fermentation broth, or clarified and optionally concentrated fermentation broth.

In an embodiment said enzyme is a thermostable enzyme. Said embodiment is advantageous because thermostability correlates with other types of stability, particularly long-term stability at ambient temperatures. Furthermore, said embodiment is also advantageous for use in the manufacturing of pulp and paper, where temperatures above 50°C, preferably above 60°C, more preferably above 70°C, are frequently applied.

In an embodiment said enzyme is a sterol esterase derived from a fungus, preferably a thermophilic fungus.

In an embodiment said enzyme is a sterol esterase and the catalytic triad is composed of S, H and E.

In an embodiment said enzyme is a sterol esterase derived from a thermophilic fungus selected from the group consisting of *Scytalidium thermophilum, Myceliophthora thermophila, Thielavia terestris, Corynascus thermophilus, Myriococcum thermophilum, Thermomyces stellatus, Thielavia australiensis, Malbranchea cinnamomea, Melanocarpus albomyces* and *Chaetomium thermophilum* and fragments and conservative alterations of such sterol esterases. In another embodiment the enzyme is the closest sterol esterase homolog, in the above group of thermophilic fungi, of the enzyme having the amino acid sequence according to any one of SEQ ID NO: 1-32.

In an embodiment said enzyme has at least 60 %, preferably at least 70 %, more preferably at least 75 %, most preferably at least 80 % sequence identity with a sterol esterase from *Melanocarpus albomyces* or *Chaetomium thermophilum.*

In another embodiment the enzyme has at least 50, 60, 70, 75, 80, 85, 90, 95 or 99 % sequence identity with the corresponding amino acid with SEQ ID NO: 1 or 2.

In another embodiment the enzyme has at least 50, 60, 70, 75, 80, 85, 90, 95 or 99 % sequence identity with the corresponding sequence of SEQ ID NO: 1, 2, 3, 4, 5, 6, 7, 8, 9 or 10, and preferably being a thermostable sterol esterase, more preferably a fungal thermostable sterol esterase.

In another embodiment the enzyme has at least 50, 60, 70, 75, 80, 85, 90, 95 or 99 % sequence identity with the corresponding sequence of SEQ ID NO: 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31 or 32.

In an embodiment the enzyme does not have 100% sequence identity with any of the sequences SEQ ID NO: 1-32.

In an embodiment said polyhydroxy compound has three or more, preferably four or more, more preferably six or more hydroxyl groups per molecule.

In an embodiment said polyhydroxy compound has four or more, preferably five or more, more preferably six or more hydroxyl groups per molecule. Said polyhydroxy compounds are particularly advantageous in being able to stabilise various enzymes of the invention.

In an embodiment said polyhydroxy compound is a sugar alcohol.

In an embodiment said sugar alcohol is selected from the group consisting of sorbitol, mannitol, maltitol, xylitol and glycerol.

In an embodiment said sugar alcohol is selected from the group consisting of sorbitol, mannitol, maltitol, and xylitol. Said embodiment is advantageous in applications where presence of glycerol is not desirable, such as in applications where glycerol interferes with function of enzymes, or is not compatible in the use. Because glycerol is a product of hydrolysis of glycerol esters, glycerol may cause decrease of enzyme activity due to product inhibition. Thus, instead of glycerol, usage of a polyhydroxy compound that has four or more hydroxyl groups per molecule, preferably from the group consisting of sorbitol, mannitol, maltitol and xylitol, may be desirable when applying a composition according to the invention for hydrolysis of glycerol esters. An example for an industrial application of enzymatic hydrolysis of glycerol esters is in the manufacturing of pulp and paper. Glycerol esters and sterol esters are present in wood resin, which causes sticky deposits during manufacturing of wood pulp and paper, and are preferably hydrolysed quickly before deposition occurs.

In an embodiment the polyhydroxy compound constitute by weight at least one fifth, preferably at least one fourth, more preferably at least one third of said composition. These amounts are preferable because they provide the stabilizing effect for a long time.

In an embodiment the solution stable enzyme composition comprises antimicrobial preservative selected from isothiazolinones, preferably selected from the group consisting of 1,2-benzisothiazolin-3-one, 2-methyl-4-isothiazolin-3-one, 5-chloro-2-methyl-4-isothiazolin-3-one, 2-octylisothiazolin-3-one and 4,5-dichlor-2-octylisothiazolin-3-one. These preservatives are preferable because they are compatible with enzyme compositions and provide antimicrobial effect for a long time.

In an embodiment said preservative is isothiazolinone, preferably selected from the group consisting of 1,2-benzisothiazolin-3-one, 2-methyl-4-isothiazolin-3-one, 5-chloro-2-methyl-4-isothiazolin-3-one, 2-octylisothiazolin-3-one and 4,5-dichlor-2-octylisothiazolin-3-one.

In an embodiment said preservative is selected from the group consisting of benzoic acid, 4-hydroxy-benzoic acid, 2-hydroxy-benzoic acid, and salts thereof and esters thereof. Such an embodiment containing preservatives that are allowed to be added to food are advantageous as a necessary prerequisite for food grade registration of compositions according to the invention. Food and food contact approval may also be required or advantageous for use in the manufacturing of food, feed, pulp and paper.

In an embodiment said preservative is active against at least one microorganism selected from the group consisting of acid-tolerant bacteria, molds and yeasts.

In an embodiment the solution stable enzyme composition is essentially free from polyethylene glycol p-alkyl-phenyl ethers, preferably free from all nonionic surfactants, more preferably free from all surfactants. This embodiment is particularly useful in applications where presence of surfactants is not desired, for example undesirable foam formation due to surfactants.

In an embodiment the solution stable enzyme composition is essentially free from serine protease inhibitors, preferably free from all protease inhibitors. Said embodiment is advantageous when simultaneous activity of a protease and an enzyme composition according to the invention is applied, for example in detergent compositions, for washing laundry and other cleaning applications.

The present invention is further described by the following examples, which do not limit the scope of the invention, which is defined by the appended claims and their equivalents.

### EXAMPLES

### EXAMPLE 1 - MEASUREMENT OF HYDROLYTIC ENZYME ACTIVITY ON THE STEROL ESTER CHOLESTERYL LINOLEATE

This method is a modification of the methods described by St 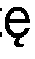 pień et al. in Acta Biochimica Polonica 2013 pp 401-403 and by Sigma-Aldrich at http://www.sigmaaldrich.com/technical-documents/protocols/biology/assay-procedure-for-cholesterol-esterase.html . The following chemicals were purchased from Sigma-Aldrich (now Merck) with the associated order numbers: cholesteryl linoleate C0289, cholesterol oxidase C8649, peroxidase from horseradish 77332, lyophilized bovine serum albumin A2153, 4-aminoantipyrine A4382, sodium 3,5-dichloro-2-hydroxybenzenesulfonate D4645, Triton X-100 X100. The following solutions were freshly prepared:
The AA-solution was 1.76 g 4-aminoantipyrine dissolved in 100ml water. The DHBS-solution was 6 g sodium 3,5-dichloro-2-hydroxybenzenesulfonate dissolved in 100ml water. The CL-solution was 9.8 mg cholesteryl linoleate dissolved in 0.5 ml isopropanol and a 1% Triton X-100 solution at 75°C added, cooled to 25°C and filled up to 25 ml with 1% Triton X-100. The peroxidase powder was dissolved and diluted to 150 PU/ml with 0.1M potassium hydrogenphosphate buffer pH 7.0. The cholesterol oxidase powder was dissolved and diluted to 30.3 U/ml with ice-cold water. The buffer solution, which was used for sample dilutions, was 0.095 g magnesium chloride, 0.0695 g sodium salt of ethylenediaminetetraacetic acid and 1 g lyophilized bovine serum albumin dissolved in 500 ml 0.2M potassium hydrogenphosphate buffer pH 7.5. The substrate solution was a mixture of 4.36 ml 0.2M potassium hydrogenphosphate buffer pH 7.0, 2.91 ml CL-solution, 0.145 ml AA-solution, 0.291 ml DHBS-solution and 0.291 ml peroxidase solution. From this substrate solution 0.917 ml were pipetted into a cuvette and incubated at 37°C. Then 0.033 ml cholesterol oxidase solution was added. Then 0.033 ml of the sample to be measured was quickly mixed into the cuvette. Immediately afterwards the change of absorption per minute, abbreviated ΔOD/min, was measured in the spectrophotometer Lambda25 from Perkin Elmer at 37 °C and 512nm. The enzyme activity, sterol esterase units, abbreviated SEU, was calculated with the formula: SEU/ml = ΔOD/min · 3.819, which takes into account the sample volume of 0.033ml in a total volume of 0.983 ml and a light path through the cuvette of 1 cm. If the sample had been diluted before adding it into the cuvette, the result was also multiplied with the appropriate dilution factor. Samples were diluted in order to measure ΔOD/min values between 0.06 and 0.22.

Significant enzyme activities were measured with the method described above in samples prepared through expression in *Trichoderma reesei* of SEQ ID: 1, 2, 3, 5, 6, 9 and 10. Expression in *Trichoderma reesei* was done as known in the art, for example through Kontkanen et al. in Applied Microbiology and Biotechnology 2006 pp 696-704.

### EXAMPLE 2 - EXPERIMENTAL EVALUATION OF LIQUID ENZYME COMPOSITION STABILITIES

In all experiments enzyme materials used for preparing test compositions were either clarified fermentation broths or concentrates of them, containing preservative to prevent microbial growth, from several different fermentations of enzyme proteins. Studied enzyme proteins were sterol esterase from *Melanocarpus albomyces,* sterol esterase from *Chaetomium thermophilum* and enzyme with the synthetic sequence of SEQ ID NO: 3, which were expressed in *Trichoderma reesei.* In the test compositions enzyme liquids were standardized to activity level range from 1200 to 1900 SEU/g and stabilized using different stabilizing conditions as shown in the following tables. All test compositions were taken under accelerated storage stability study. Total storage time was 24 weeks. For stability study each liquid was divided in 15 ml screw cap tubes, 5 ml of liquid per tube. One tube was prepared for each storage time point. Starting point sample tubes were placed to freezer right after preparing and visual observation. All other sample tubes were placed to 37 °C climate chamber. From each time point one sample tube was taken under visual observation and placed to freezer before activity analysis. Liquid appearance from each storage time point were compared to the appearance at start. Also activities from different storage time points were compared to the starting point activity. Physical stability results are represented in tables 3 - 11.

**Table 3. Stability of enzyme compositions comprising the sterol esterase from Melanocarpus albomyces, 0.04 % (w/w) 1,2-benzisothiazolin-3-one as preservative, sodium citrate as pH buffer, and the polyhydroxy compound in the first column. The plus sign marks stable clear liquid compositions fulfilling commercial liquid product quality criteria, whereas the minus sign marks unstable compositions that developed turbidity and/or precipitation during storage at 37°C.**

| Stabilizer component | pH | after 4 weeks | after 24 weeks |
|---|---|---|---|
| None | 4.92 | - | - |
| None | 5.95 | - | - |
| 15 % (w/w) propylene glycol | 5.03 | - | - |
| 30 % (w/w) propylene glycol | 4.39 | - | - |
| 30 % (w/w) propylene glycol | 5.16 | - | - |
| 40 % (w/w) propylene glycol | 5.32 | - | - |
| 15 % (w/w) propylene glycol and 15 % (w/w) sorbitol | 5.02 | - | - |
| 15 % (w/w) propylene glycol and 35 % (w/w) sorbitol | 5.01 | + | - |
| 15 % (w/w) sorbitol | 4.89 | - | - |
| 30 % (w/w) sorbitol | 4.85 | + | - |
| 40 % (w/w) sorbitol | 4.79 | + | + |
| 50 % (w/w) sorbitol | 4.81 | + | + |
| 15 % (w/w) glycerol | 5.05 | - | - |
| 40 % (w/w) glycerol | 5.00 | + | + |
| 20 % (w/w) maltitol | 5.01 | + | - |
| 40 % (w/w) maltitol | 5.04 | + | + |
| 20 % (w/w) sorbitol and 20 % (w/w) maltitol | 5.00 | + | + |
| 20 % (w/w) sorbitol and 20 % (w/w) glycerol | 4.90 | + | + |
| 25 % (w/w) sorbitol and 25 % (w/w) glycerol | 4.95 | + | + |
| 20 % (w/w) glycerol and 6 % (w/w) maltitol | 5.10 | + | - |

According to the data of table 3, compositions comprising the sterol esterase from *Melanocarpus albomyces* without stabilizer or with propylene glycol as polyhydroxy compound at citrate buffered acidic pH are not physically stable. The data also reveal that 15 % sorbitol concentration is not high enough to prevent physical instability in the presence of 15 % propylene glycol. However 35 % sorbitol in the presence of 15 % propylene glycol is already enough to maintain physically stable liquid for 4 weeks. A composition with 30 % sorbitol is maintaining physically stable liquid for at least 4 weeks. Compositions with 40 - 50 % sorbitol or glycerol or mixtures thereof (20 % + 20 % or 25 % + 25 %) are long-term stable. According to 4 weeks data maltitol is an equally good stabilizer as sorbitol and glycerol. Maltitol also functions in mixtures with sorbitol or glycerol at high concentrations.

**Table 4. Stability of enzyme compositions comprising the sterol esterase from Chaetomium thermophilum, 0.35 % (w/w) sodium benzoate as preservative, sodium citrate as pH buffer, and the polyhydroxy compound in the first column. The plus sign marks stable clear liquid compositions, whereas the minus sign marks unstable compositions that developed turbidity and/or precipitation during storage at 37°C.**

| Stabilizer component | pH | after 4 weeks | after 24 weeks |
|---|---|---|---|
| 40 % (w/w) propylene glycol | 5.25 | - | - |
| 10 % (w/w) mannitol | 4.81 | - | - |
| 50 % (w/w) sorbitol | 4.78 | + | + |
| 25 % (w/w) sorbitol and 6 % (w/w) mannitol | 4.65 | + | + |
| 25 % (w/w) glycerol and 6 % (w/w) mannitol | 4.77 | + | + |
| 20 % (w/w) sorbitol and 6 % (w/w) mannitol | 4.80 | + | + |

According to the data of table 4, composition comprising the sterol esterase from *Chaetomium thermophilum* with 40 % propylene glycol at citrate buffered acidic pH are not physically stable. Physically stable compositions have been achieved using similar high concentrations of sorbitol or mixtures of mannitol with sorbitol or glycerol like used in physically stable compositions of the sterol esterase from *Melanocarpus albomyces* in table 3. This also reveals that mannitol can be an equally good stabilizer as sorbitol, glycerol and maltitol, but due to solubility limitations, mannitol functions in mixures with other polyhydroxy compounds to maintain good physical stability.

**Table 5. Stability of enzyme compositions comprising the enzyme with the synthetic sequence of SEQ ID NO: 3, 0.35 % (w/w) sodium benzoate as preservative, sodium citrate as pH buffer, and the polyhydroxy compound in the first column. The plus sign marks stable clear liquid compositions, whereas the minus sign marks unstable compositions that developed turbidity and/or precipitation during storage at 37°C.**

| Stabilizer component | pH | after 4 weeks | after 24 weeks |
|---|---|---|---|
| 40 % (w/w) propylene glycol | 5.42 | - | - |
| 10 % (w/w) mannitol | 4.92 | - | - |
| 50 % (w/w) sorbitol | 4.89 | + | + |
| 25 % (w/w) sorbitol and 25 % (w/w) glycerol | 5.11 | + | + |

The results shown in table 5 reveal comparable physical stability of compositions comprising the enzyme with the synthetic sequence of SEQ ID NO: 3 as the other two sterol esterases in the same compositions as presented in the tables 3 and 4, respectively.

**Table 6. Stability of enzyme compositions comprising the sterol esterase from Melanocarpus albomyces, 0.35 % (w/w) sodium benzoate as preservative, sodium citrate as pH buffer, and the polyhydroxy compound in the first column. The plus sign marks stable clear liquid compositions, whereas the minus sign marks unstable compositions that developed turbidity and/or precipitation during storage at 37°C.**

| Stabilizer component | pH | after 4 weeks | after 24 weeks |
|---|---|---|---|
| 6 % (w/w) mannitol | 4.82 | - | - |
| 50 % (w/w) sorbitol | 4.78 | + | + |
| 50 % (w/w) sorbitol | 5.02 | + | + |
| 25 % (w/w) glycerol and 6 % (w/w) mannitol | 4.93 | + | + |
| 25 % (w/w) sorbitol and 6 % (w/w) mannitol | 4.82 | + | + |
| 20 % (w/w) sorbitol and 4 % (w/w) mannitol | 4.78 | + | + |

Physical stability data shown in the tables 3 and 6 reveal analog trends in the presence of both studied preservatives, sodium benzoate and 1,2-benzisothiazolin-3-one.

**Table 7. Stability of enzyme compositions comprising the sterol esterase from Melanocarpus albomyces, 0.35 % (w/w) sodium benzoate as preservative, sodium acetate as pH buffer, and the polyhydroxy compound in the first column. The plus sign marks stable clear liquid compositions, whereas the minus sign marks unstable compositions that developed turbidity and/or precipitation during storage at 37°C.**

| Stabilizer component | pH | after 4 weeks | after 24 weeks |
|---|---|---|---|
| 20 % (w/w) propylene glycol | 4.93 | - | - |
| 40 % (w/w) propylene glycol | 4.69 | - | - |
| 40 % (w/w) propylene glycol | 5.22 | - | - |
| 20 % (w/w) sorbitol | 4.70 | + | - |
| 40 % (w/w) sorbitol | 4.70 | + | + |
| 20 % (w/w) sorbitol and 20 % (w/w) glycerol | 4.77 | + | + |

**Table 8. Stability of enzyme compositions comprising the sterol esterase from Melanocarpus albomyces, 0.04 % (w/w) 1,2-benzisothiazolin-3-one as preservative, sodium acetate as pH buffer, and the polyhydroxy compound in the first column. The plus sign marks stable clear liquid compositions, whereas the minus sign marks unstable compositions that developed turbidity and/or precipitation during storage at 37°C.**

| Stabilizer component | pH | after 4 weeks | after 24 weeks |
|---|---|---|---|
| 20 % (w/w) propylene glycol | 4.90 | - | - |
| 40 % (w/w) propylene glycol | 4.41 | - | - |
| 40 % (w/w) propylene glycol | 5.18 | - | - |
| 20 % (w/w) sorbitol and 4 % (w/w) mannitol | 4.68 | + | - |
| 20 % (w/w) sorbitol and 4 % (w/w) maltitol | 4.69 | + | - |
| 20 % (w/w) sorbitol and 4 % (w/w) xylitol | 4.69 | + | - |
| 40 % (w/w) sorbitol | 3.93 | + | + |
| 40 % (w/w) sorbitol | 4.67 | + | + |
| 50 % (w/w) sorbitol | 3.93 | + | + |

Physical stability data shown in the tables 3, 6, 7 and 8 reveal analog trends in the presence of both studied buffers, sodium acetate and sodium citrate. Also xylitol, mannitol and maltitol are equivalent when used in a mixture with sorbitol.

**Table 9. Stability of enzyme compositions comprising the sterol esterase from Melanocarpus albomyces, 0.04 % (w/w) 1,2-benzisothiazolin-3-one as preservative, no pH buffer, and the polyhydroxy compound in the first column. The plus sign marks stable clear liquid compositions, whereas the minus sign marks unstable compositions that developed turbidity and/or precipitation during storage at 37°C.**

| Stabilizer component | pH | after 4 weeks | after 24 weeks |
|---|---|---|---|
| none | 7.50 | - | - |
| 15 % (w/w) propylene glycol | 7.46 | - | - |
| 15 % (w/w) propylene glycol | 8.10 | - | - |
| 20 % (w/w) propylene glycol | 5.15 | - | - |
| 30 % (w/w) propylene glycol | 7.61 | - | - |
| 30 % (w/w) propylene glycol | 8.06 | - | - |
| 50 % (w/w) sorbitol | 5.00 | + | + |
| 20 % (w/w) sorbitol and 4 % (w/w) mannitol | 7.55 | + | - |
| 20 % (w/w) sorbitol and 4 % (w/w) maltitol | 7.65 | + | + |
| 20 % (w/w) sorbitol and 4 % (w/w) xylitol | 7.69 | + | + |
| 25 % (w/w) sorbitol and 25 % (w/w) glycerol | 5.01 | + | + |
| 20 % (w/w) glycerol and 4 % (w/w) mannitol | 7.82 | + | + |
| 20 % (w/w) glycerol and 4 % (w/w) maltitol | 7.68 | + | + |
| 20 % (w/w) glycerol and 4 % (w/w) xylitol | 7.86 | + | + |

Physical stability data shown in table 9 reveal trends presented in the previous tables also in the case of non-buffered sterol esterase compositions at pH close to 5 but also at higher pH range 7.5 - 8.1.

**Table 10. Stability of enzyme compositions comprising the sterol esterase from Melanocarpus albomyces, 0.04 % (w/w) 1,2-benzisothiazolin-3-one as preservative, sodium potassium phosphate as pH buffer, and the polyhydroxy compound in the first column. The plus sign marks stable clear liquid compositions, whereas the minus sign marks unstable compositions that developed turbidity and/or precipitation during storage at 37°C.**

| Stabilizer component | pH | after 4 weeks | after 24 weeks |
|---|---|---|---|
| None | 7.58 | - | - |
| 15 % (w/w) propylene glycol | 7.75 | - | - |
| 30 % (w/w) propylene glycol | 7.90 | - | - |
| 15 % (w/w) propylene glycol and 15 % (w/w) sorbitol | 7.63 | - | - |
| 25 % (w/w) sorbitol and 25 % (w/w) glycerol | 7.39 | + | + |

**Table 11. Stability of enzyme compositions comprising the sterol esterase from Chaetomium thermophilum, 0.35 % (w/w) sodium benzoate as preservative, sodium potassium phosphate as pH buffer, and the polyhydroxy compound in the first column. The plus sign marks stable clear liquid compositions, whereas the minus sign marks unstable compositions that developed turbidity and/or precipitation during storage at 37°C.**

| Stabilizer component | pH | after 4 weeks | after 24 weeks |
|---|---|---|---|
| 30 % (w/w) propylene glycol | 8.11 | - | - |
| 10 % (w/w) mannitol | 7.62 | - | - |
| 25 % (w/w) sorbitol and 25 % (w/w) glycerol | 7.48 | + | + |

Physical stability data shown in the tables 10 and 11 reveal trends presented in the previous tables in sodium potassium phosphate buffered compositions at pH 7.4-8.1.

### EXAMPLE 3 - EXPERIMENTAL EVALUATION OF INCREASING pH ON STABILITY OF ENZYME COMPOSITIONS

In all experiments enzyme materials used for preparing test compositions were concentrates of clarified fermentation broths, containing preservative to prevent microbial growth, from several different fermentations of the same three enzyme proteins as in the previous experiments in example 2. In the test compositions enzyme liquids were not standardized to certain activity level but only stabilized using different stabilizing conditions shown in the following tables. All test compositions were stored for 4 weeks in climate chamber at 20°C. Otherwise storage stability study was done in the same manner as described in the example 2. Physical stability results are represented in table 12.

**Table 12. Stability of compositions comprising the indicated enzyme and the experimental conditions presented in the table. Unstable compositions developed turbidity and/or precipitation immediately at room temperature and/or during storage at 20°C. The plus sign marks stable clear liquid compositions, whereas the minus sign marks unstable compositions.**

| Enzyme * | Stabilizing conditions of enzyme compositions not standardized by activity | pH | immediate observations | after 4 weeks |
|---|---|---|---|---|
| MA | 0.04 % (w/w) 1,2-benzisothiazolin-3-one | 5.56 | - | - |
| MA | 0.04 % (w/w) 1,2-benzisothiazolin-3-one | 7.48 | + | - |
| MA | 30 % (w/w) propylene glycol, 0.04 % (w/w) 1,2-benzisothiazolin-3-one | 5.91 | - | - |
| MA | 30 % (w/w) propylene glycol, 0.04 % (w/w) 1,2-benzisothiazolin-3-one | 7.50 | - | - |
| MA | 0.35 % (w/w) sodium benzoate | 5.98 | - | - |
| MA | 0.35 % (w/w) sodium benzoate | 7.60 | - | - |
| MA | 30 % (w/w) propylene glycol, 0.35 % (w/w) sodium benzoate | 6.22 | - | - |
| MA | 30 % (w/w) propylene glycol, 0.35 % (w/w) sodium benzoate | 7.60 | - | - |
| CT | 0.35 % (w/w) sodium benzoate | 5.92 | - | - |
| CT | 0.35 % (w/w) sodium benzoate | 7.47 | - | - |
| CT | 30 % (w/w) propylene glycol, 0.35 % (w/w) sodium benzoate | 6.22 | - | - |
| CT | 30 % (w/w) propylene glycol, 0.35 % (w/w) sodium benzoate | 7.44 | - | - |
| SEQ3 | 0.35 % (w/w) sodium benzoate | 5.80 | - | - |
| SEQ3 | 0.35 % (w/w) sodium benzoate | 7.49 | - | - |
| SEQ3 | 30 % (w/w) propylene glycol, 0.35 % (w/w) sodium benzoate | 6.10 | - | - |
| SEQ3 | 30 % (w/w) propylene glycol, 0.35 % (w/w) sodium benzoate | 7.61 | - | - |

| | | | | |
|---|---|---|---|---|
| * MA for *Melanocarpus albomyces* sterol esterase, CT for *Chaetomium thermophilum* sterol esterase, and SEQ3 for the enzyme with the synthetic sequence of SEQ ID NO: 3 | | | | |

As the examples in Table 12 reveal, increasing pH was not sufficient for physical stability. In one experiment with the composition comprising the sterol esterase from *Melanocarpus albomyces* and 1,2-benzisothiazolin-3-one as preservative appearance of the liquid was improved by adjusting pH from 5.6 to 7.5 but the composition is clear only at the time of preparing and is not physically stable. Also high pH like 7.5 has overall stability decreasing effect as seen in the following table 13 showing activity drop of standardized enzyme compositions at pH 5 and 7.5.

### EXAMPLE 4 - EXPERIMENTAL EVALUATION OF LIQUID ENZYME COMPOSITION STABILITIES MEASURED AS REMAINING ACTIVITY

Materials and methods were as described in example 1 and 2.

**Table 13. Stability of compositions comprising enzymes, polyhydroxy compounds, antimicrobial preservatives and pH buffers as indicated in the table. Stability was evaluated in this example as remaining enzyme activity after storage. Enzyme activities were measured as described in example 1 and normalized to the respective enzyme activity in the beginning. Thus, enzyme activities start at 100 %, and then during storage at 37°C decrease depending on the enzyme compositions, which comprise a sterol esterase at standardized activity level range from 1200 to 1900 SEU/g, as antimicrobial preservative 0.35 % (w/w) sodium benzoate or 0.04 % (w/w) 1,2-benzisothiazolin-3-one, which were abbreviated in the table as benzoate and isothiazolinone, respectively, and different polyhydroxy compounds as indicated in the table.**

| **Composition** | | | | | **Enzyme activity** | | |
|---|---|---|---|---|---|---|---|
| **Enzyme*** | **Stabilizer component** | **Preservative** | **Buffer** | **pH** | **at start** | **after 4 weeks** | **after 8 weeks** |
| MA | 30 % propylene glycol | isothiazolinone | no buffer | 7.61 | 100 % | 33 % | 17 % |
| MA | None | isothiazolinone | no buffer | 7.50 | 100 % | 10 % | 4 % |
| MA | None | isothiazolinone | citrate | 4.92 | 100 % | 28 % | 17 % |
| MA | 50 % sorbitol | benzoate | citrate | 5.02 | 100 % | 63 % | 52 % |
| SEQ3 | 40 % propylene glycol | benzoate | citrate | 5.42 | 100 % | 3 % | 0 % |
| SEQ3 | 50 % sorbitol | benzoate | citrate | 4.89 | 100 % | 89 % | 80 % |
| SEQ3 | 25 % sorbitol and 25 % glycerol | benzoate | citrate | 5.11 | 100 % | 96 % | 54 % |
| CT | 30 % propylene glycol | benzoate | phosphate | 8.11 | 100 % | 0 % | 0 % |
| CT | 40 % propylene glycol | benzoate | citrate | 5.25 | 100 % | 1 % | 0 % |
| CT | 25 % sorbitol and 6 % mannitol | benzoate | citrate | 4.65 | 100 % | 80 % | 74 % |
| CT | 25 % sorbitol and 25 % glycerol | benzoate | phosphate | 7.48 | 100 % | 51 % | 43 % |

| | | | | | | | |
|---|---|---|---|---|---|---|---|
| *MA for *Melanocarpus albomyces* sterol esterase, CT for *Chaetomium thermophilum* sterol esterase, and SEQ3 for the enzyme with the synthetic sequence of SEQ ID NO: 3. | | | | | | | |

A remaining enzyme activity of more than 50% after 4 weeks storage at 37°C demonstrates a stable enzyme composition. Also more than 40% remaining enzyme activity after 8 weeks storage at 37°C demonstrate a stable enzyme composition.

The disclosure above has provided by way of non-limiting examples of particular implementations and embodiments of the invention a full and informative description of the best mode presently contemplated by the inventors for carrying out the invention. It is however clear to a person skilled in the art that the invention is not restricted to details of the embodiments presented above, but that it can be implemented in other embodiments using equivalent means without deviating from the characteristics of the invention.

Furthermore, some of the features of the above-disclosed embodiments of this invention may be used to advantage without the corresponding use of other features. As such, the foregoing description should be considered as merely illustrative of the principles of the present invention, and not in limitation thereof. Hence, the scope of the invention is only restricted by the appended patent claims.

## Claims

1. A solution stable enzyme composition comprising:
(a) an enzyme component comprising an enzyme **characterized by** having the amino acids sequence WGESAG and a catalytic triad composed of S, H and E/D;
(b) a stabilizer component comprising an organic polyhydroxy compound that has three or more hydroxyl groups per molecule;
(c) an optional antimicrobial preservative component preventing growth of a microorganism; and
(d) water having dissolved therein said components (a), (b) and (c).

2. The solution stable enzyme composition according to claim 1, wherein said enzyme is a hydrolase **characterized by** having enzyme activity on a sterol ester, preferably an ester of sterol with a fatty acid, more preferably an ester of cholesterol with linoleic acid.

3. The solution stable enzyme composition according to claims 1-2, wherein said enzyme is a thermostable enzyme.

4. The solution stable enzyme composition according to claims 1-3, wherein said enzyme is a sterol esterase derived from a fungus, preferably a thermophilic fungus.

5. The solution stable enzyme composition according to claims 1-4 wherein the enzyme is a sterol esterase and the catalytic triad is composed of S, H and E.

6. The solution stable enzyme composition according to claims 1-5, wherein said enzyme is a sterol esterase derived from a thermophilic fungus selected from the group consisting of *Scytalidium thermophilum, Myceliophthora thermophila, Thielavia terestris, Corynascus thermophilus, Myriococcum thermophilum, Thermomyces stellatus, Thielavia australiensis, Malbranchea cinnamomea, Melanocarpus albomyces* and *Chaetomium thermophilum* and fragments thereof and conservative alterations thereof.

7. The solution stable enzyme composition according to claims 1-6, wherein said enzyme has at least 60 %, preferably at least 70 %, more preferably at least 75 %, most preferably at least 80 % sequence identity with a sterol esterase from *Melanocarpus albomyces* or *Chaetomium thermophilum.*

8. The solution stable enzyme composition according to claims 1-7, wherein said polyhydroxy compound has four or more, preferably five or more, more preferably six or more hydroxyl groups per molecule.

9. The solution stable enzyme composition according to claims 1-8, wherein said polyhydroxy compound is a sugar alcohol.

10. The solution stable enzyme composition according to claims 1-9, wherein said sugar alcohol is selected from the group consisting of sorbitol, mannitol, maltitol, xylitol and glycerol.

11. The solution stable enzyme composition according to claims 1-10, wherein said polyhydroxy compound constitute by weight at least one fifth, preferably at least one fourth, more preferably at least one third of said composition.

12. The solution stable enzyme composition according to claims 1-11 comprising antimicrobial preservative selected from isothiazolinones, preferably selected from the group consisting of 1,2-benzisothiazolin-3-one, 2-methyl-4-isothiazolin-3-one, 5-chloro-2-methyl-4-isothiazolin-3-one, 2-octylisothiazolin-3-one and 4,5-dichlor-2-octylisothiazolin-3-one.

13. The solution stable enzyme composition according to claims 1-12 comprising antimicrobial preservative selected from the group consisting of benzoic acid, 4-hydroxy-benzoic acid, 2-hydroxy-benzoic acid, and salts thereof and esters thereof.

14. The solution stable enzyme composition according to claims 1-13, wherein the preservative is active against at least one microorganism selected from the group consisting of acid-tolerant bacteria, molds and yeasts.

15. The solution stable enzyme composition according to claims 1-14, wherein the composition is essentially free from polyethylene glycol p-alkyl-phenyl ethers, preferably free from all nonionic surfactants, more preferably free from all surfactants.

16. The solution stable enzyme composition according claims 1-15, wherein the composition is essentially free from serine protease inhibitors, preferably free from all protease inhibitors.

17. A use of the solution stable enzyme composition according to claims 1-16 in the manufacture of pulp.

18. A use of the solution stable enzyme composition according to claims 1-16 in the manufacture of paper.
